# EUROPEAN PATENT SPECIFICATION

(11) **EP 4 410 992 B1**
(45) Date of publication and mention of the grant of the patent: **17.12.2025**
(21) Application number: 23382083.6
(22) Date of filing: 01.02.2023
(51) Int. Cl.: C12Q 1/6806

(54) **LINEAR DNA WITH ENHANCED RESISTANCE AGAINST EXONUCLEASES AND METHODS FOR THE PRODUCTION THEREOF**
LINEARE DNS MIT ERHÖHTER RESISTENZ GEGEN EXONUKLEASEN UND VERFAHREN ZU IHRER HERSTELLUNG
ADN LINEAIRE A RESISTANCE AMELIOREE CONTRE LES EXONUCLEASES ET PROCEDES DE PRODUCTION DE CET ADN

(43) Date of publication of application: 07.08.2024
(73) Proprietor: 4basebio UK Ltd, Cambridge CB24 5QE (GB)
(72) Inventor: LANCKRIET, Heikki, Over, Cambridge, CB24 5QE (GB); WALKER, Amy, Over, Cambridge, CB24 5QE (GB); PICHER, Ángel, E-28049 Cantoblanco, Madrid (ES)
(74) Representative: Boult Wade Tennant LLP

(56) References cited:
- WO-A1-2016/132129
- WO-A1-2021/123062
- WO-A1-2021/152147

## Description

### TECHNICAL FIELD

The present invention relates to methods for producing a linear deoxyribonucleic acid (DNA) product with enhanced resistance to nuclease digestion are provided. The methods comprise, (a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase target sequence is non-functional; (b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and (c) incubating the precursor double-stranded DNA molecule with a protelomerase to generate the linear DNA product, wherein the protelomerase closes the first end of the precursor double-stranded DNA molecule at the first functional protelomerase target sequences.

### BACKGROUND

DNA is susceptible to degradation by nucleases which are naturally occurring enzymes within organisms and which have a vital role in the regulation of many cellular processes, while also protecting against foreign DNA species. Enzymatic DNA degradation can render gene therapies ineffective and is a substantial consideration when developing gene therapies or DNA vaccines.

Considerable efforts have been made to extend the effective molecular lifetime of nucleic acids by increasing resistance of the nucleic acid molecules to both extracellular and intracellular nucleases.

Resistance to nuclease digestion can be accomplished by using closed DNA molecules, such as plasmids or minicircles. However, plasmids and minicircles have limited utility *in vivo* due to their frequent contamination with toxic agents derived from cell components, fidelity issues that alter the sequence of interest, and presence of different species (supercoiled, linear and open circular).

Alternatively, resistance to nuclease digestion may be accomplished by producing closed linear DNA molecules. For example, WO2010/086626 A1 describes a method for producing a closed linear DNA by utilizing a protelomerase, relying on a complete protelomerase target sequence being present at each end of a DNA sequence of interest. This may generate a desired closed DNA product but also a closed product that is unwanted (e.g. a closed plasmid backbone that is resistant to, e.g., exonuclease digestion), and difficult to separate from the desired product. It also lacks flexibility to produce closed linear DNA products with different closed ends, or linear DNA product that are partially closed.

Thus, a need exists for a more flexible method for producing a linear DNA product with an enhanced resistance to nuclease (e.g. exonuclease) digestion.

### DESCRIPTION

The invention provides a method for producing a linear deoxyribonucleic acid (DNA) product (e.g. a closed linear DNA product) with enhanced resistance to nuclease digestion. The invention is based on the addition of adaptor molecules to a double-stranded DNA molecule and the action of protelomerase to close open ends of DNA at a protelomerase target sequence. The method of the invention relies on the addition of the adaptor molecules, an endonuclease and a protelomerase to the double-stranded DNA molecule comprising a truncated protelomerase target sequence, which may be carried out in a single reaction volume (or single contiguous aqueous volume), or in sequential reactions. Thus, the method for producing a linear DNA product comprises the steps: digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase target sequence is non-functional; appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and incubating the precursor double-stranded DNA molecule with a protelomerase to generate the linear DNA product, wherein the protelomerase closes the first end of the precursor double-stranded DNA molecule at the first functional protelomerase target sequence.

The invention relies on the action of protelomerase, an enzyme that cleaves DNA at a protelomerase target sequence and ligates the cleaved ends together to form covalently closed ends. For example, one protelomerase target sequence, TelN, consists of 56-bp target site consists of a central telO palindrome of 22 base pairs and two 14-base pair flanking sequences (telR and telL) comprising inverted repeats. telO is separated from these repeats by 3 base pairs on each side (SEQ ID NO:1). In general, a protelomerase target sequence is a palindromic sequence, i.e. a double stranded DNA sequence that includes an inverted repeat and is symmetrical around a central point, the cleavage point. Different protelomerase enzymes recognise different sequences, as well known in the art. Examples of protelomerases are TelN protelomerase from *E. coli* phage N15, Klebsiella phage Phi K02 protelomerase, Yersinia phage PY54 protelomerase, Vibrio phage VP882 protelomerase, Borrelia burgdorferi protelomerase along with their respective target sequences.

Preferably, the linear DNA product has enhanced resistance to exonuclease (e.g. exonuclease I, exonuclease III and/or exonuclease VIII) digestion. The linear DNA product may be a closed linear DNA product. The linear DNA product may comprise nuclease-resistant nucleotides (i.e. protected nucleotides), such as phosphorothioated nucleotides. The linear DNA product may be a partially closed linear DNA product. The partially closed linear DNA product may comprise nuclease-resistant nucleotides (i.e. protected nucleotides), such as phosphorothioated nucleotides. The linear DNA product may comprise a cassette. The cassette may comprise a coding sequence.

The invention provides a method of producing a linear DNA product, wherein the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase target sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(c) incubating the precursor double-stranded DNA molecule with a protelomerase to generate the linear DNA product, wherein the protelomerase closes the first end of the precursor double-stranded DNA molecule at the first functional protelomerase target sequence.

The first and second adaptor molecules may be identical molecules or they may be different molecules. The first adaptor molecule comprises a truncated protelomerase target sequence, which is non-functional, that when appended to the double-stranded DNA molecule forms a functional protelomerase sequence with the truncated protelomerase target sequence at the first end of the digested double-stranded DNA molecule, which truncated protelomerase target sequence is also non-functional. The second adaptor molecule may comprise a truncated protelomerase target sequence, which is non-functional, that when appended to the double-stranded DNA molecule forms a functional protelomerase sequence with the truncated protelomerase target sequence at the first end of the digested double-stranded DNA molecule, which truncated protelomerase target sequence is also non-functional. The second adaptor molecule may comprise a hairpin. The second adaptor molecule may be a double stranded nucleic acid molecule comprising one or more nuclease-resistant nucleotides. The first adaptor molecule may comprise a truncated protelomerase target sequence and the second adaptor molecule may be a double-stranded linear nucleic acid molecule comprising one or more nuclease-resistant nucleotides. The first adaptor molecule may comprise a truncated protelomerase target sequence and the second adaptor molecule may comprise a hairpin. Thus, the linear DNA product produced by the methods described herein is resistant to nuclease (e.g. exonuclease) digestion.

The double-stranded DNA molecule comprises a truncated protelomerase sequence; the truncated protelomerase target sequence may consist of SEQ ID NO:s 4, 5, 6 or 20. The truncated protelomerase sequence may consist of SEQ ID NO: 4. The truncated protelomerase sequence may consist of SEQ ID NO: 5. The truncated protelomerase sequence may consist of SEQ ID NO: 6. The truncated protelomerase sequence may consist of SEQ ID NO: 20.

The first adaptor molecule and/or the second adaptor molecule may comprise a sequence selected from SEQ ID NO:8, 9, 10, 11, 12 or 13. The first adaptor and/or the second adaptor molecule may consist of a pair of complementary sequences selected from SEQ ID NO:s 8 and 9, SEQ ID NO:s 10 and 11 or SEQ ID NO:s 12 or 13.

The truncated protelomerase sequence may consist of SEQ ID NO:23, SEQ ID NO: 30, SEQ ID NO: 31 or SEQ ID NO: 32. The truncated protelomerase sequence may consist of SEQ ID NO: 23. The truncated protelomerase sequence may consist of SEQ ID NO: 30. The truncated protelomerase sequence may consist of SEQ ID NO: 31. The truncated protelomerase sequence may consist of SEQ ID NO: 32.

The first adaptor molecule and/or the second adaptor molecule may comprise a sequence selected from SEQ ID NO:24, 25, 26, 27, 28 or 29. The first adaptor and/or the second adaptor molecule may consist of a pair of complementary sequences selected from SEQ ID NO:s 24 and 25, SEQ ID NO:s 26 and 27 or SEQ ID NO:s 28 or 29.

Steps a) to c) may be carried out in a single contiguous aqueous volume, or they may be performed sequentially in separate reactions.

The step of appending a first adaptor molecule and a second adaptor molecule to the first and second ends of the digested DNA molecule is preferably performed in the presence of a ligase. Thus, the invention provides a method of producing a linear DNA product, wherein the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase target sequence is non-functional;
(b) ligating a first adaptor molecule to the first end of the digested double-stranded DNA molecule and ligating a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(c) incubating the precursor double-stranded DNA molecule with a protelomerase to generate the linear DNA product, wherein the protelomerase closes the first end of the precursor double-stranded DNA molecule at the first functional protelomerase target sequences.

Steps a) to c) may be carried out in a single contiguous aqueous volume, or they may be performed sequentially in separate reactions.

The appending (or linking or closing) of the first adaptor molecule and/or the second adaptor molecule may be performed by hybridization or ligation of the adaptor molecules to the ends of the linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The appending of the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region. The appending may be performed via a linker or spacer molecule which facilitates joining of the adaptor molecule to the first and/or second end of the linear double-stranded region.

The method may further comprise, before step (a) (i.e. the step of digesting the double-stranded DNA molecule with an endonuclease that cleaves the endonuclease target sequence to generate a digested double-stranded DNA molecule), a step of amplification of a DNA template molecule to produce the double-stranded DNA molecule. Thus, the invention provides a method for producing a linear DNA product, the method comprises:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(d) incubating the precursor double-stranded DNA molecule with a protelomerase to generate the linear DNA product, wherein the protelomerase closes the first end of the precursor double-stranded DNA molecule at the first functional protelomerase target sequences.

Steps b) to d) may be carried out in a single contiguous aqueous volume, or they may be performed sequentially in separate reactions.

The amplification may be an *in vitro* or *in vivo* amplification. Preferably, the amplification is an *in vitro* amplification. For example, the amplification may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification.

Thus, the invention provides a method for producing a linear deoxyribonucleic acid (DNA) product, the method comprises:
(a) rolling circle amplification of a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(d) incubating the precursor double-stranded DNA molecule with a protelomerase to generate the linear DNA product, wherein the protelomerase closes the first end of the precursor double-stranded DNA molecule at the first functional protelomerase target sequences.

Steps b) to d) may be carried out in a single contiguous aqueous volume, or they may be performed sequentially in separate reactions.

The method may further comprise (after the step of amplification and before the step of digesting the double-stranded DNA molecule with an endonuclease that cleaves the endonuclease target sequence to generate a digested double-stranded DNA molecule) a step of heat-deactivation. Thus, the invention provides a method for producing a linear DNA product, the method comprises:
(a) amplifying a template DNA molecule comprising a truncated protelomerase target sequence and an endonuclease target sequence to generate a double-stranded DNA molecule, wherein the truncated protelomerase sequence is non-functional;
(b) heat deactivation of the reaction of step a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(e) incubating the precursor double-stranded DNA molecule with a protelomerase to generate the linear DNA product, wherein the protelomerase closes the first end of the precursor double-stranded DNA molecule at the first functional protelomerase target sequences.

Steps c) to e) may be carried out in a single contiguous aqueous volume, or they may be performed sequentially in separate reactions.

Preferably, amplification is rolling-circle amplification.

The step of heat-deactivation may be performed under conditions sufficient to inactive the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

The inventors of the present application have surprisingly discovered that large concatemeric products of the rolling circle amplification reaction can be used to produce the DNA products described herein. This is surprising as the product of the rolling circle amplification has high viscosity and typically has to undergo purification steps before it can be utilized for downstream applications.

In the method described herein, after the step of amplification, the steps of digesting the double-stranded DNA molecule with an endonuclease that cleaves the endonuclease target sequence to generate a digested double-stranded DNA molecule, appending a first adaptor molecule to the first end of the digested double-stranded DNA molecule and appending a second adaptor molecule to the second end of the digested double-stranded DNA molecule to generate a precursor double-stranded DNA molecule, and incubating the precursor double-stranded DNA molecule with a protelomerase to generate the linear DNA product may be performed without purifying the product of the amplification reaction. That is to say that the steps of digesting the double-stranded DNA molecule with an endonuclease that cleaves the endonuclease target sequence to generate a digested double-stranded DNA molecule, appending a first adaptor molecule to the first end of the digested double-stranded DNA molecule and appending a second adaptor molecule to the second end of the digested double-stranded DNA molecule to generate a precursor double-stranded DNA molecule, and incubating the precursor double-stranded DNA molecule with a protelomerase to generate the linear DNA productmay be performed directly after the step of amplification. The steps of digesting digesting the double-stranded DNA molecule with an endonuclease that cleaves the endonuclease target sequence to generate a digested double-stranded DNA molecule, appending a first adaptor molecule to the first end of the digested double-stranded DNA molecule and appending a second adaptor molecule to the second end of the digested double-stranded DNA molecule to generate a precursor double-stranded DNA molecule, and incubating the precursor double-stranded DNA molecule with a protelomerase to generate the linear DNA product may be performed directly after the step of heat-deactivation.

In the method of the invention, the steps of digesting the double-stranded DNA molecule with an endonuclease that cleaves the endonuclease target sequence to generate a digested double-stranded DNA molecule, appending a first adaptor molecule to the first end of the digested double-stranded DNA molecule and appending a second adaptor molecule to the second end of the digested double-stranded DNA molecule to generate a precursor double-stranded DNA molecule, and incubating the precursor double-stranded DNA molecule with a protelomerase to generate the linear DNA product may be performed in a single contiguous aqueous volume.

The steps of digesting the double-stranded DNA molecule with an endonuclease that cleaves the endonuclease target sequence to generate a digested double-stranded DNA molecule and appending a first adaptor molecule to the first end of the digested double-stranded DNA molecule and appending a second adaptor molecule to the second end of the digested double-stranded DNA molecule to generate a precursor double-stranded DNA molecule may be carried out in a single contiguous aqueous volume, followed by the step of incubating the precursor double-stranded DNA molecule with a protelomerase to generate the linear DNA product.

The step of digesting the double-stranded DNA molecule with an endonuclease that cleaves the endonuclease target sequence to generate a digested double-stranded DNA molecule may be carried out, followed by the steps of appending a first adaptor molecule to the first end of the digested double-stranded DNA molecule and appending a second adaptor molecule to the second end of the digested double-stranded DNA molecule to generate a precursor double-stranded DNA molecule, and incubating the precursor double-stranded DNA molecule with a protelomerase to generate the linear DNA product in a single contiguous aqueous volume.

In the method of the invention, each step of digesting the double-stranded DNA molecule with an endonuclease that cleaves the endonuclease target sequence to generate a digested double-stranded DNA molecule, appending a first adaptor molecule to the first end of the digested double-stranded DNA molecule and appending a second adaptor molecule to the second end of the digested double-stranded DNA molecule to generate a precursor double-stranded DNA molecule, and incubating the precursor double-stranded DNA molecule with a protelomerase to generate the linear DNA product may be performed sequentially in separate reactions.

The inventors of the present application have discovered a method which requires a very few steps to produce the DNA product described herein. The methods described herein are very time efficient. This is, in part, due to the fact that a step of purification is not required after the amplification reaction. Optionally, the product of the amplification reaction may be heat-deactivated. Surprising, the method described herein where the steps of:
(a) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(c) incubating the precursor double-stranded DNA molecule with a protelomerase to generate the linear DNA product, wherein the protelomerase closes the first end of the precursor double-stranded DNA molecule at the first functional protelomerase target sequences is performed directly after the step of amplification (i.e. without the step of purification) produces higher yields of the DNA product described herein when compared to a method in which the two steps are separated by purification of the amplification product.

The method may further comprise, after the step of incubating the precursor double-stranded DNA molecule with a protelomerase to generate the linear DNA product, a step of purification of the linear DNA product.

The method may further comprise, after the step of incubating the precursor double-stranded DNA molecule with a protelomerase to generate the linear DNA product, a step of nuclease digestion. The nuclease digestion may be exonuclease digestion, such as exonuclease I and/or exonuclease III and/or T5 exonuclease digestion. The step of nuclease digestion may take place before or after the step of purification. The step of nuclease digestion may allow for removal of any double-stranded DNA molecules and/or adaptor molecules which have not been used to produce linear DNA products.

Thus, the method for producing a linear DNA product may comprise the steps:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(c) incubating the precursor double-stranded DNA molecule with a protelomerase to generate the linear DNA product, wherein the protelomerase closes the first end of the precursor double-stranded DNA molecule at the first functional protelomerase target sequences.
(d) incubating the linear DNA product with a nuclease (e.g. an exonuclease).

The method for producing a linear DNA product may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(d) incubating the precursor double-stranded DNA molecule with a protelomerase to generate the linear DNA product, wherein the protelomerase closes the first end of the precursor double-stranded DNA molecule at the first functional protelomerase target sequences; and
(e) incubating the linear DNA product with a nuclease (e.g. an exonuclease).

The step of incubating the linear DNA product with a nuclease (e.g., an exonuclease) may be carried out in the same reaction volume as the step of incubating with a protelomerase, i.e., without purifying the linear DNA product prior to the step of incubating the linear DNA product with a nuclease, e.g., an exonuclease.

The method for producing a linear DNA product may comprise the steps:
a. amplifying a template DNA molecule comprising a truncated protelomerase target sequence and an endonuclease target sequence to generate a double-stranded DNA molecule, wherein the truncated protelomerase sequence is non-functional;
b. digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
c. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
d. incubating the precursor double-stranded DNA molecule with a protelomerase to generate the linear DNA product, wherein the protelomerase closes the first end of the precursor double-stranded DNA molecule at the first functional protelomerase target sequences;
e. purification of the linear DNA product; and
f. incubating the product of step (d) with a nuclease (e.g. an exonuclease).

The method for producing a linear DNA product may comprise the steps:
a. amplifying a template DNA molecule comprising a truncated protelomerase target sequence and an endonuclease target sequence to generate a double-stranded DNA molecule, wherein the truncated protelomerase sequence is non-functional;
b. digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
c. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
d. incubating the precursor double-stranded DNA molecule with a protelomerase to generate the linear DNA product, wherein the protelomerase closes the first end of the precursor double-stranded DNA molecule at the first functional protelomerase target sequences;
e. incubating the single contiguous aqueous volume with a nuclease (e.g. an exonuclease); and f. purification of the linear DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF51, BstMAI, BstV11, BstV2I, Bsul, BtgZI, BtsCI, Btsl-v2, BtsMutl, Bvel, Csel, CspCl, Eam11041, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCI, PciSI, Pctl, Plel, PpsI, Psrl, Schl, SfaNI, Taqll, TspDTI and/or TspGWI restriction enzyme.

Type IIS restriction endonucleases cleave the double-stranded DNA molecule outside of the recognition sequence (i.e. an endonuclease target sequence), which means that the recognition sequence (i.e. endonuclease target sequence) is not included in the linear DNA product.

The present inventors have surprisingly discovered methods for production of a linear DNA product of enhanced resistance to nuclease digestion. Specifically, the linear DNA product produced by the methods described herein has enhanced resistance to exonuclease digestion (e.g. exonuclease III digestion). The enhanced resistance to exonuclease digestion extends the life of the linear DNA product in a cell (i.e. the linear DNA product has enhanced resistance to intracellular exonucleases) and in a cell-free system (i.e. the linear DNA product has enhanced resistance to extracellular exonucleases). The present inventors have developed a method which relies on the addition of adaptor molecules (i.e. a first adaptor molecule and a second adaptor molecule) to both ends of a double-stranded DNA molecule. At least one adaptor molecule comprises a truncated protelomerase target sequence, that when appended to a double-stranded DNA molecule comprising a truncated protelomerase target sequence, forms a functional protelomerase target sequence, such that protelomerase is able to cleave and religate the double stranded DNA to form covalently closed ends of the linear double stranded DNA molecule. One of the adaptor molecules may comprise a hairpin or a loop to form a closed linear DNA product with enhanced resistance to nuclease (e.g. exonuclease) digestion. In addition, the present inventors have discovered a method for efficient introduction of protected nucleotides in the form of a first adaptor molecule or a second adaptor molecule at both ends of a linear double-stranded region of the linear DNA product. The methods described herein may use a hairpin or a loop adaptor on one end of the DNA product and an adaptor comprising a truncated protelomerase target sequence that is subsequently closed by a protelomerase at the other end of the linear DNA product. The methods described herein may use a linear adaptor comprising protected nucleotides on one end of the DNA product and an adaptor comprising a truncated protelomerase target sequence at the other end of the DNA product.. That is to say that any type of adaptors described herein may be used at the other end of the linear DNA product to the adaptor comprising the truncated protelomerase sequence that is subsequently closed by a protelomerase, as long as the final DNA product is protected from nuclease (e.g. exonuclease) digestion. The methods of the invention provide protection from digestion by exonucleases that cleave the 3'-end nucleotides (e.g. exonuclease III) and exonucleases that cleave the 5'-end nucleotides (e.g. exonuclease VIII). Thus, the linear DNA product produced by the methods of the invention has prolonged *in vivo* expression when compared to a linear DNA product that does not comprise an adaptor molecule described herein.

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to nuclease digestion (e.g. exonuclease **III** digestion). For example, the adaptor molecules may comprise nuclease resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

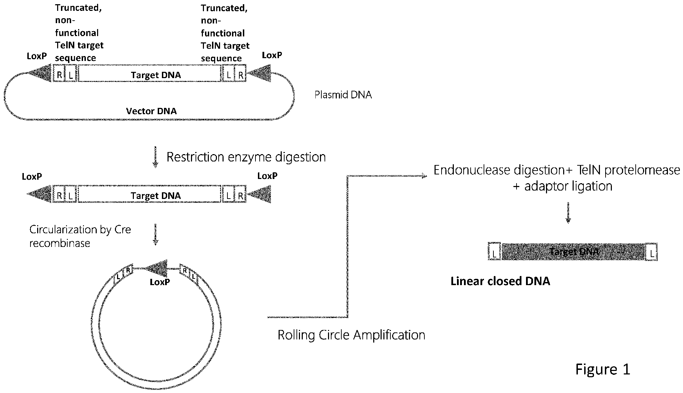

As used herein, "truncated protelomerase target sequence" refers to a protelomerase target sequence that has had sufficient base pairs removed such that protelomerase no longer processes the DNA to produce closed ends, *i.e.,* the protelomerase is unable to cleave and religate the double-stranded DNA to form covalently closed linear DNA. In other words, the truncated protelomerase sequence as used herein refers to a non-functional protelomerase sequence. With reference to the TelN sequence shown in FIG. 1, as an example, the base pairs may be absent from the telR sequence of a protelomerase target sequence or from the telL sequence of a protelomerase target sequence. The base pairs may be absent from the 5' end of the telR sequence, or from the 3' end of the telR sequence. The base pairs may be absent from the 5' end of the telL sequence, or from the 3' end of the telL sequence. Base pairs may also be removed from the 5' or 3' of the telO sequence of a protelomerase target sequence. By truncating the sequence by removing some of the base pairs from the telR or telL sequence, the protelomerase target sequence is rendered non-functional.

When the double stranded DNA molecule comprises two truncated protelomerase sequences, one at a first end and one at a second end (again with reference to the TelN sequence shown in FIG. 1, as an example), the truncated protelomerase sequences at the first end and the second end may have base pairs deleted from the telR sequence; the truncated protelomerase sequences at the first end and the second end may have base pairs deleted from the telL sequence; the truncated protelomerase sequence at the first end may have base pairs deleted form the telR sequence and the truncated protelomerase sequence at the second may have base pairs deleted from the telL sequence; the truncated protelomerase sequence at the first end may have base pairs deleted form the telL sequence and the truncated protelomerase sequence at the second may have base pairs deleted from the telR sequence. The number of base pairs deleted from each truncated sequence may be the same or may be different. For example, if different adaptor molecules are to be appended to the first end and the second end, different number of case pairs may be deleted in order to truncate the protelomerase sequence, provided that the number of base pairs deleted is sufficient to render the truncated protelomerase target sequence non-functional. One example method to test for whether a truncated sequence is non-functional is described in Example 2.

The linear DNA product (e.g. the closed linear DNA product) produced by the methods of the present invention has additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes the linear DNA product produced by the methods of the present invention particularly suitable for use in a pharmaceutical composition.

Unexpectedly, the present inventors have discovered a method for production of a linear DNA product of enhanced resistance to nuclease digestion which allows for efficient production of large quantities of the linear DNA product with enhanced resistance to exonuclease digestion. The large-scale manufacture of the product may be in a cell-free system, which results in the production of a pure sample comprising the linear DNA product substantively free of bacterial contaminants (e.g. remaining after cell lysis).

### 1. Methods for producing closed linear DNA product

The methods described herein may be used to produce a closed linear DNA product e.g. a covalently closed linear DNA product.

The invention provides a method for producing a closed linear DNA product, the method comprises the steps of:
a. digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
b. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
c. incubating the precursor double-stranded DNA molecule with a protelomerase to generate the closed linear DNA product, wherein the protelomerase closes the first and second ends of the precursor double-stranded DNA molecule at the first and second functional protelomerase target sequences.

The step of appending a first adaptor molecule to the first end of the digested double-stranded DNA molecule and appending a second adaptor molecule to the second end of the digested double-stranded DNA molecule to generate a precursor double-stranded DNA molecule is preferably performed in the presence of a ligase. Thus, the invention provides a method for producing a closed linear DNA product, wherein the method comprises:
a. digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
b. ligating a first adaptor molecule to the first end of the digested double-stranded DNA molecule and ligating a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
c. incubating the precursor double-stranded DNA molecule with a protelomerase to generate the closed linear DNA product, wherein the protelomerase closes the first and second ends of the precursor double-stranded DNA molecule at the first and second functional protelomerase target sequences.

The step of appending a first adaptor molecule to the first end of the digested double-stranded DNA molecule and appending a second adaptor molecule to the second end of the digested double-stranded DNA molecule to generate a precursor double-stranded DNA molecule may be performed in the absence of a ligase, and may rely on hybridisation. Thus, the invention provides a method for producing a closed linear DNA product, wherein the method comprises:
a. digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
b. hybridizing a first adaptor molecule to the first end of the digested double-stranded DNA molecule and hybridizing a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
c. incubating the precursor double-stranded DNA molecule with a protelomerase to generate the closed linear DNA product, wherein the protelomerase closes the first and second ends of the precursor double-stranded DNA molecule at the first and second functional protelomerase target sequences.

A closed linear DNA product has particular utility as a therapeutic agent (i.e. DNA therapeutic) which can be used to express a gene product in vivo. This is because its closed structure (e.g. covalently closed structure) prevents attack by enzymes such as exonucleases, leading to enhanced stability and longevity of gene expression as compared to "open" DNA molecules with exposed DNA ends. Linear double-stranded open-ended cassettes have been demonstrated to be inefficient with respect to gene expression when introduced into host tissue. This has been attributed to cassette instability due to the action of exonucleases in the extracellular space.

Sequestering DNA ends inside closed structures also has other advantages. The DNA ends are prevented from integrating with genomic DNA and so closed linear DNA products offer improved safety. In addition, the closed linear structure reduces concatamerisation of DNA products inside host cells and thus expression levels of the gene product can be regulated in a more sensitive manner.

The method of the invention may be used for production of DNA for in vitro expression in a host cell, for example, in DNA vaccines. DNA vaccines typically encode a modified form of an infectious organism's DNA. DNA vaccines are administered to a subject where they then express the selected protein of the infectious organism, initiating an immune response against that protein which is typically protective. DNA vaccines may also encode a tumour antigen in a cancer immunotherapy approach.

The method may produce other types of therapeutic DNA molecules e.g. those used in gene therapy. For example, such DNA molecules can be used to express a functional gene where a subject has a genetic disorder caused by a dysfunctional version of that gene. Examples of such diseases include sickle cell anaemia, cystic fibrosis, Huntington disease, Duchenne's Muscular Dystrophy, Haemophilia A, α1-antitrypsin deficiency, primary ciliary dyskinesia, or respiratory distress syndrome of prematurity. Other diseases where gene therapy may be useful include metabolic diseases, respiratory diseases, inflammatory diseases, autoimmune, chronic and infectious diseases, including such disorders as AIDS, cancer, neurological diseases, cardiovascular disease, hypercholesterolemia, various blood disorders including various anaemias, thalassemia and haemophilia, and emphysema. For the treatment of solid tumours, genes encoding toxic peptides (i.e., chemotherapeutic agents such as ricin, diptheria toxin and cobra venom factor), tumour suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The double-stranded DNA molecule comprises a truncated protelomerase sequence; the truncated protelomerase target sequence may consist of SEQ ID NO:s 4, 5, 6 or 20. The truncated protelomerase sequence may consist of SEQ ID NO: 4. The truncated protelomerase sequence may consist of SEQ ID NO: 5. The truncated protelomerase sequence may consist of SEQ ID NO: 6. The truncated protelomerase sequence may consist of SEQ ID NO: 20.

The first adaptor molecule and/or the second adaptor molecule may comprise a sequence selected from SEQ ID NO:8, 9, 10, 11, 12 or 13. The first adaptor and/or the second adaptor molecule may consist of a pair of complementary sequences selected from SEQ ID NO:s 8 and 9, SEQ ID NO:s 10 and 11 or SEQ ID NO:s 12 or 13.

The truncated protelomerase sequence may consist of SEQ ID NO:23, SEQ ID NO: 30, SEQ ID NO: 31 or SEQ ID NO: 32. The truncated protelomerase sequence may consist of SEQ ID NO: 23. The truncated protelomerase sequence may consist of SEQ ID NO: 30. The truncated protelomerase sequence may consist of SEQ ID NO: 31. The truncated protelomerase sequence may consist of SEQ ID NO: 32.

The first adaptor molecule and/or the second adaptor molecule may comprise a sequence selected from SEQ ID NO:24, 25, 26, 27, 28 or 29. The first adaptor and/or the second adaptor molecule may consist of a pair of complementary sequences selected from SEQ ID NO:s 24 and 25, SEQ ID NO:s 26 and 27 or SEQ ID NO:s 28 or 29.

The closing of the linear double-stranded region of the precursor double-stranded DNA molecule by the first adaptor molecule and/or the second adaptor molecule may be performed by hybridization or ligation of the adaptor molecules to the ends of the linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The closing of the linear double-stranded region by the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region.

When the method of the invention is performed in a single contiguous aqueous volume, it may be performed under conditions that promote appending (or linking) of the first and second adaptor molecules to the linear double-stranded region to produce the closed linear DNA product. The appending may be performed by creating a covalent link between the first and/or second adaptor molecule and the first and/or second end of the linear double-stranded region.

The single contiguous aqueous volume may be incubated under conditions that promote digestion of the double-stranded DNA molecule to produce the linear region of the double-stranded DNA molecule. The digestion of the double-stranded DNA molecule to produce the digested double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

If the step of digesting the double-stranded DNA molecule with an endonuclease that that cleaves an endonuclease target sequence to generate a digested double stranded DNA molecule is carried out as a separate reaction, it may be performed under conditions that promote digestion of the double-stranded DNA molecule to produce the linear region of the double-stranded DNA molecule. The digestion of the double-stranded DNA molecule to produce the digested double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

When the method if performed as a single contiguous aqueous volume, the single contiguous aqueous volume may be incubated under conditions that promote ligation of the digested double-stranded DNA molecule to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested double-stranded DNA molecules may be incorporated into closed linear DNA products. Preferably, the ligation is at least 15% efficient.

If the step of appending or ligating a first adaptor molecule to the first end of the digested double-stranded DNA molecule and appending or ligating a second adaptor molecule to the second end of the digested double stranded DNA molecule to generate a precursor double-stranded DNA molecule is carried out as a separate reaction, it may be performed under conditions that promote ligation of the digested double-stranded DNA molecule to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested double-stranded DNA molecules may be incorporated into closed linear DNA products. Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining open DNA constructs and adaptor molecules excess.

For example, the double-stranded DNA molecule generated by the rolling circle amplification is first quantified so that the amount of the double-stranded DNA molecule used as the starting material during the digestion/ligation/protelomerase reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the digested double-stranded DNA molecule to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The single contiguous aqueous volume may be incubated under conditions that promote the action of protelomerase to cleave and covalently close the protelomerase target sequence to form the closed linear DNA product. The incubation of the precursor double-stranded DNA molecule with protelomerase may be carried out at a third temperature of 1°C-100°C, 1°C -80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

If the step of incubating the precursor double-stranded DNA molecule with a protelomerase to generate the closed linear DNA product is performed as a separate reaction, it may be performed at a third temperature of 1°C-100°C, 1°C -80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

The single contiguous aqueous volume may be performed in a step comprising incubating at a first temperature and then incubating at a second temperature and/or then incubating at a third temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. The third temperature may be 1°C-100°C, 1°C -80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

Preferably, the first temperature is 35°C-39°C and the second temperature is 14°C-18°C and the third temperature is 28°C-32°C. Using these conditions, the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or *E. coli* DNA ligase and the protelomerase may be TelN protelomerase from *E. coli* phage N15 TelN protelomerase along with the sequence defined herein. Alternatively, the protelomerase may be Klebsiella phage Phi K02 protelomerase, Yersinia phage PY54 protelomerase, Vibrio phage VP882 protelomerase, Borrelia burgdorferi protelomerase.

If a protelomerase other than TelN is used, the truncated protelomerase target sequence may be a truncated variant of a protelomerase target sequence specific for the protelomerase enzyme that is used.

The single contiguous aqueous volume may be incubated isothermally. The step of incubating the single contiguous aqueous volume may comprise incubating at a constant temperature. The constant temperature promotes simultaneous digestion of the double-stranded DNA molecule to produce the digested double-stranded DNA molecule and ligation of the digested double-stranded DNA molecule to the first and second adaptor molecules and closing of the precursor double-stranded DNA molecule with protelomerase. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C.

Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature and/or the third temperature. The step of incubating the single contiguous aqueous volume may comprise cycling between the first temperature and the second temperature and/or the third temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature and/or the third temperature less than 40, less than 35, less than 30 times, less than 29, less than 25 times. The step of incubating the single contiguous aqueous volume may comprise cycling between the first temperature and the second temperature and/or the third temperature 2-100, 5-80, 10-70, 20-60, or 30-60 times. The step of incubating the single contiguous aqueous volume may comprise cycling between the first temperature and the second temperature and/or the third temperature 2-20, 5-29, 61-100, or 65-80 times.

The method may further comprise, before step (a) (i.e. the step of digesting a double-stranded DNA molecule with an endonuclease that cleaves an endonuclease target sequence to generate a digested double-stranded DNA molecule), a step of amplifying a DNA template molecule to produce the double-stranded DNA molecule. Thus, the invention provides a method for producing a closed linear DNA product, the method comprises:
a. amplifying a template DNA molecule comprising a truncated protelomerase target sequence and an endonuclease target sequence to generate a double-stranded DNA molecule, wherein the truncated protelomerase sequence is non-functional;
b. digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;
c. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
d. incubating the precursor double-stranded DNA molecule with a protelomerase to generate the closed linear DNA product, wherein the protelomerase closes the first and second ends of the precursor double-stranded DNA molecule at the first and second functional protelomerase target sequences.

Steps b) to d) may be carried out in a single contiguous aqueous volume, or they may be performed sequentially in separate reactions.

The step of amplifying may be performed by in vitro or in vivo amplification. Preferably, the step of amplifying is performed by in vitro amplification. For example, the step of amplifying may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the step of amplifying is performed by rolling circle amplification. Thus, the invention provides a method for producing a closed linear DNA product, the method comprises:
a. amplifying a template DNA molecule comprising a truncated protelomerase target sequence and an endonuclease target sequence to generate a double-stranded DNA molecule by rolling circle amplification, wherein the truncated protelomerase sequence is non-functional;
b. digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;
c. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
d. incubating the precursor double-stranded DNA molecule with a protelomerase to generate the closed linear DNA product, wherein the protelomerase closes the first and second ends of the precursor double-stranded DNA molecule at the first and second functional protelomerase target sequences.

Steps b) to d) may be carried out in a single contiguous aqueous volume, or they may be performed sequentially in separate reactions.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

In the methods described herein, the DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF51, BstMAI, BstV11, BstV21, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva12691, NmeAIII, PaqCI, PciSI, Pctl, Plel, PpsI, Psrl, Schl, SfaNI, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the closed linear DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF51, BstMAI, BstV11, BstV2I, Bsul, BtgZI, BtsCI, Btsl-v2, BtsMutl, Bvel, Csel, CspCI, Eam11041, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCI, PciSI, Pctl, Plel, PpsI, Psrl, Schl, SfaNI, Taqll, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The protelomerase may be TelN protelomerase from *E. coli* phage N15 TelN protelomerase along with the sequence defined herein. Alternatively, the protelomerase may be Klebsiella phage Phi K02 protelomerase, Yersinia phage PY54 protelomerase, Vibrio phage VP882 protelomerase, Borrelia burgdorferi protelomerase.

If a protelomerase other than TelN is used, the truncated protelomerase target sequence may be a truncated variant of a protelomerase target sequence specific for the protelomerase enzyme that is used.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The method may further comprise (after the step of amplification and before the step of digesting the double-stranded DNA molecule with an endonuclease that cleaves the endonuclease target sequence to generate a digested double-stranded DNA molecule) a step of heat-deactivation. Thus, the invention provides a method for producing a closed linear DNA product, the method comprises:
(a) amplifying a template DNA molecule comprising a truncated protelomerase target sequence and an endonuclease target sequence to generate a double-stranded DNA molecule, wherein the truncated protelomerase sequence is non-functional;
(b) heat-deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and .
(e) incubating the precursor double-stranded DNA molecule with a protelomerase to generate the closed linear DNA product, wherein the protelomerase closes the first and second ends of the precursor double-stranded DNA molecule at the first and second functional protelomerase target.

Steps c) to e) may be carried out in a single contiguous aqueous volume, or they may be performed sequentially in separate reactions.

Preferably, amplification is rolling-circle amplification.

The step of heat-deactivation may be performed under conditions sufficient to inactive the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

In the method described herein, after the step of amplification, the step of contacting the double-stranded DNA molecule with an endonuclease, a ligase and first and second adaptor molecules to form a single contiguous aqueous volume may be performed without purifying the product of the amplification reaction. That is to say that the step of digesting a double-stranded DNA molecule with an endonuclease that cleaves an endonuclease target sequence to generate a digested double-stranded DNA molecule, may be performed directly after the step of amplification. The step of digesting a double-stranded DNA molecule with an endonuclease that cleaves an endonuclease target sequence to generate a digested double-stranded DNA moleculemay be performed directly after the step of heat-deactivation.

The method may further comprise, after the step of incubating the precursor double-stranded DNA molecule with a protelomerase to generate the closed linear DNA product, a step of purification of the closed linear DNA product.

The method may further comprise, after the step of incubating the precursor double-stranded DNA molecule with a protelomerase to generate the closed linear DNA product, a step of nuclease digestion. The nuclease digestion may be exonuclease digestion, such as exonuclease I and/or exonuclease **III** digestion. The step of nuclease digestion may take place before or after the step of purification. This step allows for removal of any double-stranded DNA molecules and/or adaptor molecules which have not been used in the course of performing the method. Thus, the method may comprise the steps:
a. amplifying a template DNA molecule comprising a truncated protelomerase target sequence and an endonuclease target sequence to generate a double-stranded DNA molecule, wherein the truncated protelomerase sequence is non-functional;
b. digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;
c. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
d. incubating the precursor double-stranded DNA molecule with a protelomerase to generate the closed linear DNA product, wherein the protelomerase closes the first and second ends of the precursor double-stranded DNA molecule at the first and second functional protelomerase target sequences; and
e. incubating the single contiguous aqueous volume with a nuclease (e.g. exonuclease).

Steps c) to e) may be carried out in a single contiguous aqueous volume, or they may be performed sequentially in separate reactions.

In the method described herein, after the step of amplification, the step of digesting the double-stranded DNA molecule with an endonuclease that cleaves the endonuclease target sequence to generate a digested double-stranded DNA molecule may be performed without purifying the product of the amplification reaction. That is to say that the step of digesting the double-stranded DNA molecule with an endonuclease that cleaves the endonuclease target sequence to generate a digested double-stranded DNA molecule may be performed directly after the step of amplification. The step of digesting the double-stranded DNA molecule with an endonuclease that cleaves the endonuclease target sequence to generate a digested double-stranded DNA molecule may be performed directly after the step of heat-deactivation.

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
(d) incubating the precursor double-stranded DNA molecule with a protelomerase to generate the closed linear DNA product, wherein the protelomerase closes the first and second ends of the precursor double-stranded DNA molecule at the first and second functional protelomerase target sequences;
(e) purifying the closed linear DNA product; and
(f) incubating the purified product of step (e) with a nuclease (e.g. exonuclease).

Steps b) to d) may be carried out in a single contiguous aqueous volume, or they may be performed sequentially in separate reactions.

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) heat deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
(e) incubating the precursor double-stranded DNA molecule with a protelomerase to generate the closed linear DNA product, wherein the protelomerase closes the first and second ends of the precursor double-stranded DNA molecule at the first and second functional protelomerase target sequences;
(f) purifying the closed linear DNA product; and
(g) incubating the purified product of step (f) with a nuclease (e.g. exonuclease).

Steps c) to e) may be carried out in a single contiguous aqueous volume, or they may be performed sequentially in separate reactions.

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
(d) incubating the precursor double-stranded DNA molecule with a protelomerase to generate the closed linear DNA product, wherein the protelomerase closes the first and second ends of the precursor double-stranded DNA molecule at the first and second functional protelomerase target sequences;
(e) incubating the reaction of step (d) with a nuclease (e.g. exonuclease); and
(f) purifying the closed linear DNA product.

Steps b) to d) may be carried out in a single contiguous aqueous volume, or they may be performed sequentially in separate reactions.

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) heat deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
(e) incubating the precursor double-stranded DNA molecule with a protelomerase to generate the closed linear DNA product, wherein the protelomerase closes the first and second ends of the precursor double-stranded DNA molecule at the first and second functional protelomerase target sequences;
(f) incubating the reaction of step (e) with a nuclease (e.g. exonuclease); and
(g) purifying the closed linear DNA product.

Steps c) to f) may be carried out in a single contiguous aqueous volume,or they may be performed sequentially in separate reactions. The single contiguous aqueous volume (or the purified product of step (g)) may be incubated with a nuclease and may be performed at a temperature of 5-90°C, 10-80°C, 15-70°C, 20-60°C, 25-50°C, 30-45°C or 35-40°C. The step of incubating the single contiguous aqueous volume (or the purified product of step (g)) with a nuclease may be performed for at least 10, at least 20, at least 30, at least 40, at least 50, or at least 60 min. The step of incubating the single contiguous aqueous volume (or the purified product of step (g)) may be performed at two different temperatures. For example, the step of incubating the single contiguous aqueous volume (or the purified product of step (g)) may be performed at 15-40°C for 10-60 minutes followed by a temperature of 60-90°C for 10-30 min. The higher temperature typically inactivates the nuclease (e.g. exonuclease). Thus, the method further provides a step of inactivating the nuclease (e.g. exonuclease). The step of incubating the single contiguous aqueous volume (or the purified product of step (g)) may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The methods of the invention may comprise a (further) step of heat-deactivation after the step of incubating with the protelomerase and prior to treatment with an exonuclease. The heat-deactivation may be carried out after the steps of digesting a double-stranded DNA molecule to generate a digested double-stranded molecule; appending a first adaptor molecule to a first end of the digested double stranded DNA molecule and appending a second adaptor molecule to a second end of the digested double-stranded DNA molecule to generate a precursor double-stranded DNA molecule; and incubating the precursor double-stranded DNA molecule with a protelomerase to generate the closed DNA product, whether the steps are performed in a single contiguous aqueous volume or whether the step are carried out sequentially in separate reactions.

Thus, the method of the invention may comprise:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
(c) incubating the precursor double-stranded DNA molecule with a protelomerase to generate the partially closed DNA product, wherein the protelomerase closes the first and second ends of the precursor double-stranded DNA molecule at the first and second functional protelomerase target sequences; and
(d) heat-deactivation of the reaction of steps (a) to (c), or step (c).

Heat-deactivation may be carried out at a temperature of 50-90°C, preferably from 60-80°C, more preferably from 70-80°C, or 72-77°C or 75°C. The heat deactivation may be performed from 1 to 10 minutes, from 2 to 9 minutes, for 3 to 8 minutes, from 4 to 7 minutes, for 6 minutes or, most preferably for 5 minutes.

The method may be a cell-free method.

The closed linear DNA product may be partially double-stranded and/or partially single-stranded. The closed linear DNA product may comprise a portion that is double-stranded and a portion that is single-stranded.

The closed linear DNA product may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The closed linear DNA product may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long.

The closed linear DNA product may comprise an inverted terminal repeat sequence.

The closed linear DNA product may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the closed linear DNA product is at least 50 base pairs long.

The double-stranded DNA molecule may be circular, or branched.

The double-stranded DNA molecule may not comprise an adaptor. The double-stranded DNA molecule may not comprise a hairpin, a loop or a stem-loop structure.

The double-stranded DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule.

The double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may comprise one or more cleavable (e.g. endonuclease) target sequences. The double-stranded DNA molecule may comprise two cleavable (e.g. endonuclease) target sequences. The one or more cleavable (e.g. endonuclease) target sequences may be Type IIS endonuclease target sequences. The one or more cleavable (e.g. endonuclease) target sequences may be Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF5I, BstMAI, BstV1I, BstV21, Bsul, BtgZI, BtsCI, Btsl-v2, BtsMutl, Bvel, Csel, CspCI, Eam11041, Earl, Ecil, Eco31I, Eco571, Esp3I, Faql, Faul, Fokl, Gsul, Hgal, Hphl, HpyAV, Lgul, Lmnl, Lsp11091, Lwel, Mboll, Mlyl, Mmel, Mnll, Mva1269I, NmeAIII, PaqCI, PciSI, Pctl, Plel, PpsI, Psrl, Schl, SfaNI, Taqll, TspDTI and/or TspGWI target sequences.

The double-stranded DNA molecule may be a product of amplification. Preferably, the amplification is rolling circle amplification.

The linear double-stranded region may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The linear double-stranded region may comprise a sequence that is at least 40%, 45%, 50%, 55%, 60%, 65%, 70%, 75%, 80%, 85%, 90%, 95%, or 100% identical to the sequence of the double-stranded DNA molecule.

The first end and the second end of the linear double-stranded region (may be resistant to nuclease digestion. Preferably, the first end and the second end of the linear double-stranded region are resistant to the exonuclease digestion, such as exonuclease III digestion and/or exonuclease I digestion.

The linear double-stranded region may comprise a 3'-OH group at first and/or second ends. The 3'-OH group may facilitate ligation to the first and/or second adaptor molecule(s) (which may comprise a 5' phosphate). The linear double-stranded region may comprise a 5' phosphate at first and/or second ends. The 5' phosphate may facilitate ligation to the first and/or second adaptor molecule(s) (which may comprise a 3'-OH group).

The linear double-stranded region (e.g. of the double-stranded DNA molecule) may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the sense strand or the antisense strand of the linear double-stranded region.

The linear region of the double-stranded DNA molecule (e.g. of the double-stranded DNA molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, double-stranded DNA molecule is at least 50 base pairs long.

The double-stranded DNA molecule comprises a truncated protelomerase sequence, wherein the truncated protelomerase sequence is non-functional. The truncated protelomerase target sequence may be a truncated TelN protelomerase target sequence. The truncated protelomerase target sequence may consist of SEQ ID NO:s 4, 5, 6 or 20. The truncated protelomerase sequence may consist of SEQ ID NO: 4. The truncated protelomerase sequence may consist of SEQ ID NO: 5. The truncated protelomerase sequence may consist of SEQ ID NO: 6. The truncated protelomerase sequence may consist of SEQ ID NO: 20. The truncated protelomerase sequence may consist of SEQ ID NO:23, SEQ ID NO: 30, SEQ ID NO: 31 or SEQ ID NO: 32. The truncated protelomerase sequence may consist of SEQ ID NO: 23. The truncated protelomerase sequence may consist of SEQ ID NO: 30. The truncated protelomerase sequence may consist of SEQ ID NO: 31. The truncated protelomerase sequence may consist of SEQ ID NO: 32.

The first adaptor molecule and/or the second adaptor molecule may be a synthetic adaptor molecule.

The first adaptor molecule may be a nucleic acid adaptor molecule. The second adaptor molecule may be a nucleic acid adaptor molecule. The second adaptor molecule may comprise a self-complementary element which creates a loop, such as a hairpin loop or a stem loop. The second adaptor molecule may comprise a hairpin or a stem-loop. The second adaptor molecule may comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (or of the digested double-stranded DNA molecule) may comprise a 3' or a 5' overhang. A portion of the first adaptor molecule (e.g. the overhang) may be complementary to the first end of the linear double-stranded region. A portion of the second adaptor molecule may be complementary to the second end of the linear double-stranded region.

The first adaptor molecule and/or the second adaptor molecule may comprise a truncated protelomerase target sequence. The first and/or second adaptor molecule may or may not comprise all base pairs that are deleted from the truncated protelomerase sequence present in the double-stranded DNA molecule, e.g., if the truncated protelomerase target sequence in the double stranded DNA molecule is missing eight base pairs, those eight base pairs may be present in the adaptor molecule sequence, or fewer than those eight base pairs may be present in the adaptor molecule. The portion of a protelomerase target sequence present in the first/and or second adaptor molecule are sufficient such that, wherein the first and/or second adaptor molecule is appended to a first and/or second end of the double-stranded DNA molecule comprising a truncated protelomerase target sequence the protelomerase target sequence is rendered functional and the protelomerase is able to cleave and ligate the sequence to covalently close the ends of the adaptor molecules and therefore the ends of the DNA molecule. The protelomerase target sequence may not be fully complete when the adaptor molecule is appended, provided that some activity is restored. The amount of activity restored may be 20%, 30%, 40%, 50%, 60% 70%, 80%, 90% or 100% of the full protelomerase activity with reference to the full protelomerase target sequence. The truncated protelomerase target sequences of the DNA molecule and/or of the adaptor molecule may comprise one or more point mutations, provided that activity is restored by the protelomerase target sequence formed by the adaptor molecule appending to the DNA molecule. The point mutations may be present in the truncated protelomerase target sequence of the double-stranded DNA molecule and/or in the truncated protelomerase target sequence of the adaptor molecule(s).

The first adaptor molecule and/or the second adaptor molecule may comprise a truncated TelN protelomerase target sequence. The first and/or second adaptor molecules may comprise a sequence selected from SEQ ID NO:8, 9, 10, 11, 12 or 13. The first adaptor and/or the second adaptor molecule may consist of a pair of complementary sequences selected from SEQ ID NO:s 8 and 9, SEQ ID NO:s 10 and 11 or SEQ ID NO:s 12 or 13. The first adaptor molecule and/or the second adaptor molecule may comprise a sequence selected from SEQ ID NO:24, 25, 26, 27, 28 or 29. The first adaptor and/or the second adaptor molecule may consist of a pair of complementary sequences selected from SEQ ID NO:s 24 and 25, SEQ ID NO:s 26 and 27 or SEQ ID NO:s 28 or 29.

The closed linear DNA product may be a covalently closed linear DNA product. Thus, in embodiments where each of the adaptor molecules comprise a truncated protelomerase target sequence, protelomerase covalently closes the ends of the adaptor molecules appended to the ends of linear double stranded region, which forms a first and/or second functional protelomerase target sequence, to form a covalently closed linear DNA product. In embodiments where one of the adaptor molecules comprises a loop (e.g. a hairpin), the adaptor molecule closes an end of the linear double-stranded region and protelomerase closes the other end of the linear double-stranded region (via the adaptor molecule comprising the truncated protelomerase sequence together with the truncated sequence of the digested double-stranded DNA molecule), forming a covalently closed linear DNA product.

The invention provides a method for producing a covalently closed linear DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises an overhang that is complementary to and anneals to an overhang at the first end of the digested double-stranded DNA molecule and (ii) the second adaptor molecule comprises an overhang that is complementary to and anneals to an overhang at the second end of the digested double-stranded DNA molecule, and wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
(c) incubating the precursor double-stranded DNA molecule with a protelomerase to generate the closed linear DNA product, wherein the protelomerase closes the first and second ends of the precursor double-stranded DNA molecule at the first and second functional protelomerase target sequences.

The invention provides a method for producing a covalently closed linear DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises an overhang that is complementary to and anneals to an overhang at the first end of the digested double-stranded DNA molecule and (ii) the second adaptor molecule comprises an overhang that is complementary to and anneals to an overhang at the second end of the digested double-stranded DNA molecule, and wherein the first adaptor molecule is ligated to the first end of the digested double-stranded DNA molecule and the second adaptor molecule is ligated to the second end of the digested double-stranded DNA molecule, and wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
(c) incubating the precursor double-stranded DNA molecule with a protelomerase to generate the closed linear DNA product, wherein the protelomerase closes the first and second ends of the precursor double-stranded DNA molecule at the first and second functional protelomerase target sequences.

The first adaptor molecule and/or the second adaptor molecule may not be a plasmid or a vector DNA.

The first adaptor and/or the second adaptor molecule comprise a truncated protelomerase target sequence. The truncated protelomerase target sequence may comprise a partial telR sequence. The truncated protelomerase target sequence may comprise a partial telR sequence and a partial telO sequence. The first and/or second adaptor molecule may comprise 10, 11, 12, 13, 14, 15, 16 17, 18 19, 20, 21, 22, 23, 24, 25, 26, 27, 28 base pairs of a telR sequence. Preferably, the adaptor comprises at least 21 base pairs of a telR sequence. The truncated protelomerase target sequence may comprise a partial telL sequence. The truncated protelomerase target sequence may comprise a partial telL sequence and a partial telO sequence. The first and/or second adaptor molecule may comprise 10, 11, 12, 13, 14, 15, 16 17, 18 19, 20, 21, 22, 23, 24, 25, 26, 27, 28 base pairs of a telL sequence. Preferably, the adaptor comprises at least 21 base pairs of a telL sequence. The first and/or second adaptor molecules may comprise the same sequence or different sequences, i.e., the first adaptor molecule may comprise a different sequence in the overhang portion such that it only appends (by hybridization and/or ligation) to a first end of the digested double-stranded DNA molecule, and not to the second end. Different adaptor molecules may hybridize or ligate to the first end and the second end of the digested double-stranded DNA molecule.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the linear double-stranded region (which may comprise a 3'-OH group at first and/or second ends). The first adaptor molecule and/or the second adaptor molecule may comprise a 3'-OH. The 3'-OH may facilitate ligation to the linear double-stranded region (which may comprise a 5' phosphate at first and/or second ends).

The first and/or second adaptor molecule may comprise the sequence of SEQ ID NO: 14 or a portion thereof. The first and/or second adaptor molecule may comprise at least 13, at least 14, at least 15, at least 16, at least 17, at least 18, or at least 19 contiguous nucleotides of SEQ ID NO: 14. The double-stranded portion of the first and/or second adaptor molecule may comprise the sequence of SEQ ID NO: 15 or a portion thereof. The double-stranded portion of the first and/or second adaptor molecule may comprise at least 8, at least 9, at least 10, at least 11, at least 12, at least 13, at least 14 or at least 15 contiguous nucleotides of SEQ ID NO: 15.

The first and second adaptor molecules may comprise an identical nucleic acid sequence. The first and second adaptor molecules may comprise a different nucleic acid sequence.

The first adaptor molecule may comprise a portion that is complementary to the first end of the linear double-stranded region (of the digested double-stranded DNA molecule). The second adaptor molecule may comprise a portion that is complementary to the second end of the linear double-stranded region (of the digested double-stranded DNA molecule). The first adaptor molecule may comprise a portion that anneals to the first end of the linear double-stranded region (of the digested double-stranded DNA molecule). The second adaptor molecule may comprise a portion that anneals to the second end of the linear double-stranded region (of the digested double-stranded DNA molecule). The first adaptor molecule may comprise a portion that is complementary and anneals to the first end of the linear double-stranded region (of the digested double-stranded DNA molecule). The second adaptor molecule may comprise a portion that is complementary and anneals to the second end of the linear double-stranded region (of the digested double-stranded DNA molecule).

The portion that is complementary or anneals to the first or second end of the linear double-stranded region (of the digested double-stranded DNA molecule) may be a 5' overhang or a 3' overhang of the first and/or second adaptor molecule. The overhang of the first adaptor molecule may be complementary to the first end of the linear double-stranded region and/or the overhang of the second adaptor molecule may be complementary to the second end of the double-stranded region. The overhang of the first adaptor molecule may anneal to the first end of the linear double-stranded region and/or the overhang of the second adaptor molecule may anneal to the second end of the linear double-stranded region. The overhang of the first adaptor molecule may be complementary to and anneal to the first end of the linear double-stranded region and/or the overhang of the second adaptor molecule may be complementary to and anneal to the second end of the linear double-stranded region.

The first adaptor molecule and/or the second adaptor molecule may not comprise a Type IIS endonuclease target sequence. The first adaptor molecule and/or the second adaptor molecule may not comprise Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6I, BstF5I, BstMAI, BstV11, BstV2I, Bsul, BtgZI, BtsCI, Btsl-v2, BtsMutl, Bvel, Csel, CspCI, Eam11041, Earl, Ecil, Eco31I, Eco571, Esp3I, Faql, Faul, Fokl, Gsul, Hgal, Hphl, HpyAV, Lgul, Lmnl, Lsp1109I, Lwel, Mboll, Mlyl, Mmel, Mnll, Mva12691, NmeAIII, PaqCI, PciSI, Pctl, Plel, PpsI, Psrl, Schl, SfaNI, Taqll, TspDTI and/or TspGWI target sequences.

The first adaptor molecule and/or the second adaptor molecule may comprise a Type IIS endonuclease target sequence, provided it is different to a type IIS endonuclease target sequence that is present in the double-stranded DNA molecule.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides. The protected nucleotides may be located in the single-stranded portion (e.g. hairpin portion) or the double-stranded portion. The protected nucleotides may be located in the overhang portion of the adaptor molecules.

The closed linear DNA product may comprise a plurality of phosphorothioated nucleotides at internal positions in each strand. For example, the closed linear DNA product may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions in each strand. Preferably, the closed linear DNA product comprises at least 2 protected nucleotides (e.g. phosphorothioated nucleotides) at internal positions in each strand.

The internal positions may not be located between the second and penultimate nucleotide of the closed linear DNA product.

The linear double-stranded region (of the double-stranded DNA molecule) may comprise a plurality of phosphorothioated nucleotides at internal positions in each strand. For example, the linear double-stranded region (of the double-stranded DNA molecule) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) at internal positions in each strand. Preferably, the linear double-stranded region (of the double-stranded DNA molecule) comprises at least 2 protected nucleotides (e.g. phosphorothioated nucleotides) at internal positions in each strand. The internal positions may not be located between the second and penultimate nucleotide of the linear double-stranded region (of the double-stranded DNA molecule).

The nucleotides resistant to exonuclease digestion (i.e. protected nucleotides) suitable for use in the methods described herein may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nucleotides resistant to exonuclease digestion (i.e. protected nucleotides) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first end of the digested double-stranded DNA molecule may be complementary to a portion of the first adaptor molecule. The second end of the digested double-stranded DNA molecule may be complementary to a portion of the second adaptor molecule. The first end and/or the second end of the digested double-stranded DNA molecule may be generated by endonuclease digestion.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the closed linear DNA product. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the DNA product, the functional portion may comprise a fluorophore, a radioactive compound or a barcode.

A signal corresponding to the presence, absence and/or level of the closed linear DNA product may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the closed linear DNA product. The binding moiety is capable of binding to 3' and/or 5' end of the closed linear DNA product. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the closed linear DNA product and the second binding moiety linked to the second barcoded portion may bind to the 5' end of the closed linear DNA product. The 3' and 5' ends may comprise a 3' overhang, a 5' overhang or a blunt end.

A signal corresponding to the presence, absence and/or level of the closed linear DNA product may be measured using a fluorophore (i.e. a fluorescently-labelled molecule), which is attached or bound to the 3' overhang, the 5' overhang or the blunt end of the closed linear DNA product. The signal may be measured by flow cytometry and/or fluorescence-activated cell sorting.

The functional portion may also facilitate DNA sequencing. For example, the functional portion may be a sequencing adaptor. The term "sequencing adaptor" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeg^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different serotypes. An inverted terminal repeat sequence may comprise a terminal resolution site and a Rep binding site.

The first adaptor molecule and/or the second adaptor molecule may comprise an aptamer.

The first adaptor molecule and/or the second adaptor molecule may confer resistance to the nuclease digestion, such as exonuclease digestion (e.g. exonuclease I and/or exonuclease III digestion).

The closing of the precursor double-stranded DNA at the first end by protelomerase may generate a first closed end of the closed linear DNA product. The closing of the precursor double-stranded DNA molecule at the second end may generate a second closed end of the closed linear DNA product. The first closed end and the second closed end of the closed linear DNA product may be resistant to nuclease digestion. The nuclease digestion may be exonuclease digestion. Preferably, the nuclease digestion is exonuclease III digestion and/or exonuclease I digestion.

### 2. Methods for producing a closed linear DNA product

The methods described herein may be used to produce a closed linear DNA product e.g. a covalently closed linear DNA product.

The invention provides a method for producing a closed linear DNA product, the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(c) incubating the precursor double-stranded DNA molecule with a protelomerase to generate the closed linear DNA product, wherein the protelomerase closes the first end of the precursor double-stranded DNA molecule at the first functional protelomerase target sequences, and the second end of the precursor double-stranded product is closed by the second adaptor molecule.

The step of appending a first adaptor molecule to the first end of the digested double-stranded DNA molecule and appending a second adaptor molecule to the second end of the digested double-stranded DNA molecule to generate a precursor double-stranded DNA molecule is preferably performed in the presence of a ligase. Thus, the invention provides a method for producing a closed linear DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase sequence is non-functional;
(b) ligating a first adaptor molecule to the first end of the digested double-stranded DNA molecule and ligating a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA; and
(c) incubating the precursor double-stranded DNA molecule with a protelomerase to generate the closed linear DNA product, wherein the protelomerase closes the first end of the precursor double-stranded DNA molecule at the first functional protelomerase target sequence and the second end of the precursor double-stranded DNA molecule is closed by the second adaptor.

The step of appending a first adaptor molecule to the first end of the digested double-stranded DNA molecule and appending a second adaptor molecule to the second end of the digested double-stranded DNA molecule to generate a precursor double-stranded DNA molecule may be performed in the absence of a ligase, and may rely on hybridisation. Thus, the invention provides a method for producing a closed linear DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase sequence is non-functional;
(b) hybridizing a first adaptor molecule to the first end of the digested double-stranded DNA molecule and hybridizing a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(c) incubating the precursor double-stranded DNA molecule with a protelomerase to generate the closed linear DNA product, wherein the protelomerase closes the second end of the precursor double-stranded DNA molecule at the first functional protelomerase target sequence and the second end of the precursor double-stranded DNA molecule is closed by the second adaptor.

A closed linear DNA product has particular utility as a therapeutic agent (i.e. DNA therapeutic) which can be used to express a gene product in vivo. This is because its closed structure (e.g. covalently closed structure) prevents attack by enzymes such as exonucleases, leading to enhanced stability and longevity of gene expression as compared to "open" DNA molecules with exposed DNA ends. Linear double-stranded open-ended cassettes have been demonstrated to be inefficient with respect to gene expression when introduced into host tissue. This has been attributed to cassette instability due to the action of exonucleases in the extracellular space.

Sequestering DNA ends inside closed structures also has other advantages. The DNA ends are prevented from integrating with genomic DNA and so closed linear DNA products offer improved safety. In addition, the closed linear structure reduces concatamerisation of DNA products inside host cells and thus expression levels of the gene product can be regulated in a more sensitive manner.

The method of the invention may be used for production of DNA for in vitro expression in a host cell, for example, in DNA vaccines. DNA vaccines typically encode a modified form of an infectious organism's DNA. DNA vaccines are administered to a subject where they then express the selected protein of the infectious organism, initiating an immune response against that protein which is typically protective. DNA vaccines may also encode a tumour antigen in a cancer immunotherapy approach.

The method may produce other types of therapeutic DNA molecules e.g. those used in gene therapy. For example, such DNA molecules can be used to express a functional gene where a subject has a genetic disorder caused by a dysfunctional version of that gene. Examples of such diseases include sickle cell anaemia, cystic fibrosis, Huntington disease, Duchenne's Muscular Dystrophy, Haemophilia A, α1-antitrypsin deficiency, primary ciliary dyskinesia, or respiratory distress syndrome of prematurity. Other diseases where gene therapy may be useful include metabolic diseases, respiratory diseases, inflammatory diseases, autoimmune, chronic and infectious diseases, including such disorders as AIDS, cancer, neurological diseases, cardiovascular disease, hypercholesterolemia, various blood disorders including various anaemias, thalassemia and haemophilia, and emphysema. For the treatment of solid tumours, genes encoding toxic peptides (i.e., chemotherapeutic agents such as ricin, diptheria toxin and cobra venom factor), tumour suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The closing of the linear double-stranded region of the precursor double-stranded DNA molecule by the first adaptor molecule and/or the second adaptor molecule may be performed by hybridization or ligation of the adaptor molecules to the ends of the linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The closing of the linear double-stranded region by the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region.

The steps of digesting a double-stranded DNA molecule with an endonuclease that cleaves an endonuclease target sequence to generate a digested double-stranded DNA molecule; appending a first adaptor molecule to the first end of the digested double-stranded DNA molecule and appending a second adaptor molecule to the second end of the digested double-stranded DNA molecule to generate a precursor double-stranded DNA molecule; and incubating the precursor double-stranded DNA molecule with a protelomerase to generate the closed linear DNA product, may be performed in a single contiguous aqueous volume. When the method of the invention is performed in a single contiguous aqueous volume, it may be performed under conditions that promote appending (or linking) of the first and second adaptor molecules to the linear double-stranded region to produce the closed linear DNA product. The appending may be performed by creating a covalent link between the first and/or second adaptor molecule and the first and/or second end of the linear double-stranded region.

When the method of the invention is performed in a single contiguous aqueous volume, it may be performed under conditions that promote appending (or linking) of the first and second adaptor molecules to the linear double-stranded region to produce the closed linear DNA product. The appending may be performed by creating a covalent link between the first and/or second adaptor molecule and the first and/or second end of the linear double-stranded region.

The single contiguous aqueous volume may be incubated under conditions that promote digestion of the double-stranded DNA molecule to produce the linear region of the double-stranded DNA molecule. The digestion of the double-stranded DNA molecule to produce the digested double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

If the step of digesting the double-stranded DNA molecule with an endonuclease that that cleaves an endonuclease target sequence to generate a digested double stranded DNA molecule is carried out as a separate reaction, it may be performed under conditions that promote digestion of the double-stranded DNA molecule to produce the linear region of the double-stranded DNA molecule. The digestion of the double-stranded DNA molecule to produce the digested double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

When the method if performed as a single contiguous aqueous volume, the single contiguous aqueous volume may be incubated under conditions that promote ligation of the digested double-stranded DNA molecule to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested double-stranded DNA molecules may be incorporated into closed linear DNA products. Preferably, the ligation is at least 15% efficient.

If the step of appending or ligating a first adaptor molecule to the first end of the digested double-stranded DNA molecule and appending or ligating a second adaptor molecule to the second end of the digested double stranded DNA molecule to generate a precursor double-stranded DNA molecule is carried out as a separate reaction, it may be performed under conditions that promote ligation of the digested double-stranded DNA molecule to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested double-stranded DNA molecules may be incorporated into closed linear DNA products. Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining open DNA constructs and adaptor molecules excess.

For example, the double-stranded DNA molecule generated by the rolling circle amplification is first quantified so that the amount of the double-stranded DNA molecule used as the starting material during the digestion/ligation/protelomerase reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the digested double-stranded DNA molecule to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The single contiguous aqueous volume may be incubated under conditions that promote the action of protelomerase to cleave and covalently close the protelomerase target sequence to form the closed linear DNA product. The incubation of the precursor double-stranded DNA molecule with protelomerase may be carried out at a third temperature of 1°C-100°C, 1°C-80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

If the step of incubating the precursor double-stranded DNA molecule with a protelomerase to generate the closed linear DNA product is performed as a separate reaction, it may be performed at a third temperature of 1°C-100°C, 1°C -80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

The single contiguous aqueous volume may be performed in a step comprising incubating at a first temperature and then incubating at a second temperature and/or then incubating at a third temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. The third temperature may be 1°C-100°C, 1°C -80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

Preferably, the first temperature is 35°C-39°C and the second temperature is 14°C-18°C and the third temperature is 28°C-32°C. Using these conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or *E. coli* DNA ligase and the protelomerase may be TelN protelomerase from *E. coli* phage N15 TelN protelomerase along with the sequence defined herein. Alternatively, the protelomerase may be Klebsiella phage Phi K02 protelomerase, Yersinia phage PY54 protelomerase, Vibrio phage VP882 protelomerase, Borrelia burgdorferi protelomerase.

If a protelomerase other than TelN is used, the truncated protelomerase target sequence may be a truncated variant of a protelomerase target sequence specific for the protelomerase enzyme that is used.

The single contiguous aqueous volume may be incubated isothermally. The step of incubating the single contiguous aqueous volume may comprise incubating at a constant temperature. The constant temperature promotes simultaneous digestion of the double-stranded DNA molecule to produce the digested double-stranded DNA molecule and ligation of the digested double-stranded DNA molecule to the first and second adaptor molecules and closing of the precursor double-stranded DNA molecule with protelomerase. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature and/or the third temperature. The step of incubating the single contiguous aqueous volume may comprise cycling between the first temperature and the second temperature and/or the third temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature and/or the third temperature less than 40, less than 35, less than 30 times, less than 29, less than 25 times. The step of incubating the single contiguous aqueous volume may comprise cycling between the first temperature and the second temperature and/or the third temperature 2-100, 5-80, 10-70, 20-60, or 30-60 times. The step of incubating the single contiguous aqueous volume may comprise cycling between the first temperature and the second temperature and/or the third temperature 2-20, 5-29, 61-100, or 65-80 times.

The method may further comprise, before step (a) (i.e. the step of digesting a double-stranded DNA molecule with an endonuclease that cleaves an endonuclease target sequence to generate a digested double-stranded DNA molecule), a step of amplifying a DNA template molecule to produce the double-stranded DNA molecule. Thus, the invention provides a method for producing a closed linear DNA product, the method comprises:
a) amplifying a template DNA molecule comprising a truncated protelomerase target sequence and an endonuclease target sequence to generate a double-stranded DNA molecule, wherein the truncated protelomerase sequence is non-functional;
b) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
d) incubating the precursor double-stranded DNA molecule with a protelomerase to generate the closed linear DNA product, wherein the protelomerase closes the first end of the precursor double-stranded DNA molecule at the first functional protelomerase target sequence and the second end of the precursor double-stranded DNA molecule is closed by the second adaptor.

The step of amplifying may be performed by in vitro or in vivo amplification. Preferably, the step of amplifying is performed by in vitro amplification. For example, the step of amplifying may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the step of amplifying is performed by rolling circle amplification. Thus, the invention provides a method for producing a closed linear DNA product, the method comprises:
(a) amplifying a template DNA molecule comprising a truncated protelomerase target sequence and an endonuclease target sequence to generate a double-stranded DNA molecule by rolling circle amplification, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(d) incubating the precursor double-stranded DNA molecule with a protelomerase to generate the closed linear DNA product, wherein the protelomerase closes the first end of the precursor double-stranded DNA molecule at the first functional protelomerase target sequence and the second end of the precursor double-stranded DNA molecule is closed by the second adaptor.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

In the methods described herein, the DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso311, BspCNI, BspMI, BspPI, BspQI, BspTNI, BsrDI, Bsrl, Bst6I, BstF51, BstMAI, BstV11, BstV21, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva12691, NmeAIII, PaqCI, PciSI, Pctl, Plel, PpsI, Psrl, Schl, SfaNI, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the closed linear DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF51, BstMAI, BstV11, BstV2I, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCI, PciSI, Pctl, Plel, PpsI, Psrl, Schl, SfaNI, Taqll, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The protelomerase may be TelN protelomerase from *E. coli* phage N15 TelN protelomerase along with the sequence defined herein. Alternatively, the protelomerase may be Klebsiella phage Phi K02 protelomerase, Yersinia phage PY54 protelomerase, Vibrio phage VP882 protelomerase, Borrelia burgdorferi protelomerase.

If a protelomerase other than TelN is used, the truncated protelomerase target sequence may be a truncated variant of a protelomerase target sequence specific for the protelomerase enzyme that is used.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The method may further comprise (after the step of amplification and before the step of digesting the double-stranded DNA molecule with an endonuclease that cleaves the endonuclease target sequence to generate a digested double-stranded DNA molecule) a step of heat-deactivation. Thus, the invention provides a method for producing a closed linear DNA product, the method comprises:
(a) amplifying a template DNA molecule comprising a truncated protelomerase target sequence and an endonuclease target sequence to generate a double-stranded DNA molecule, wherein the truncated protelomerase sequence is non-functional;
(b) heat-deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and .
(e) incubating the precursor double-stranded DNA molecule with a protelomerase to generate the closed linear DNA product, wherein the protelomerase closes the first end of the precursor double-stranded DNA molecule at the first functional protelomerase target sequence and the second end of the precursor double-stranded DNA molecule is closed by the second adaptor.

Preferably, amplification is rolling-circle amplification.

The step of heat-deactivation may be performed under conditions sufficient to inactive the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

In the method described herein, after the step of amplification, the step of digesting a double-stranded DNA molecule with an endonuclease that cleaves an endonuclease target sequence to generate a digested double-stranded DNA molecule may be performed without purifying the product of the amplification reaction. That is to say that the step of digesting a double-stranded DNA molecule with an endonuclease that cleaves an endonuclease target sequence to generate a digested double-stranded DNA molecule, may be performed directly after the step of amplification. The step of digesting a double-stranded DNA molecule with an endonuclease that cleaves an endonuclease target sequence to generate a digested double-stranded DNA molecule may be performed directly after the step of heat-deactivation.

The method may further comprise, after the step of incubating the precursor double-stranded DNA molecule with a protelomerase to generate the closed linear DNA product, a step of purification of the closed linear DNA product.

The method may further comprise, after the step of incubating the precursor double-stranded DNA molecule with a protelomerase to generate the closed linear DNA product, a step of nuclease digestion. The nuclease digestion may be exonuclease digestion, such as exonuclease I and/or exonuclease **III** digestion. The step of nuclease digestion may take place before or after the step of purification. This step allows for removal of any double-stranded DNA molecules and/or adaptor molecules which have not been used in the course of performing the method. Thus, the method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(d) incubating the precursor double-stranded DNA molecule with a protelomerase to generate the closed linear DNA product, wherein the protelomerase closes the first end of the precursor double-stranded DNA molecule at the first functional protelomerase target sequence and the second end of the precursor double-stranded DNA molecule is closed by the second adaptor; and
(e) incubating the reaction of step (d) with a nuclease (e.g. exonuclease).

In the method described herein, after the step of amplification, the step of digesting the double-stranded DNA molecule with an endonuclease that cleaves the endonuclease target sequence to generate a digested double-stranded DNA molecule may be performed without purifying the product of the amplification reaction. That is to say that the step of digesting the double-stranded DNA molecule with an endonuclease that cleaves the endonuclease target sequence to generate a digested double-stranded DNA molecule may be performed directly after the step of amplification. The step of digesting the double-stranded DNA molecule with an endonuclease that cleaves the endonuclease target sequence to generate a digested double-stranded DNA molecule may be performed directly after the step of heat-deactivation.

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(d) incubating the precursor double-stranded DNA molecule with a protelomerase to generate the closed linear DNA product, wherein the protelomerase closes the first end of the precursor double-stranded DNA molecule at the first functional protelomerase target sequences and the second end of the precursor double-stranded DNA molecule is closed by the second adaptor;
(e) purifying the closed linear DNA product; and
(f) incubating the purified product of step (e) with a nuclease (e.g. exonuclease).

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) heat deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(e) incubating the precursor double-stranded DNA molecule with a protelomerase to generate the closed linear DNA product, wherein the protelomerase closes the first end of the precursor double-stranded DNA molecule at the first functional protelomerase target sequence and the second end of the precursor double-stranded DNA molecule is closed by the second adaptor;
(f) purifying the closed linear DNA product; and
(g) incubating the purified product of step (f) with a nuclease (e.g. exonuclease).

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(d) incubating the precursor double-stranded DNA molecule with a protelomerase to generate the closed linear DNA product, wherein the protelomerase closes the first ens of the precursor double-stranded DNA molecule at the first functional protelomerase target sequence and the second end of the precursor double-stranded DNA molecule is closed by the second adaptor;
(e) incubating the reaction of step (d) with a nuclease (e.g. exonuclease); and
(f) purifying the closed linear DNA product.

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) heat deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA;
(e) incubating the precursor double-stranded DNA molecule with a protelomerase to generate the closed linear DNA product, wherein the protelomerase closes the first end of the precursor double-stranded DNA molecule at the first functional protelomerase target sequence and the second end of the precursor double-stranded DNA molecule is closed by the second adaptor;
(f) incubating the reaction of step (e) with a nuclease (e.g. exonuclease); and
(g) purifying the closed linear DNA product.

Steps c) to f) may be carried out in a single contiguous aqueous volume. The single contiguous aqueous volume (or the purified product of step (g)) may be incubated with a nuclease and may be performed at a temperature of 5-90°C, 10-80°C, 15-70°C, 20-60°C, 25-50°C, 30-45°C or 35-40°C. The step of incubating the single contiguous aqueous volume (or the purified product of step (g)) with a nuclease may be performed for at least 10, at least 20, at least 30, at least 40, at least 50, or at least 60 min. The step of incubating the single contiguous aqueous volume (or the purified product of step (g)) may be performed at two different temperatures. For example, the step of incubating the single contiguous aqueous volume (or the purified product of step (g)) may be performed at 15-40°C for 10-60 minutes followed by a temperature of 60-90°C for 10-30 min. The higher temperature typically inactivates the nuclease (e.g. exonuclease). Thus, the method further provides a step of inactivating the nuclease (e.g. exonuclease). The step of incubating the single contiguous aqueous volume (or the purified product of step (g)) may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The methods of the invention may comprise a (further) step of heat-deactivation after the step of incubating with the protelomerase and prior to treatment with an exonuclease. The heat-deactivation may be carried out after the steps of digesting a double-stranded DNA molecule to generate a digested double-stranded molecule; appending a first adaptor molecule to a first end of the digested double stranded DNA molecule and appending a second adaptor molecule to a second end of the digested double-stranded DNA molecule to generate a precursor double-stranded DNA molecule; and incubating the precursor double-stranded DNA molecule with a protelomerase to generate the closed DNA product, whether the steps are performed in a single contiguous aqueous volume or whether the step are carried out sequentially in separate reactions.

Thus, the method of the invention may comprise:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(c) incubating the precursor double-stranded DNA molecule with a protelomerase to generate the partially closed DNA product, wherein the protelomerase closes the first end of the precursor double-stranded DNA molecule at the first functional protelomerase target sequence and the second end of the precursor double-stranded DNA molecule is closed by the second adaptor; and
(d) heat-deactivation of the reaction of steps (a) to (c), or step (c).

Heat-deactivation may be carried out at a temperature of 50-90°C, preferably from 60-80°C, more preferably from 70 to 80°C, 72-77 or 75°C. The heat deactivation may be performed from 1 to 10 minutes, from 2 to 9 minutes, for 3 to 8 minutes, from 4 to 7 minutes, for 6 minutes or, most preferably for 5 minutes.

The method may be a cell-free method.

The closed linear DNA product may be partially double-stranded and/or partially single-stranded. The closed linear DNA product may comprise a portion that is double-stranded and a portion that is single-stranded.

The closed linear DNA product may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The closed linear DNA product may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long.

The closed linear DNA product may comprise an inverted terminal repeat sequence.

The closed linear DNA product may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the closed linear DNA product is at least 50 base pairs long.

The double-stranded DNA molecule may be circular, or branched.

The double-stranded DNA molecule may not comprise an adaptor. The double-stranded DNA molecule may not comprise a hairpin, a loop or a stem-loop structure.

The double-stranded DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule.

The double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may comprise one or more cleavable (e.g. endonuclease) target sequences. The double-stranded DNA molecule may comprise two cleavable (e.g. endonuclease) target sequences. The one or more cleavable (e.g. endonuclease) target sequences may be Type IIS endonuclease target sequences. The one or more cleavable (e.g. endonuclease) target sequences may be Bbsl, Bsal, BsmBI, BspQI, BtgZI, Esp3I,SapI, Aarl, Acc361, AcIWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF5I, BstMAI, BstV1I, BstV21, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp11091, Lwel, Mboll, Mlyl, Mmel, Mnll, Mva1269I, NmeAIII, PaqCI, PciSI, Pctl, Plel, PpsI, Psrl, Schl, SfaNI, Taqll, TspDTI and/or TspGWI target sequences.

The double-stranded DNA molecule may be a product of amplification. Preferably, the amplification is rolling circle amplification.

The linear double-stranded region of the double-stranded DNA molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule comprises a truncated protelomerase sequence, wherein the truncated protelomerase sequence is non-functional. The truncated protelomerase target sequence may be a truncated TelN protelomerase target sequence. The truncated protelomerase target sequence may consist of SEQ ID NO:s 4, 5, 6 or 20. The truncated protelomerase sequence may consist of SEQ ID NO: 4. The truncated protelomerase sequence may consist of SEQ ID NO: 5. The truncated protelomerase sequence may consist of SEQ ID NO: 6. The truncated protelomerase sequence may consist of SEQ ID NO: 20. The truncated protelomerase sequence may consist of SEQ ID NO:23, SEQ ID NO: 30, SEQ ID NO: 31 or SEQ ID NO: 32. The truncated protelomerase sequence may consist of SEQ ID NO: 23. The truncated protelomerase sequence may consist of SEQ ID NO: 30. The truncated protelomerase sequence may consist of SEQ ID NO: 31. The truncated protelomerase sequence may consist of SEQ ID NO: 32.

The linear double-stranded region may comprise a sequence that is at least 40%, 45%, 50%, 55%, 60%, 65%, 70%, 75%, 80%, 85%, 90%, 95%, or 100% identical to the sequence of the double-stranded DNA molecule.

The first end and the second end of the linear double-stranded region (e.g. of the double-stranded DNA molecule) may be resistant to nuclease digestion. Preferably, the first end and the second end of the linear double-stranded region are resistant to the exonuclease digestion, such as exonuclease III digestion and/or exonuclease I digestion.

The linear double-stranded region of the digested double-stranded DNA molecule may comprise a 3'-OH group at first and/or second ends. The 3'-OH group may facilitate ligation to the first and/or second adaptor molecule(s) (which may comprise a 5' phosphate). The linear double-stranded region may comprise a 5' phosphate at first and/or second ends. The 5' phosphate may facilitate ligation to the first and/or second adaptor molecule(s) (which may comprise a 3'-OH group).

The linear double-stranded region (e.g. of the digested double-stranded DNA molecule) may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the sense strand or the antisense strand of the linear double-stranded region.

The linear region of the double-stranded DNA molecule (e.g. of the double-stranded DNA molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, double-stranded DNA molecule is at least 50 base pairs long.

The first adaptor molecule and/or the second adaptor molecule may be a synthetic adaptor molecule.

The first adaptor molecule may be a nucleic acid adaptor molecule. The second adaptor molecule may be a nucleic acid adaptor molecule. The second adaptor molecule may comprise a self-complementary element which creates a loop, such as a hairpin loop or a stem loop. The second adaptor molecule may comprise a hairpin or a stem-loop. The second adaptor molecule may comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (of the double-stranded DNA molecule) may comprise a 3' or a 5' overhang.

A portion of the first adaptor molecule (e.g. the overhang) may be complementary to the first end of the linear double-stranded region. A portion of the second adaptor molecule may be complementary to the second end of the linear double-stranded region.

The first adaptor molecule and/or the second adaptor molecule may comprise a truncated protelomerase target sequence. The first and/or second adaptor molecule may or may not comprise all base pairs that are deleted from the truncated protelomerase sequence present in the double-stranded DNA molecule, e.g., if the truncated protelomerase target sequence in the double stranded DNA molecule is missing eight base pairs, those eight base pairs may be present in the adaptor molecule sequence, or fewer than those eight base pairs may be present in the adaptor molecule. The portion of a protelomerase target sequence present in the first/and or second adaptor molecule are sufficient such that, wherein the first and/or second adaptor molecule is appended to a first and/or second end of the double-stranded DNA molecule comprising a truncated protelomerase target sequence the protelomerase target sequence is rendered functional and the protelomerase is able to cleave and ligate the sequence to covalently close the ends of the adaptor molecules and therefore the ends of the DNA molecule. The protelomerase target sequence may not be fully complete when the adaptor molecule is appended, provided that some activity is restored. The amount of activity restored may be 20%, 30%, 40%, 50%, 60% 70%, 80%, 90% or 100% of the full protelomerase activity with reference to the full TelN target sequence. The truncated protelomerase target sequences of the DNA molecule and/or of the adaptor molecule may comprise one or more point mutations, provided that activity is restored by the protelomerase target sequence formed by the adaptor molecule appending to the DNA molecule. The point mutations may be present in the truncated protelomerase target sequence of the double-stranded DNA molecule and/or in the truncated protelomerase target sequence of the adaptor molecule(s).

The first adaptor molecule and/or the second adaptor molecule may comprise a truncated TelN protelomerase target sequence. The first and/or second adaptor molecules may comprise a sequence selected from SEQ ID NO:8, 9, 10, 11, 12 or 13. The first adaptor and/or the second adaptor molecule may consist of a pair of complementary sequences selected from SEQ ID NO:s 8 and 9, SEQ ID NO:s 10 and 11 or SEQ ID NO:s 12 or 13. The first adaptor molecule and/or the second adaptor molecule may comprise a sequence selected from SEQ ID NO:24, 25, 26, 27, 28 or 29. The first adaptor and/or the second adaptor molecule may consist of a pair of complementary sequences selected from SEQ ID NO:s 24 and 25, SEQ ID NO:s 26 and 27 or SEQ ID NO:s 28 or 29.

The closed linear DNA product may be a covalently closed linear DNA product. Thus, in embodiments where an adaptor molecules comprises a truncated protelomerase target sequence, protelomerase covalently closes the end of the adaptor molecule appended to the end of linear double stranded region which forms a first functional protelomerase target sequence, to form a covalently closed linear DNA product. In embodiments where one of the adaptor molecules comprises a loop (e.g. a hairpin), the adaptor molecule closes an end (e.g., the first end) of the linear double-stranded region and protelomerase closes the other end (e.g., the second end) of the linear double-stranded region, to form a covalently closed linear DNA product.

The invention provides a method for producing a covalently closed linear DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule, wherein (i) the first adaptor molecule comprises an overhang that is complementary to and anneals to an overhang at the first end of the linear double-stranded region and (ii) the second adaptor molecule comprises an overhang that is complementary to and anneals to an overhang at the second end of the linear double-stranded region; and
(c) incubating the precursor double-stranded DNA molecule with a protelomerase to generate the closed linear DNA product, wherein the protelomerase closes the first end of the precursor double-stranded DNA molecule at the first functional protelomerase target sequences and the second end of the precursor double stranded DNA molecule is closed by the second adaptor molecule.

The invention provides a method for producing a covalently closed linear DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule, wherein (i) the first adaptor molecule comprises an overhang that is complementary to and anneals to an overhang at the first end of the linear double-stranded region and (ii) the second adaptor molecule comprises an overhang that is complementary to and anneals to an overhang at the second end of the linear double-stranded region, and wherein the first adaptor molecule is ligated to the first end of the linear double-stranded region and the second adaptor molecule is ligated to the second end of the linear double-stranded region; and
(c) incubating the precursor double-stranded DNA molecule with a protelomerase to generate the closed linear DNA product, wherein the protelomerase closes the first end of the precursor double-stranded DNA molecule at the first functional protelomerase target sequences and the second end of the precursor double stranded DNA molecule is closed by the second adaptor molecule.

The first adaptor molecule and/or the second adaptor molecule may not be a plasmid or a vector DNA.

The first adaptor and/or the second adaptor molecule comprise a truncated protelomerase target sequence. The first adaptor and/or the second adaptor molecule may comprise a truncated TelN protelomerase target sequence. The truncated protelomerase target sequence may comprise a partial telR sequence. The truncated protelomerase target sequence may comprise a partial telR sequence and a partial telO sequence. The first and/or second adaptor molecule may comprise 10, 11, 12, 13, 14, 15, 16 17, 18 19, 20, 21, 22, 23, 24, 25, 26, 27, 28 base pairs of a telR sequence. Preferably, the adaptor comprises at least 21 base pairs of a telR sequence. The truncated protelomerase target sequence may comprise a partial telL sequence. The truncated protelomerase target sequence may comprise a partial telL sequence and a partial telO sequence. The first and/or second adaptor molecule may comprise 10, 11, 12, 13, 14, 15, 16 17, 18 19, 20, 21, 22, 23, 24, 25, 26, 27, 28 base pairs of a telL sequence. Preferably, the adaptor comprises at least 21 base pairs of a telL sequence. The first and/or second adaptor molecules may comprise the same sequence or different sequences, i.e., the first adaptor molecule may comprise a different sequence in the overhang portion such that it only appends (by hybridization and/or ligation) to a first end of the digested double-stranded DNA molecule, and not to the second end.

The second adaptor molecule may comprise a single-stranded portion. The single-stranded portion may form a hairpin or a stem-loop. Thus, the second adaptor molecule may comprise a loop portion. The single-stranded portion may comprise less than 10, 9, 8, 7, 6, 5, 4, 3, 2 nucleotides. Preferably, the single-stranded portion comprises 5 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the linear double-stranded region (which may comprise a 3'-OH group at first and/or second ends). The first adaptor molecule and/or the second adaptor molecule may comprise a 3'-OH. The 3'-OH may facilitate ligation to the linear double-stranded region (which may comprise a 5' phosphate at first and/or second ends).

The first and/or second adaptor molecule may comprise the sequence of SEQ ID NO: 14 or a portion thereof. The first and/or second adaptor molecule may comprise at least 13, at least 14, at least 15, at least 16, at least 17, at least 18, or at least 19 contiguous nucleotides of SEQ ID NO: 14. The double-stranded portion of the first and/or second adaptor molecule may comprise the sequence of SEQ ID NO: 15 or a portion thereof. The double-stranded portion of the first and/or second adaptor molecule may comprise at least 8, at least 9, at least 10, at least 11, at least 12, at least 13, at least 14 or at least 15 contiguous nucleotides of SEQ ID NO: 15. The single-stranded portion of the first and/or second adaptor molecule may comprise a sequence of ACTCA. The single-stranded portion of the first adaptor molecule and/or the second adaptor molecule may comprise at least 1, at least 2, at least 3, at least 4 or at least 5 contiguous nucleotides of the sequence ACTCA. The second adaptor molecule may comprise or consist of the sequence of SEQ ID NO:17.

The first and second adaptor molecules may comprise an identical nucleic acid sequence. The first and second adaptor molecules may comprise a different nucleic acid sequence.

The first adaptor molecule may comprise a portion that is complementary to the first end of the digested double-stranded DNA molecule. The second adaptor molecule may comprise a portion that is complementary to the second end of the digested double-stranded DNA molecule. The first adaptor molecule may comprise a portion that anneals to the first end of the digested double-stranded DNA molecule. The second adaptor molecule may comprise a portion that anneals to the second end of the digested double-stranded DNA molecule. The first adaptor molecule may comprise a portion that is complementary and anneals to the first end of the digested double-stranded DNA molecule. The second adaptor molecule may comprise a portion that is complementary and anneals to the second end of the digested double-stranded DNA molecule.

The portion that is complementary or anneals to the first or second end of digested double-stranded DNA molecule may be a 5' overhang or a 3' overhang of the first and/or second adaptor molecule. The overhang of the first adaptor molecule may be complementary to the first end of the digested double-stranded DNA molecule and/or the overhang of the second adaptor molecule may be complementary to the second end of the digested double-stranded DNA molecule. The overhang of the first adaptor molecule may anneal to the first end of the digested double-stranded DNA molecule and/or the overhang of the second adaptor molecule may anneal to the second end of the digested double-stranded DNA molecule. The overhang of the first adaptor molecule may be complementary to and anneal to the first end of the digested double-stranded DNA molecule and/or the overhang of the second adaptor molecule may be complementary to and anneal to the second end of the digested double-stranded DNA molecule.

The first adaptor molecule and/or the second adaptor molecule may not comprise a Type IIS endonuclease target sequence. The first adaptor molecule and/or the second adaptor molecule may not comprise Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6I, BstF5I, BstMAI, BstV11, BstV2I, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva12691, NmeAIII, PaqCI, PciSI, Pctl, Plel, PpsI, Psrl, Schl, SfaNI, Taqll, TspDTI and/or TspGWI target sequences.

The first adaptor molecule and/or the second adaptor molecule may comprise a Type IIS endonuclease target sequence, provided it is different to a type IIS endonuclease target sequence that is present in the double-stranded DNA molecule.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides. The protected nucleotides may be located in the single-stranded portion (e.g. hairpin portion) or the double-stranded portion. The protected nucleotides may be located in the overhang portion of the adaptor molecules.

The closed linear DNA product may comprise a plurality of phosphorothioated nucleotides at internal positions in each strand. For example, the closed linear DNA product may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions in each strand. Preferably, the closed linear DNA product comprises at least 2 protected nucleotides (e.g. phosphorothioated nucleotides) at internal positions in each strand.

The internal positions may not be located between the second and penultimate nucleotide of the closed linear DNA product.

The linear double-stranded region (of the double-stranded DNA molecule) may comprise a plurality of phosphorothioated nucleotides at internal positions in each strand. For example, the linear double-stranded region (of the double-stranded DNA molecule) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) at internal positions in each strand. Preferably, the linear double-stranded region (of the double-stranded DNA molecule) comprises at least 2 protected nucleotides (e.g. phosphorothioated nucleotides) at internal positions in each strand. The internal positions may not be located between the second and penultimate nucleotide of the linear double-stranded region (of the double-stranded DNA molecule).

The nucleotides resistant to exonuclease digestion (i.e. protected nucleotides) suitable for use in the methods described herein may be phosphorothioated nucleotides. For example, phosphorothioated nucleotides may be α-S-dATP (i.e. 2'-deoxyadenosine-5'-(a-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nucleotides resistant to exonuclease digestion (i.e. protected nucleotides) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first end of the linear double-stranded region may be complementary to a portion of the first adaptor molecule. The second end of the linear double-stranded region may be complementary to a portion of the second adaptor molecule. The first end and/or the second end of the linear double-stranded region may be generated by endonuclease digestion.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probetarget base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the closed linear DNA product. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the DNA product, the functional portion may comprise a fluorophore, a radioactive compound or a barcode.

A signal corresponding to the presence, absence and/or level of the closed linear DNA product may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the closed linear DNA product. The binding moiety is capable of binding to 3' and/or 5' end of the closed linear DNA product. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the closed linear DNA product and the second binding moiety linked to the second barcoded portion may bind to the 5' end of the closed linear DNA product. The 3' and 5' ends may comprise a 3' overhang, a 5' overhang or a blunt end.

A signal corresponding to the presence, absence and/or level of the closed linear DNA product may be measured using a fluorophore (i.e. a fluorescently-labelled molecule), which is attached or bound to the 3' overhang, the 5' overhang or the blunt end of the closed linear DNA product. The signal may be measured by flow cytometry and/or fluorescence-activated cell sorting.

The functional portion may also facilitate DNA sequencing. For example, the functional portion may be a sequencing adaptor. The term "sequencing adaptor" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different serotypes. An inverted terminal repeat sequence may comprise a terminal resolution site and a Rep binding site.

The first adaptor molecule and/or the second adaptor molecule may comprise an aptamer.

The first adaptor molecule and/or the second adaptor molecule may confer resistance to the nuclease digestion, such as exonuclease digestion (e.g. exonuclease I and/or exonuclease III digestion).

The closing of the precursor double-stranded DNA molecule at the first end by protelomerase may generate a first closed end of the closed linear DNA product. The closing of the precursor double-stranded DNA molecule at the second end by the appending, or ligation, of the second adaptor may generate a second closed end of the closed linear DNA product. The first closed end and the second closed end of the closed linear DNA product may be resistant to nuclease digestion. The nuclease digestion may be exonuclease digestion. Preferably, the nuclease digestion is exonuclease III digestion and/or exonuclease I digestion.

### 3. Methods for producing a partially closed linear DNA product comprising nuclease-resistant nucleotides

The methods described herein may be used to produce a partially closed linear DNA product comprising nuclease-resistant (i.e. protected nucleotides).

The methods described herein may be used to produce a partially closed DNA product e.g. a covalently partially closed DNA product.

The invention provides a method for producing a partially closed DNA product, the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides; and
(c) incubating the precursor double-stranded DNA molecule with a protelomerase to generate the partially closed DNA product, wherein the protelomerase closes the first end of the precursor double-stranded DNA molecule at the first functional protelomerase target sequences.

The step of appending a first adaptor molecule to the first end of the digested double-stranded DNA molecule and appending a second adaptor molecule to the second end of the digested double-stranded DNA molecule to generate a precursor double-stranded DNA molecule is preferably performed in the presence of a ligase. Thus, the invention provides a method for producing a partially closed DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase sequence is non-functional;
(b) ligating a first adaptor molecule to the first end of the digested double-stranded DNA molecule and ligating a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA and wherein the second adaptor molecule is a nucleic acid molecule that comprises one or more nuclease resistant nucleotides; and
(c) incubating the precursor double-stranded DNA molecule with a protelomerase to generate the partially closed DNA product, wherein the protelomerase closes the first end of the precursor double-stranded DNA molecule at the first functional protelomerase target sequence.

The step of appending a first adaptor molecule to the first end of the digested double-stranded DNA molecule and appending a second adaptor molecule to the second end of the digested double-stranded DNA molecule to generate a precursor double-stranded DNA molecule may be performed in the absence of a ligase, and may rely on hybridisation. Thus, the invention provides a method for producing a partially closed DNA product, wherein the method comprises:
a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase sequence is non-functional;
b) hybridizing a first adaptor molecule to the first end of the digested double-stranded DNA molecule and hybridizing a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides; and
c) incubating the precursor double-stranded DNA molecule with a protelomerase to generate the partially closed DNA product, wherein the protelomerase closes the first end of the precursor double-stranded DNA molecule at the first functional protelomerase target sequence.

A partially closed DNA product has particular utility as a therapeutic agent (i.e. DNA therapeutic) which can be used to express a gene product in vivo. This is because its partially closed structure prevents attack by enzymes such as exonucleases, leading to enhanced stability and longevity of gene expression as compared to "open" DNA molecules with exposed unprotected DNA ends. Linear double-stranded open-ended cassettes have been demonstrated to be inefficient with respect to gene expression when introduced into host tissue. This has been attributed to cassette instability due to the action of exonucleases in the extracellular space. Having one end of the partially closed DNA product closed gives it increased stability and efficiency, particularly when used in IVT

Sequestering DNA ends inside closed structures also has other advantages. The DNA ends are prevented from integrating with genomic DNA and so partially closed DNA products offer improved safety. In addition, the closed linear structure reduces concatamerisation of DNA products inside host cells and thus expression levels of the gene product can be regulated in a more sensitive manner.

The method of the invention may be used for production of DNA for in vitro expression in a host cell, for example, in DNA vaccines. DNA vaccines typically encode a modified form of an infectious organism's DNA. DNA vaccines are administered to a subject where they then express the selected protein of the infectious organism, initiating an immune response against that protein which is typically protective. DNA vaccines may also encode a tumour antigen in a cancer immunotherapy approach.

The method may produce other types of therapeutic DNA molecules e.g. those used in gene therapy. For example, such DNA molecules can be used to express a functional gene where a subject has a genetic disorder caused by a dysfunctional version of that gene. Examples of such diseases include sickle cell anaemia, cystic fibrosis, Huntington disease, Duchenne's Muscular Dystrophy, Haemophilia A, α1-antitrypsin deficiency, primary ciliary dyskinesia, or respiratory distress syndrome of prematurity. Other diseases where gene therapy may be useful include metabolic diseases, respiratory diseases, inflammatory diseases, autoimmune, chronic and infectious diseases, including such disorders as AIDS, cancer, neurological diseases, cardiovascular disease, hypercholesterolemia, various blood disorders including various anaemias, thalassemia and haemophilia, and emphysema. For the treatment of solid tumours, genes encoding toxic peptides (i.e., chemotherapeutic agents such as ricin, diptheria toxin and cobra venom factor), tumour suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The closing of the linear double-stranded region of the precursor double-stranded DNA molecule by the first adaptor molecule and the protecting of the second end by the second adaptor molecule may be performed by hybridization or ligation of the adaptor molecules to the ends of the linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The closing of the linear double-stranded region by the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region.

The steps of digesting a double-stranded DNA molecule with an endonuclease that cleaves an endonuclease target sequence to generate a digested double-stranded DNA molecule; appending a first adaptor molecule to the first end of the digested double-stranded DNA molecule and appending a second adaptor molecule to the second end of the digested double-stranded DNA molecule to generate a precursor double-stranded DNA molecule; and incubating the precursor double-stranded DNA molecule with a protelomerase to generate the partially closed DNA product, may be performed in a single contiguous aqueous volume. When the method of the invention is performed in a single contiguous aqueous volume, it may be performed under conditions that promote appending (or linking) of the first and second adaptor molecules to the linear double-stranded region to produce the partially closed DNA product. The appending may be performed by creating a covalent link between the first and/or second adaptor molecule and the first and/or second end of the linear double-stranded region.

The single contiguous aqueous volume may be incubated under conditions that promote digestion of the double-stranded DNA molecule to produce the linear region of the double-stranded DNA molecule. The digestion of the double-stranded DNA molecule to produce the digested double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

If the step of digesting the double-stranded DNA molecule with an endonuclease that cleaves an endonuclease target sequence to generate a digested double stranded DNA molecule is carried out as a separate reaction, it may be performed under conditions that promote digestion of the double-stranded DNA molecule to produce the linear region of the double-stranded DNA molecule. The digestion of the double-stranded DNA molecule to produce the digested double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

When the method if performed as a single contiguous aqueous volume, the single contiguous aqueous volume may be incubated under conditions that promote ligation of the digested double-stranded DNA molecule to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested double-stranded DNA molecules may be incorporated into closed linear DNA products. Preferably, the ligation is at least 15% efficient.

If the step of appending or ligating a first adaptor molecule to the first end of the digested double-stranded DNA molecule and appending or ligating a second adaptor molecule to the second end of the digested double stranded DNA molecule to generate a precursor double-stranded DNA molecule is carried out as a separate reaction, it may be performed under conditions that promote ligation of the digested double-stranded DNA molecule to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested double-stranded DNA molecules may be incorporated into closed linear DNA products. Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining open DNA constructs and adaptor molecules excess.

For example, the double-stranded DNA molecule generated by the rolling circle amplification is first quantified so that the amount of the double-stranded DNA molecule used as the starting material during the digestion/ligation/protelomerase reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the digested double-stranded DNA molecule to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The single contiguous aqueous volume may be incubated under conditions that promote the action of protelomerase to cleave and covalently close the protelomerase target sequence to form the closed linear DNA product. The incubation of the precursor double-stranded DNA molecule with protelomerase may be carried out at a third temperature of 1°C-100°C, 1°C -80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

If the step of incubating the precursor double-stranded DNA molecule with a protelomerase to generate the closed linear DNA product is performed as a separate reaction, it may be performed at a third temperature of 1°C-100°C, 1°C -80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

The single contiguous aqueous volume may be performed in a step comprising incubating at a first temperature and then incubating at a second temperature and/or then incubating at a third temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. The third temperature may be 1°C-100°C, 1°C -80°C, 5°C-70°C, 10°C-60°C, 15°C-55°C, 20°C-50°C, 25°C -45°C, 30°C-40°C, 26°C-34°C, 27°C-33°C 25°C-35°C, 28°C-32°C, 29°C-31°C or at about 30°C.

Preferably, the first temperature is 35°C-39°C and the second temperature is 14°C-18°C and the third temperature is 28°C-32°C. Using these conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or *E. coli* DNA ligase and the protelomerase may be TelN protelomerase from *E. coli* phage N15 TelN protelomerase along with the sequence defined herein. Alternatively, the protelomerase may be Klebsiella phage Phi K02 protelomerase, Yersinia phage PY54 protelomerase, Vibrio phage VP882 protelomerase, Borrelia burgdorferi protelomerase.

If a protelomerase other than TelN is used, the truncated protelomerase target sequence may be a truncated variant of a protelomerase target sequence specific for the protelomerase enzyme that is used.

The single contiguous aqueous volume may be incubated isothermally. The step of incubating the single contiguous aqueous volume may comprise incubating at a constant temperature. The constant temperature promotes simultaneous digestion of the double-stranded DNA molecule to produce the digested double-stranded DNA molecule and ligation of the linear double-stranded region to the first and second adaptor molecules and closing of the precursor double-stranded DNA molecule with protelomerase. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature and/or the third temperature. The step of incubating the single contiguous aqueous volume may comprise cycling between the first temperature and the second temperature and/or the third temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature and/or the third temperature less than 40, less than 35, less than 30 times, less than 29, less than 25 times. The step of incubating the single contiguous aqueous volume may comprise cycling between the first temperature and the second temperature and/or the third temperature 2-100, 5-80, 10-70, 20-60, or 30-60 times. The step of incubating the single contiguous aqueous volume may comprise cycling between the first temperature and the second temperature and/or the third temperature 2-20, 5-29, 61-100, or 65-80 times.

The method may further comprise, before step (a) (i.e. the step of digesting a double-stranded DNA molecule with an endonuclease that cleaves an endonuclease target sequence to generate a digested double-stranded DNA molecule), a step of amplifying a DNA template molecule to produce the double-stranded DNA molecule. Thus, the invention provides a method for producing a partially closed DNA product, the method comprises:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides; and
(d) incubating the precursor double-stranded DNA molecule with a protelomerase to generate the partially closed DNA product, wherein the protelomerase closes the first end of the precursor double-stranded DNA molecule at the first functional protelomerase target sequence.

The step of amplifying may be performed by in vitro or in vivo amplification. Preferably, the step of amplifying is performed by in vitro amplification. For example, the step of amplifying may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the step of amplifying is performed by rolling circle amplification. Thus, the invention provides a method for producing a partially closed DNA product, the method comprises:
(a) amplifying a template DNA molecule comprising a truncated protelomerase target sequence and an endonuclease target sequence to generate a double-stranded DNA molecule by rolling circle amplification, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides; and
(d) incubating the precursor double-stranded DNA molecule with a protelomerase to generate the partially closed DNA product, wherein the protelomerase closes the first end of the precursor double-stranded DNA molecule at the first functional protelomerase target sequence.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

In the methods described herein, the DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso311, BspCNI, BspMI, BspPI, BspQI, BspTNI, BsrDI, Bsrl, Bst6I, BstF51, BstMAI, BstV11, BstV21, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva12691, NmeAIII, PaqCI, PciSI, Pctl, Plel, PpsI, Psrl, Schl, SfaNI, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the partially closed DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF51, BstMAI, BstV11, BstV21, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCI, PciSI, Pctl, Plel, PpsI, Psrl, Schl, SfaNI, Taqll, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The protelomerase may be TelN protelomerase from *E. coli* phage N15 TelN protelomerase along with the sequence defined herein. Alternatively, the protelomerase may be Klebsiella phage Phi K02 protelomerase, Yersinia phage PY54 protelomerase, Vibrio phage VP882 protelomerase, Borrelia burgdorferi protelomerase.

If a protelomerase other than TelN is used, the truncated protelomerase target sequence may be a truncated variant of a protelomerase target sequence specific for the protelomerase enzyme that is used.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The method may further comprise (after the step of amplification and before the step of digesting the double-stranded DNA molecule with an endonuclease that cleaves the endonuclease target sequence to generate a digested double-stranded DNA molecule) a step of heat-deactivation. Thus, the invention provides a method for producing a partially closed DNA product, the method comprises:
(a) amplifying a template DNA molecule comprising a truncated protelomerase target sequence and an endonuclease target sequence to generate a double-stranded DNA molecule, wherein the truncated protelomerase sequence is non-functional;
(b) heat-deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides; and .
(e) incubating the precursor double-stranded DNA molecule with a protelomerase to generate the partially closed DNA product, wherein the protelomerase closes the first end of the precursor double-stranded DNA molecule at the first functional protelomerase target sequence.

Preferably, amplification is rolling-circle amplification.

The step of heat-deactivation may be performed under conditions sufficient to inactive the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

In the method described herein, after the step of amplification, the step of digesting a double-stranded DNA molecule with an endonuclease that cleaves an endonuclease target sequence to generate a digested double-stranded DNA molecule may be performed without purifying the product of the amplification reaction. That is to say that the step of digesting a double-stranded DNA molecule with an endonuclease that cleaves an endonuclease target sequence to generate a digested double-stranded DNA molecule, may be performed directly after the step of amplification. The step of digesting a double-stranded DNA molecule with an endonuclease that cleaves an endonuclease target sequence to generate a digested double-stranded DNA molecule may be performed directly after the step of heat-deactivation.

The method may further comprise, after the step of incubating the precursor double-stranded DNA molecule with a protelomerase to generate the partially closed DNA product, a step of purification of the partially closed DNA product.

The method may further comprise, after the step of incubating the precursor double-stranded DNA molecule with a protelomerase to generate the partially closed DNA product, a step of nuclease digestion. The nuclease digestion may be exonuclease digestion, such as exonuclease I and/or exonuclease III digestion. The step of nuclease digestion may take place before or after the step of purification. This step allows for removal of any double-stranded DNA molecules and/or adaptor molecules which have not been used in the course of performing the method. Thus, the method may comprise the steps:
a) amplifying a template DNA molecule comprising a truncated protelomerase target sequence and an endonuclease target sequence to generate a double-stranded DNA molecule, wherein the truncated protelomerase sequence is non-functional;
b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides;
d) incubating the precursor double-stranded DNA molecule with a protelomerase to generate the partially closed DNA product, wherein the protelomerase closes the first end of the precursor double-stranded DNA molecule at the first functional protelomerase target sequence; and
e) incubating the reaction of step (d) with a nuclease (e.g. exonuclease).

In the method described herein, after the step of amplification, the step of digesting the double-stranded DNA molecule with an endonuclease that cleaves the endonuclease target sequence to generate a digested double-stranded DNA molecule may be performed without purifying the product of the amplification reaction. That is to say that the step of digesting the double-stranded DNA molecule with an endonuclease that cleaves the endonuclease target sequence to generate a digested double-stranded DNA molecule may be performed directly after the step of amplification. The step of digesting the double-stranded DNA molecule with an endonuclease that cleaves the endonuclease target sequence to generate a digested double-stranded DNA molecule may be performed directly after the step of heat-deactivation.

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides;
(d) incubating the precursor double-stranded DNA molecule with a protelomerase to generate the partially closed DNA product, wherein the protelomerase closes the first end of the precursor double-stranded DNA molecule at the first functional protelomerase target sequence;
(e) purifying the partially closed DNA product; and
(f) incubating the purified product of step (e) with a nuclease (e.g. exonuclease).

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) heat deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides;
(e) incubating the precursor double-stranded DNA molecule with a protelomerase to generate the partially closed DNA product, wherein the protelomerase closes the first end of the precursor double-stranded DNA molecule at the first functional protelomerase target sequence;
(f) purifying the partially closed DNA product; and
(g) incubating the purified product of step (f) with a nuclease (e.g. exonuclease).

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(c) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides;
(d) incubating the precursor double-stranded DNA molecule with a protelomerase to generate the partially closed DNA product, wherein the protelomerase closes the first ens of the precursor double-stranded DNA molecule at the first functional protelomerase target sequence;
(e) incubating the reaction of step (d) with a nuclease (e.g. exonuclease); and
(f) purifying the partially closed DNA product.

The method may comprise the steps:
(a) amplifying a template DNA molecule comprising a truncated protelomerase target sequence and an endonuclease target sequence to generate a double-stranded DNA molecule, wherein the truncated protelomerase sequence is non-functional;
(b) heat deactivation of the reaction of step (a);
(c) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end;
(d) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides;
(e) incubating the precursor double-stranded DNA molecule with a protelomerase to generate the partially closed DNA product, wherein the protelomerase closes the first end of the precursor double-stranded DNA molecule at the first functional protelomerase target sequence;
(f) incubating the reaction of step (e) with a nuclease (e.g. exonuclease); and
(g) purifying the partially closed DNA product.

Steps c) to f) may be carried out in a single contiguous aqueous volume. The single contiguous aqueous volume (or the purified product of step (g)) may be incubated with a nuclease and may be performed at a temperature of 5-90°C, 10-80°C, 15-70°C, 20-60°C, 25-50°C, 30-45°C or 35-40°C. The step of incubating the single contiguous aqueous volume (or the purified product of step (g)) with a nuclease may be performed for at least 10, at least 20, at least 30, at least 40, at least 50, or at least 60 min. The step of incubating the single contiguous aqueous volume (or the purified product of step (g)) may be performed at two different temperatures. For example, the step of incubating the single contiguous aqueous volume (or the purified product of step (g)) may be performed at 15-40°C for 10-60 minutes followed by a temperature of 60-90°C for 10-30 min. The higher temperature typically inactivates the nuclease (e.g. exonuclease). Thus, the method further provides a step of inactivating the nuclease (e.g. exonuclease). The step of incubating the single contiguous aqueous volume (or the purified product of step (g)) may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The methods of the invention may comprise a (further) step of heat-deactivation after the step of incubating with the protelomerase and prior to treatment with an exonuclease. The heat-deactivation may be carried out after the steps of digesting a double-stranded DNA molecule to generate a digested double-stranded molecule; appending a first adaptor molecule to a first end of the digested double stranded DNA molecule and appending a second adaptor molecule to a second end of the digested double-stranded DNA molecule to generate a precursor double-stranded DNA molecule; and incubating the precursor double-stranded DNA molecule with a protelomerase to generate the partially closed DNA product, whether the steps are performed in a single contiguous aqueous volume or whether the step are carried out sequentially in separate reactions.

Thus, the method of the invention may comprise:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides;
(c) incubating the precursor double-stranded DNA molecule with a protelomerase to generate the partially closed DNA product, wherein the protelomerase closes the first end of the precursor double-stranded DNA molecule at the first functional protelomerase target sequence; and
(d) heat-deactivation of the reaction of steps (a) to (c), or step (c).

Heat-deactivation may be carried out at a temperature of 50-90°C, preferably from 60-80°C, more preferably from 70 to 80°C, 72-77 or 75°C. The heat deactivation may be performed from 1 to 10 minutes, from 2 to 9 minutes, for 3 to 8 minutes, from 4 to 7 minutes, for 6 minutes or, most preferably for 5 minutes.

The method may be a cell-free method.

The partially closed DNA product may be partially double-stranded and/or partially single-stranded. The partially closed DNA product may comprise a portion that is double-stranded and a portion that is single-stranded.

The partially closed DNA product may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The partially closed DNA product may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long.

The partially closed DNA product may comprise an inverted terminal repeat sequence.

The partially closed DNA product may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the partially closed DNA product is at least 50 base pairs long.

The double-stranded DNA molecule may be circular, or branched.

The double-stranded DNA molecule may not comprise an adaptor. The double-stranded DNA molecule may not comprise a hairpin, a loop or a stem-loop structure.

The double-stranded DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule.

The double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may comprise one or more cleavable (e.g. endonuclease) target sequences. The double-stranded DNA molecule may comprise two cleavable (e.g. endonuclease) target sequences. The one or more cleavable (e.g. endonuclease) target sequences may be Type IIS endonuclease target sequences. The one or more cleavable (e.g. endonuclease) target sequences may be Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF5I, BstMAI, BstV1I, BstV21, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp11091, Lwel, Mboll, Mlyl, Mmel, Mnll, Mva1269I, NmeAIII, PaqCI, PciSI, Pctl, Plel, PpsI, Psrl, Schl, SfaNI, Taqll, TspDTI and/or TspGWI target sequences.

The double-stranded DNA molecule may be a product of amplification. Preferably, the amplification is rolling circle amplification.

The linear double-stranded region (e.g. of the double-stranded DNA molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The linear double-stranded region (e.g. of the double-stranded DNA molecule) may comprise a sequence that is at least 40%, 45%, 50%, 55%, 60%, 65%, 70%, 75%, 80%, 85%, 90%, 95%, or 100% identical to the sequence of the double-stranded DNA molecule.

The first end and the second end of the linear double-stranded region (e.g. of the double-stranded DNA molecule) may be resistant to nuclease digestion. Preferably, the first end and the second end of the linear double-stranded region are resistant to the exonuclease digestion, such as exonuclease III digestion and/or exonuclease I digestion.

The linear double-stranded region may comprise a 3'-OH group at first and/or second ends. The 3'-OH group may facilitate ligation to the first and/or second adaptor molecule(s) (which may comprise a 5' phosphate). The linear double-stranded region may comprise a 5' phosphate at first and/or second ends. The 5' phosphate may facilitate ligation to the first and/or second adaptor molecule(s) (which may comprise a 3'-OH group).

The linear double-stranded region (e.g. of the double-stranded DNA molecule) may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the sense strand or the antisense strand of the linear double-stranded region.

The linear region of the double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, double-stranded DNA molecule is at least 50 base pairs long.

The double-stranded DNA molecule comprises a truncated protelomerase sequence, wherein the truncated protelomerase sequence is non-functional. The truncated protelomerase target sequence may be a truncated TelN protelomerase target sequence. The truncated protelomerase target sequence may consist of SEQ ID NO:s 4, 5, 6 or 20. The truncated protelomerase sequence may consist of SEQ ID NO: 4. The truncated protelomerase sequence may consist of SEQ ID NO: 5. The truncated protelomerase sequence may consist of SEQ ID NO: 6. The truncated protelomerase sequence may consist of SEQ ID NO: 20. The truncated protelomerase sequence may consist of SEQ ID NO:23, SEQ ID NO: 30, SEQ ID NO: 31 or SEQ ID NO: 32. The truncated protelomerase sequence may consist of SEQ ID NO: 23. The truncated protelomerase sequence may consist of SEQ ID NO: 30. The truncated protelomerase sequence may consist of SEQ ID NO: 31. The truncated protelomerase sequence may consist of SEQ ID NO: 32.

The first adaptor molecule and/or the second adaptor molecule may be a synthetic adaptor molecule.

The first adaptor molecule may be a nucleic acid adaptor molecule. The second adaptor molecule may be a nucleic acid adaptor molecule. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region ( of the double-stranded DNA molecule) may comprise a 3' or a 5' overhang.

A portion of the first adaptor molecule (e.g. the overhang) may be complementary to the first end of the linear double-stranded region. A portion of the second adaptor molecule may be complementary to the second end of the linear double-stranded region.

The first adaptor molecule may comprise a truncated protelomerase target sequence. The first and/or second adaptor molecule may or may not comprise all base pairs that are deleted from the truncated protelomerase sequence present in the double-stranded DNA molecule, e.g., if the truncated protelomerase target sequence in the double stranded DNA molecule is missing eight base pairs, those eight base pairs may be present in the adaptor molecule sequence, or fewer than those eight base pairs may be present in the adaptor molecule. The portion of a protelomerase target sequence present in the first adaptor molecule are sufficient such that, wherein the first adaptor molecule is appended to a first end of the double-stranded DNA molecule comprising a truncated protelomerase target sequence, the protelomerase target sequence is rendered functional and the protelomerase is able to cleave and ligate the sequence to covalently close the ends of the adaptor molecules and therefore the ends of the DNA molecule. The protelomerase target sequence may not be fully complete when the adaptor molecule is appended, provided that some activity is restored. The amount of activity restored may be 20%, 30%, 40%, 50%, 60% 70%, 80%, 90% or 100% of the full protelomerase activity with reference to the full protelomerase target sequence. The truncated protelomerase target sequences of the DNA molecule and/or of the adaptor molecule may comprise one or more point mutations, provided that activity is restored by the protelomerase target sequence formed by the adaptor molecule appending to the DNA molecule. The point mutations may be present in the truncated protelomerase target sequence of the double-stranded DNA molecule and/or in the truncated protelomerase target sequence of the adaptor molecule(s).

The first adaptor molecule comprises a truncated protelomerase target sequence, which may be a truncated TelN protelomerase target sequence. The first adaptor molecule may comprise a sequence selected from SEQ ID NO:8, 9, 10, 11, 12 or 13. The first adaptor molecule may consist of a pair of complementary sequences selected from SEQ ID NO:s 8 and 9, SEQ ID NO:s 10 and 11 or SEQ ID NO:s 12 or 13. The first adaptor molecule may comprise a sequence selected from SEQ ID NO:24, 25, 26, 27, 28 or 29. The first adaptor molecule may consist of a pair of complementary sequences selected from SEQ ID NO:s 24 and 25, SEQ ID NO:s 26 and 27 or SEQ ID NO:s 28 or 29.

The partially closed DNA product may be a partially covalently closed DNA product. Thus, in embodiments where an adaptor molecules comprises a truncated protelomerase target sequence, protelomerase covalently closes the end of the adaptor molecule appended to the end of linear double stranded region which forms a first functional protelomerase target sequence, to form a covalently partially closed DNA product. In embodiments where protelomerase closes one end (e.g., the first end) of the linear double-stranded region, the second adaptor does not close the other end of the precursor double stranded DNA molecule, (i.e., it remains open-ended and protected from nuclease digestion by protected nucleotides), to form a covalently partially closed DNA product.

The invention provides a method for producing a covalently partially closed DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides, wherein (i) the first adaptor molecule comprises an overhang that is complementary to and anneals to an overhang at the first end of the linear double-stranded region and (ii) the second adaptor molecule comprises an overhang that is complementary to and anneals to an overhang at the second end of the linear double-stranded region; and
(c) incubating the precursor double-stranded DNA molecule with a protelomerase to generate the partially closed DNA product, wherein the protelomerase closes the first end of the precursor double-stranded DNA molecule at the first functional protelomerase target sequence.

The invention provides a method for producing a covalently partially closed DNA product, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nuclease resistant nucleotides, wherein (i) the first adaptor molecule comprises an overhang that is complementary to and anneals to an overhang at the first end of the linear double-stranded region and (ii) the second adaptor molecule comprises an overhang that is complementary to and anneals to an overhang at the second end of the linear double-stranded region, and wherein the first adaptor molecule is ligated to the first end of the linear double-stranded region and the second adaptor molecule is ligated to the second end of the linear double-stranded region; and
(c) incubating the precursor double-stranded DNA molecule with a protelomerase to generate the partially closed DNA product, wherein the protelomerase closes the first end of the precursor double-stranded DNA molecule at the first functional protelomerase target sequences.

The first adaptor molecule and/or the second adaptor molecule may not be a plasmid or a vector DNA.

The first adaptor molecule comprises a truncated protelomerase target sequence, which may be a truncated TelN protelomerase target sequence. The truncated protelomerase target sequence may comprise a partial telR sequence. The truncated protelomerase target sequence may comprise a partial telR sequence and a partial telO sequence. The first adaptor molecule may comprise 10, 11, 12, 13, 14, 15, 16 17, 18 19, 20, 21, 22, 23, 24, 25, 26, 27, 28 base pairs of a telR sequence. Preferably, the adaptor comprises at least 21 base pairs of a telR sequence. The truncated protelomerase target sequence may comprise a partial telL sequence. The truncated protelomerase target sequence may comprise a partial tell sequence and a partial telO sequence. The first adaptor molecule may comprise 10, 11, 12, 13, 14, 15, 16 17, 18 19, 20, 21, 22, 23, 24, 25, 26, 27, 28 base pairs of a telL sequence. Preferably, the adaptor comprises at least 21 base pairs of a telL sequence.

The first and/or second adaptor molecules may comprise the same sequence or different sequences, i.e., the first adaptor molecule may comprise a different sequence in the overhang portion such that it only appends (by hybridization and/or ligation) to a first end of the digested double-stranded DNA molecule, and not to the second end.

The first and second adaptor molecules may comprise one or more phosphorothioated nucleotides, such that, once the adaptor molecules are appended (e.g. ligated) to the linear double-stranded region, the partially closed linear DNA product is resistant to nuclease digestion or has improved or enhanced resistance to nuclease digestion. The partially closed linear DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may be a nucleic acid adaptor molecule. The adaptor molecule may be double-stranded. The adaptor molecule may comprise a portion that is double-stranded.

The first and/or second adaptor molecules may comprise at least 3, at least 4, at least 5, at least 6, at least 7, at least 8, at least 9, at least 10, at least 11, at least 12, at least 13, at least 14, at least 15, or at least 16 base pairs.

The first and/or second adaptor molecule may comprise a plurality of phosphorothioated nucleotides in each strand. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

Once the adaptor molecules are appended to the linear double-stranded region, the partially closed linear DNA product may comprise a protected nucleotide (e.g. phosphorothioated nucleotide) at the 5'-end (or at the 5'-end region) of one or both strands. Preferably, the partially closed linear DNA product comprises a phosphorothioated nucleotide at the 5'-end (or at the 5'-end region) of one strand. The partially closed linear DNA product may comprise a phosphorothioated nucleotide at the 5'-end (or at the 5'-end region) of one strand. As most exonucleases, for example exonuclease III, remove nucleotides from the 3'-end of the polynucleotide chain, the linear DNA product may comprise a protected nucleotide at the 3'-end (or at the 3'-end region) of one strand. Preferably, the partially closed linear DNA product comprises a phosphorothioated nucleotide at the 3'-end (or the 3'-end region) of one strand. The partially closed linear DNA product may comprise at least one phosphorothioated nucleotide at the 3'-end (or the 3'-end region) and at least one phosphorothioated nucleotide at the 5'-end (or the 5'-end region) of one strand. The partially closed linear DNA product may comprise at least one phosphorothioated nucleotide at the 3'-end (or the 3'-end region) of the sense strand and the 5'-end (or the 5'-end region) if the antisense strand. The partially closed linear DNA product may comprise at least one phosphorothioated nucleotide at the 5'-end (or the 5'-end region) of the sense strand and the 3'-end (or the 3'-end region) if the antisense strand. Thus, both the sense and antisense strands in the double stranded partially closed linear DNA product may be protected from nuclease digestion by using nuclease-resistant nucleotides at one end of the partially closed linear DNA product.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the linear double-stranded region (which may comprise a 3'-OH group at first and/or second ends). The first adaptor molecule and/or the second adaptor molecule may comprise a 3'-OH. The 3'-OH may facilitate ligation to the linear double-stranded region (which may comprise a 5' phosphate at first and/or second ends). The single-stranded portion of the second adaptor molecule may comprise a sequence of ACTCA. The single-stranded portion of the first adaptor molecule and/or the second adaptor molecule may comprise at least 1, at least 2, at least 3, at least 4 or at least 5 contiguous nucleotides of the sequence ACTCA.

The first and/or second adaptor molecule may comprise the sequence of SEQ ID NO: 14 or a portion thereof. The first and/or second adaptor molecule may comprise at least 13, at least 14, at least 15, at least 16, at least 17, at least 18, or at least 19 contiguous nucleotides of SEQ ID NO: 14. The double-stranded portion of the first and/or second adaptor molecule may comprise the sequence of SEQ ID NO: 15 or a portion thereof. The double-stranded portion of the first and/or second adaptor molecule may comprise at least 8, at least 9, at least 10, at least 11, at least 12, at least 13, at least 14 or at least 15 contiguous nucleotides of SEQ ID NO: 15. The single-stranded portion of the first and/or second adaptor molecule may comprise a sequence of ACTCA. The single-stranded portion of the first adaptor molecule and/or the second adaptor molecule may comprise at least 1, at least 2, at least 3, at least 4 or at least 5 contiguous nucleotides of the sequence ACTCA. The second adaptor molecule may comprise or consist of the sequences of SEQ ID NO:18 and/or SEQ ID NO:19.

The first and second adaptor molecules may comprise an identical nucleic acid sequence. The first and second adaptor molecules may comprise a different nucleic acid sequence.

The first adaptor molecule may comprise a portion that is complementary to the first end of the digested double-stranded DNA molecule. The second adaptor molecule may comprise a portion that is complementary to the second end of the digested double-stranded DNA molecule. The first adaptor molecule may comprise a portion that anneals to the first end of the digested double-stranded DNA molecule. The second adaptor molecule may comprise a portion that anneals to the second end of the digested double-stranded DNA molecule The first adaptor molecule may comprise a portion that is complementary and anneals to the first end of the digested double-stranded DNA molecule. The second adaptor molecule may comprise a portion that is complementary and anneals to the second end of the digested double-stranded DNA molecule).

The portion that is complementary or anneals to the first or second end of the digested double-stranded DNA molecule may be a 5' overhang or a 3' overhang of the first and/or second adaptor molecule. The overhang of the first adaptor molecule may be complementary to the first end of the digested double-stranded DNA molecule and/or the overhang of the second adaptor molecule may be complementary to the second end of the digested double-stranded DNA molecule. The overhang of the first adaptor molecule may anneal to the first end of the digested double-stranded DNA molecule and/or the overhang of the second adaptor molecule may anneal to the second end of the digested double-stranded DNA molecule. The overhang of the first adaptor molecule may be complementary to and anneal to the first end of the digested double-stranded DNA molecule and/or the overhang of the second adaptor molecule may be complementary to and anneal to the second end of the digested double-stranded DNA molecule.

The first adaptor molecule and/or the second adaptor molecule may not comprise a Type IIS endonuclease target sequence. The first adaptor molecule and/or the second adaptor molecule may not comprise Bbsl, Bsal, BsmBI, BspQI, BtgZI, Esp3I,SapI, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse1I, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF5I, BstMAI, BstV11, BstV2I, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva12691, NmeAIII, PaqCI, PciSI, Pctl, Plel, PpsI, Psrl, Schl, SfaNI, Taqll, TspDTI and/or TspGWI target sequences.

The first adaptor molecule and/or the second adaptor molecule may comprise a Type IIS endonuclease target sequence, provided it is different to a type IIS endonuclease target sequence that is present in the double-stranded DNA molecule.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides. The protected nucleotides may be located in the double-stranded portion. The protected nucleotides may be located in the overhang portion of the adaptor molecules. The first and second adaptor molecules may comprise one or more phosphorothioated nucleotides, such that, once the adaptor molecules are appended (e.g. ligated) to the linear double-stranded region, the partially closed linear DNA product is resistant to nuclease digestion or has improved or enhanced resistance to nuclease digestion. The partially closed linear DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The partially closed DNA product may comprise a plurality of phosphorothioated nucleotides at internal positions in each strand. For example, the partially closed DNA product may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions in each strand. Preferably, the partially closed DNA product comprises at least 2 protected nucleotides (e.g. phosphorothioated nucleotides) at internal positions in each strand.

The internal positions may not be located between the second and penultimate nucleotide of the partially closed DNA product.

The linear double-stranded region (of the double-stranded DNA molecule) may comprise a plurality of phosphorothioated nucleotides at internal positions in each strand. For example, the linear double-stranded region (of the double-stranded DNA molecule) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) at internal positions in each strand. Preferably, the linear double-stranded region (of the double-stranded molecule) comprises at least 2 protected nucleotides (e.g. phosphorothioated nucleotides) at internal positions in each strand. The internal positions may not be located between the second and penultimate nucleotide of the linear double-stranded region (of the double-stranded molecule).

The nucleotides resistant to exonuclease digestion (i.e. protected nucleotides) may be phosphorothioated nucleotides of at least one type. For example, the at least one type of phosphorothioated nucleotides is α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The adaptor molecules may comprise at least four types of phosphorothioated nucleotides. For example, the at least four types of protected nucleotides are α-S-dATP, α-S-dCTP, α-S-dGTP and α-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first end of the linear double-stranded region may be complementary to a portion of the first adaptor molecule. The second end of the linear double-stranded region may be complementary to a portion of the second adaptor molecule. The first end and/or the second end of the linear double-stranded region may be generated by endonuclease digestion.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probetarget base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell.

The targeting sequence may be used to increased transfection efficiency of non-viral gene delivery by virtue of enhanced nuclear import of the partially closed DNA product. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the DNA product, the functional portion may comprise a fluorophore, a radioactive compound or a barcode.

A signal corresponding to the presence, absence and/or level of the partially closed DNA product may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the partially closed DNA product. The binding moiety is capable of binding to 3' and/or 5' end of the partially closed DNA product. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the partially closed DNA product and the second binding moiety linked to the second barcoded portion may bind to the 5' end of the partially closed DNA product. The 3' and 5' ends may comprise a 3' overhang, a 5' overhang or a blunt end.

A signal corresponding to the presence, absence and/or level of the partially closed DNA product may be measured using a fluorophore (i.e. a fluorescently-labelled molecule), which is attached or bound to the 3' overhang, the 5' overhang or the blunt end of the partially closed DNA product. The signal may be measured by flow cytometry and/or fluorescence-activated cell sorting.

The functional portion may also facilitate DNA sequencing. For example, the functional portion may be a sequencing adaptor. The term "sequencing adaptor" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeg^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different serotypes. An inverted terminal repeat sequence may comprise a terminal resolution site and a Rep binding site.

The first adaptor molecule and/or the second adaptor molecule may comprise an aptamer.

The first adaptor molecule and/or the second adaptor molecule may confer resistance to the nuclease digestion, such as exonuclease digestion (e.g. exonuclease I and/or exonuclease III digestion).

The closing of one end of the linear double-stranded region (of the precursor double-stranded DNA molecule) at the first end by protelomerase may generate a first closed end of the partially closed DNA product. The protection of the linear double-stranded region (of the double-stranded DNA molecule) at the second end by the appending, or ligation, of the second adaptor may generate a protected open end of the partially closed DNA product. The first closed end and the second open end of the partially closed DNA product may be resistant to nuclease digestion. The nuclease digestion may be exonuclease digestion. Preferably, the nuclease digestion is exonuclease III digestion and/or exonuclease I digestion.

An example of the method of the invention is provided with reference to Figure 1, which illustrates the workflow to obtain the closed linear DNA product by digestion and ligation of adaptor molecules in a single step, starting from amplified DNA obtained through rolling circle amplification of a circular DNA template generated through the action of Cre recombinase on substrates containing two LoxP sequences (SEQ ID NO:3) flanking the DNA of interest in the same direction.

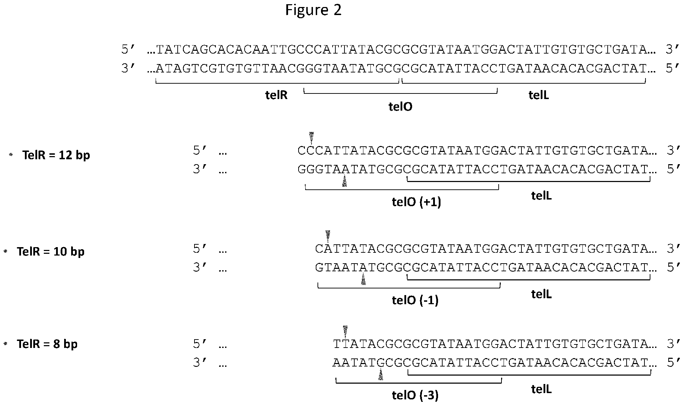

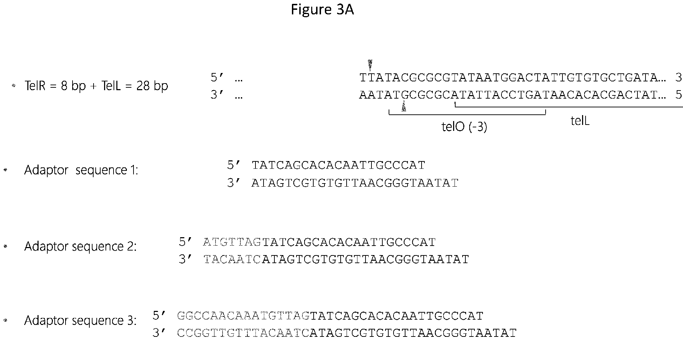

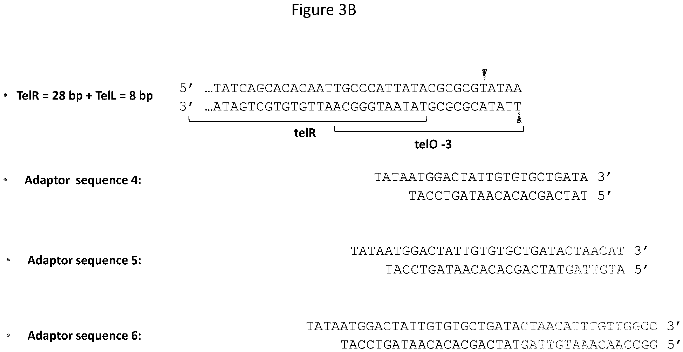

The method is further described with reference to Figures 2 and 3, which illustrate the sequences of the truncated TelN protelomerase target sequence (SEQ ID NO:s 4-6, 20, 23 and 30-32) and the adaptor molecule ligation after endonuclease digestion of an amplified double-stranded DNA molecule in each cycle of the process. Endonuclease digestion produces 4-nucleotide protruding ends at 5' at each sides of the expression cassette. Adaptor molecules (SEQ ID NO:8 to 13 and 24 to 29) containing a 4-nucleotide protruding end at 5' is then appended to either end of the expression cassette; and incubation with protelomerase results in the closed ends of the DNA product due to the formation of a functional protelomerase target sequence by the appending of the truncated protelomerase target sequence of the adaptor molecule to the truncated protelomerase target sequence of the double stranded DNA molecule.

### 4. Methods for transcription and protein expression

Described herein is a method for in vitro transcription of a linear DNA product (e.g. a closed linear DNA product or a partially closed linear DNA product), wherein the method comprises contacting the linear DNA product (e.g. a closed linear DNA product or a partially closed linear DNA product), produced by the methods described herein, with a polymerase and producing a transcription product encoded by the linear DNA product (e.g. the closed linear DNA product or a partially closed linear DNA product).

Described herein is a method for in vitro transcription of a linear DNA product (e.g. a closed linear DNA product or a partially closed linear DNA product), wherein the method comprises:
(a) producing a linear DNA product (e.g. a closed linear DNA product or a partially closed linear DNA product) by any of the methods described herein;
(b) contacting the linear DNA product (e.g. the closed linear DNA product or a partially closed linear DNA product), with a polymerase; and
(c) producing a transcription product encoded by the linear DNA product (e.g. the closed linear DNA product or a partially closed linear DNA product).

Described herein is a method for in vitro transcription of a linear DNA product, wherein the method comprises:
a. digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
b. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
c. incubating the precursor double-stranded DNA molecule with a protelomerase to generate the linear DNA product, wherein the protelomerase closes the first end of the precursor double-stranded DNA molecule at the first functional protelomerase target sequence;
d. contacting the linear DNA product with a polymerase; and
e. producing a transcription product encoded by the linear DNA product.

The method may use adaptor molecules which generate a closed linear DNA product, such as adaptor molecules described herein. A method for in vitro transcription of a closed linear DNA product may comprise:
a) digesting a double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
c) incubating the precursor double-stranded DNA molecule with a protelomerase to generate the closed linear DNA product, wherein the protelomerase closes the first and second ends of the precursor double-stranded DNA molecule at the first and second functional protelomerase target sequences;
(c) contacting the closed linear DNA product with a polymerase; and
(d) producing a transcription product encoded by the closed linear DNA product.

A method for in vitro transcription of a closed linear DNA product may comprise:
a. digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
b. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
c. incubating the precursor double-stranded DNA molecule with a protelomerase to generate the partially closed linear DNA product, wherein the protelomerase closes the first end of the precursor double-stranded DNA molecule at the first functional protelomerase target sequence, and the second end is closed by the second adaptor molecule;
d. contacting the closed linear DNA with a polymerase; and
e. producing a transcription product encoded by the closed linear DNA.

The method may use adaptor molecules which comprise protected nucleotides, and adaptor molecules that comprise a truncated protelomerase sequence, such as the adaptor molecules described herein. A method for in vitro transcription of a partially closed linear DNA product may comprise:
a. digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
b. appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nucleotide resistant nucleotides; and
c. incubating the precursor double-stranded DNA molecule with a protelomerase to generate the partially closed linear DNA product, wherein the protelomerase closes the first end of the precursor double-stranded DNA molecule at the first functional protelomerase target sequence;
d. contacting the linear DNA product with a polymerase; and
e. producing a transcription product encoded by the linear DNA product.

Described herein is a method for producing a protein, wherein the method comprises introducing the linear DNA product (e.g. the closed linear DNA product or the partially closed linear DNA product), produced by the methods described herein, into a cell (e.g. a prokaryotic cell or a eukaryotic cell) or a cell-free expression system to generate a protein encoded by the linear DNA product (e.g. the closed linear DNA product or the partially closed linear DNA product).

Described herein is a method for producing a protein, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(c) incubating the precursor double-stranded DNA molecule with a protelomerase to generate the linear DNA product, wherein the protelomerase closes the first end of the precursor double-stranded DNA molecule at the first functional protelomerase target sequence; and
(d) introducing the linear DNA product into a call (e.g., a prokaryotic cell or a eukaryotic cell) or a cell-free expression system to generate a protein encoded by the linear DNA product.

The method may use adaptor molecules which generate a closed linear DNA product, such as adaptor molecules described herein. A method for producing a protein may comprise:
a) digesting a double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
c) incubating the precursor double-stranded DNA molecule with a protelomerase to generate the closed linear DNA product, wherein the protelomerase closes the first and second ends of the precursor double-stranded DNA molecule at the first and second functional protelomerase target sequences; and
d) introducing the linear DNA product into a call (e.g., a prokaryotic cell or a eukaryotic cell) or a cell-free expression system to generate a protein encoded by the linear DNA product.

A method for producing a protein may comprise:
a) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
c) incubating the precursor double-stranded DNA molecule with a protelomerase to generate the closed linear DNA product, wherein the protelomerase closes the first end of the precursor double-stranded DNA molecule at the first functional protelomerase target sequence, and the second end is closed by the second adaptor molecule; and
d) introducing the closed linear DNA product into a call (e.g., a prokaryotic cell or a eukaryotic cell) or a cell-free expression system to generate a protein encoded by the closed linear DNA product.

The method may use adaptor molecules which comprise protected nucleotides, and adaptor molecules that comprise a truncated protelomerase sequence, such as the adaptor molecules described herein. A method for producing a protein may comprise:
a) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nucleotide resistant nucleotides; and
c) incubating the precursor double-stranded DNA molecule with a protelomerase to generate the partially closed linear DNA product, wherein the protelomerase closes the first end of the precursor double-stranded DNA molecule at the first functional protelomerase target sequence; and
d) introducing the partially closed linear DNA product into a call (e.g., a prokaryotic cell or a eukaryotic cell) or a cell-free expression system to generate a protein encoded by the partially closed linear DNA product.

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The linear DNA product or the closed linear DNA product may comprise a cassette. The desired protein might be encoded by the cassette.

The step of introducing the linear DNA product into a cell may be performed in vivo or in vitro.

The nuclease-resistant nucleotides (i.e. protected nucleotides) may be any protected nucleotides described herein.

### 5. Methods for cell transfection and cell transfection compositions

Described herein is a method for cell transfection of a linear DNA product (e.g. a closed linear DNA product or a partially closed linear DNA product), produced by any of the methods described herein, into a cell.

Described herein is a method for cell transfection of a linear DNA product (e.g., a closed linear DNA product or a partially closed linear DNA product), wherein the method comprises:
a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
c) incubating the precursor double-stranded DNA molecule with a protelomerase to generate the linear DNA product, wherein the protelomerase closes the first end of the precursor double-stranded DNA molecule at the first functional protelomerase target sequence; and
d) contacting the linear DNA product with the cell; and
e) transfecting the linear DNA product into the cytosol of the cell.

The method may use adaptor molecules which generate a closed linear DNA product, such as adaptor molecules described herein. A method for cell transfection of a closed linear DNA product into a cell may comprise:
a) digesting a double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
c) incubating the precursor double-stranded DNA molecule with a protelomerase to generate the closed linear DNA product, wherein the protelomerase closes the first and second ends of the precursor double-stranded DNA molecule at the first and second functional protelomerase target sequences;
d) contacting the closed linear DNA product with the cell; and
e) transfecting the closed linear DNA product into the cytosol of the cell.

A method for cell transfection of a closed linear DNA product into a cell may comprise:
(a) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(c) incubating the precursor double-stranded DNA molecule with a protelomerase to generate the closed linear DNA product, wherein the protelomerase closes the first end of the precursor double-stranded DNA molecule at the first functional protelomerase target sequence, and the second end is closed by the second adaptor molecule; and
(d) contacting the closed linear DNA product with the cell; and
(e) transfecting the closed linear DNA product into the cytosol of the cell.

The method may use adaptor molecules which comprise protected nucleotides, and adaptor molecules that comprise a truncated protelomerase sequence, such as the adaptor molecules described herein. A method for cell transfection of a partially closed linear DNA product into a cell may comprise:
a) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nucleotide resistant nucleotides; and
c) incubating the precursor double-stranded DNA molecule with a protelomerase to generate the partially closed linear DNA product, wherein the protelomerase closes the first end of the precursor double-stranded DNA molecule at the first functional protelomerase target sequence;
d) contacting the partially closed linear DNA product with the cell; and
e) transfecting the closed linear DNA product into the cytosol of the cell.

Described herein is a cell transfection composition comprising a linear DNA product (e.g. a closed linear DNA product) produced by (or obtainable by) the methods described herein.

The cell transfection composition may or may not comprise a carrier e.g. an agent or a formulation. Preferably, the carrier promotes accumulation of the linear DNA product at a target site and/or protects the linear DNA product from undesirable interactions with biological milieu components and/or protects the linear DNA product from metabolism and/or degradation.

Described herein is a cell transfection method comprising contacting (in vitro) a cell to be transfected with a linear DNA product (e.g. a closed linear DNA product) produced by the methods of the invention, and wherein the linear DNA product (e.g. the closed linear DNA product) is transfected into the cytosol of the cell.

The cell or cells to be transfected may be provided in a cell culture medium (e.g. in a Petri dish, culture vessel or well, etc). The linear DNA product (e.g. the closed linear DNA product) may be added directly to the cell culture medium or the cells may be added to a solution, such as saline, a buffered solution or a cell culture medium, comprising the linear DNA product (e.g. the closed linear DNA product).

The carrier may be a viral carrier or a non-viral carrier. Viral carriers include a lentiviral vector, an adenoviral vector, a retroviral vector, or an adeno associate viral vector for delivery of the linear DNA product. Non-viral carriers (or vectors) include complexing the linear DNA product with a cationic agent such as a cationic cell penetrating peptide (CPP); a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the linear DNA product. The carrier may be a nanoparticulate formulation used to encapsulate the linear DNA product (e.g. the closed linear DNA product).

The carrier may be a modification of the linear DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the linear DNA product at a target site and/or protects the linear DNA product from metabolism and/or degradation.

Described herein is a cell obtainable by the methods as described herein. Thus, the cell may comprise a linear DNA product (e.g. the closed linear DNA product) produced by the methods of the invention.

Described herein is a cell transfected with a linear DNA product (e.g. the closed linear DNA product) produced by the methods of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a linear DNA product (e.g. the closed linear DNA product) may be performed in vivo. For example the linear DNA product (e.g. the closed linear DNA product) may be administered to an organism (e.g. a subject) in need thereof. The organism (e.g. the subject) may be an animal, such as mammal (e.g. a human), a fungus, a micro-organism, or a plant.

Any or all of the linear DNA products (e.g. the closed linear DNA products) described herein may be delivered to a cell via delivery particles such as liposomes, nanoparticles, exosomes, macrovesicles, viral or non-viral vectors. Any or all of the linear DNA products (e.g. the closed linear DNA products) described herein may be delivered to a cell using a gene-gun. Any or all of the linear DNA products (e.g. the closed linear DNA products) described herein may be delivered to a cell by electroporation. Any or all of the linear DNA products (e.g. the closed linear DNA products) described herein may be delivered to a cell by hydrodynamic needle. The linear DNA products (e.g. the closed linear DNA products) described herein may be delivered to a cell without a carrier.

The nanoparticle is preferably a self-assembled nanoparticle. The nanoparticle may be a nanoparticle which is produced by a process in which pre-existing components (e.g. a lipid component, a DNA product described herein) form an organized structure as a consequence of specific, local interactions among the components themselves, without external direction.

The DNA product and the lipid component may reversibly interact to form a self-assembled nanoparticle. The DNA product and the lipid component may reversibly interact in the self-assembled nanoparticle through intermolecular forces. The DNA product and the lipid component may reversibly interact in the self-assembled nanoparticle through non-covalent interactions. The DNA product and the lipid component may reversibly interact in the self-assembled nanoparticle through hydrogen bonds, van der Waals, hydrophobic, and/or electrostatic interactions. The DNA product and the lipid component may not be conjugated or linked by forces other than inter-molecular forces in the self-assembled nanoparticle.

### 6. Pharmaceutical compositions and methods for producing pharmaceutical compositions

Described herein is a pharmaceutical composition comprising a linear DNA product (e.g. a closed linear DNA product) described herein, and a pharmaceutically acceptable carrier or excipient.

Described herein is a pharmaceutical composition comprising a linear DNA product (e.g. a closed linear DNA product) produced by (or obtainable by) the methods described herein, and a pharmaceutically acceptable carrier or excipient.

Described herein is a method for producing a pharmaceutical composition comprising the linear DNA product, wherein the method comprises performing the method described herein and formulating the resulting linear DNA product with a pharmaceutically acceptable carrier or excipient.

Described herein is a method for producing a pharmaceutical composition, wherein the method comprises:
(a) producing a linear DNA product (e.g. a closed linear DNA product) by any of the methods described herein;
(b) formulating the linear DNA product (e.g. the closed linear DNA product) with a pharmaceutically acceptable carrier or excipient.

Described herein is a method for producing a pharmaceutical composition, wherein the method comprises:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(c) incubating the precursor double-stranded DNA molecule with a protelomerase to generate the linear DNA product, wherein the protelomerase closes the first end of the precursor double-stranded DNA molecule at the first functional protelomerase target sequence; and
(d) formulating the linear DNA product with a pharmaceutically acceptable carrier or excipient

The method may use adaptor molecules which generate a closed linear DNA product, such as adaptor molecules described herein. A method for producing a pharmaceutical composition may comprise:
a) digesting a double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase sequences are non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule;
c) incubating the precursor double-stranded DNA molecule with a protelomerase to generate the closed linear DNA product, wherein the protelomerase closes the first and second ends of the precursor double-stranded DNA molecule at the first and second functional protelomerase target sequences; and
d) formulating the closed linear DNA product with a pharmaceutically acceptable carrier or excipient

A method for formulating a pharmaceutical composition may comprise:
(a) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule;
(c) incubating the precursor double-stranded DNA molecule with a protelomerase to generate the closed linear DNA product, wherein the protelomerase closes the first end of the precursor double-stranded DNA molecule at the first functional protelomerase target sequence, and the second end is closed by the second adaptor molecule; and
(d) formulating the closed linear DNA product with a pharmaceutically acceptable carrier or excipient.

The method may use adaptor molecules which comprise protected nucleotides, and adaptor molecules that comprise a truncated protelomerase sequence, such as the adaptor molecules described herein. A method for formulating a pharmaceutical composition may comprise:
a) digesting the double-stranded DNA molecule with an endonuclease that cleaves the endonuclease target sequence to generate a digested double-stranded DNA molecule, wherein the digested double-stranded DNA molecule comprises a linear double-stranded region, and a truncated protelomerase sequence at a first end, wherein the truncated protelomerase sequence is non-functional;
b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule is a nucleic acid molecule that comprises one or more nucleotide resistant nucleotides; and
c) incubating the precursor double-stranded DNA molecule with a protelomerase to generate the partially closed linear DNA product, wherein the protelomerase closes the first end of the precursor double-stranded DNA molecule at the first functional protelomerase target sequence; and
d) formulating the partially closed linear DNA product with a pharmaceutically acceptable carrier or excipient

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the linear DNA product (e.g. closed linear DNA product) can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### 7. Linear DNA products

Described herein is a linear DNA product (e.g. a closed linear DNA product).

Described herein is a closed linear DNA product comprising a linear double-stranded region, and wherein the linear double-stranded region is closed at a first end by a first adaptor molecule wherein the first adaptor molecule is closed by protelomerase, and closed at a second end by the second adaptor molecule. The end of a closed linear DNA product that has been closed by protelomerase closing the first adaptor molecule may comprise the sequence CGCGCG forming at least part of the resulting covalently closed end. Therefore, described herein is a closed linear DNA product comprising a linear double-stranded region, and wherein the linear double-stranded region is closed at a first end by a first adaptor molecule wherein the first adaptor molecule is closed by protelomerase and may comprise the sequence CGCGCG forming at least part of the covalently closed first end, and is closed at a second end by the second adaptor molecule

Described herein is a closed linear DNA product comprising a linear double-stranded region of a double-stranded DNA molecule, wherein the linear double-stranded region of the double-stranded DNA molecule is closed at a first end by a first adaptor molecule and closed at a second end by a second adaptor molecule, wherein the first adaptor molecule and the second adaptor molecule are each closed by protelomerase. The closed ends of a linear DNA product that have been closed by protelomerase closing the first adaptor molecule and the second adaptor molecule may comprise the sequence CGCGCG forming at least part of each of the resulting covalently closed end. Therefore, described herein is a closed linear DNA product comprising a linear double-stranded region, and wherein the linear double-stranded region is closed at a first end by a first adaptor molecule wherein the first adaptor molecule is closed by protelomerase and may comprise the sequence CGCGCG forming at least part of the covalently closed first end, and is closed at a second end by a second adaptor molecule comprising a hairpin.

Described herein is a partially closed linear DNA product comprising a linear double-stranded region, and wherein the first adaptor molecule is ligated to a first end of the linear double-stranded region and the second adaptor molecule is ligated to a second end of the linear double-stranded region, and wherein double-stranded DNA molecule is closed at the first end by a first adaptor molecule, and the first adaptor molecule is closed by protelomerase, and the second adaptor molecule isa nucleic acid molecule that comprises one or more nuclease-resistant nucleotides. Thus, described herein is a partially closed linear DNA product which is closed (or covalently closed) at a second end and open at a first end. The partially closed linear DNA product comprises one or more nuclease-resistant nucleotides in the open-end region and/or at the first end. The open-end region may be at the 3' end or 5' end of the molecule.

The end of a partially closed linear DNA product that has been closed by protelomerase closing the first adaptor molecule may comprise the sequence CGCGCG in the resulting covalently closed end. Therefore, described herein is a partially closed linear DNA product comprising a linear double-stranded region, and wherein the linear double-stranded region is closed at a first end by a first adaptor molecule wherein the first adaptor molecule is closed by protelomerase and may comprise the sequence CGCGCG forming part of the sequence at the covalently closed first end, and the second adaptor molecule ligated to the second end is a nucleic acid molecule that comprises one or more nuclease-resistant nucleotides

The open-end region refers to at least 5, at least 10, at least 15, or at least 20 base pairs located closest to the open end of the DNA product. The partially closed linear DNA product may comprise one or more nuclease-resistant nucleotides in a sense and/or an antisense strand. Thus, for example, one or more nucleotides of the 20 base pairs located closest to the open end of the partially closed linear DNA product may be a nuclease-resistant nucleotide. Preferably, the partially closed linear DNA product comprises at least 5 nuclease-resistant nucleotides in the open-end region.

The partially closed linear DNA product may comprise a linear double-stranded region that is closed at a first end and open at a second end. The partially closed linear DNA product may comprise a double-stranded DNA portion that is closed at a first end by a single-stranded portion (i.e. it may comprise a first hairpin at the first end) and open at a second end. The closed end is closed by protelomerase. The partially closed linear DNA product may comprise one or more nuclease-resistant nucleotides in an open-end region adjacent to the second end. The open-end region adjacent to the second end may be at the 3' end or 5' end of the molecule. The open-end region adjacent to the second end may comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 nucleotides located at the second end of the partially closed linear DNA product. This is to say that the open-end region adjacent to the second end may comprise any nucleotide between and including the end nucleotide of the second end and a nucleotide at location 2, 3, 4, 5, 6, 7, 8, 9, 10, 11, 12, 13, 14, 15, 16, 17, 18, 19, 20, 25, 30, 35, 40, 45, or 50 counting from the end nucleotide of the second end.

The open-end region adjacent to the second end may comprise a sense strand and an antisense strand. The open-end region adjacent to the second end may comprise one or more nuclease-resistant nucleotides in the sense strand or the antisense strand. The open-end region adjacent to the second end may comprise one or more nuclease-resistant nucleotides in both the sense and antisense strand. The open-end region adjacent to the second end may comprise two or more, three or more, four or more, or five or more nuclease-resistant nucleotides in both the sense and antisense strand. Preferably, the open-end region adjacent to the second end comprises five nuclease-resistant nucleotides in both the sense and antisense strand.

The partially closed linear DNA product may be closed by protelomerase at the 5' end or the 3' end. The partially closed linear DNA product may comprise a first adaptor molecule at a first end and a second adaptor molecule at a second end. The first adaptor molecule may be closed by protelomerase and a second adaptor may comprise one or more protected nucleotides (i.e. nucleotides resistant to nuclease (e.g. exonuclease) digestion). The closed end may confer resistance to nuclease (e.g. exonuclease) digestion. The presence of protected nucleotides may confer resistance to nuclease (e.g. exonuclease) digestion. The partially closed linear DNA product may be a DNA molecule having a double-stranded region closed at a first end by ligation of a first adaptor, which is closed by protelomerase, to the first end and comprising at a second end a double stranded linear adaptor comprising protected nucleotides (i.e. nucleotides resistant to nuclease (e.g. exonuclease) digestion).

The partially closed linear DNA product may comprise (i) a cassette, wherein the cassette comprises a sense strand and an antisense strand; and (ii) one or more nuclease-resistant nucleotides in an open-end region of the partially closed linear DNA product, wherein the open-end region is 5' of the sense strand of the cassette. The partially closed linear DNA product may comprise (i) a cassette, wherein the cassette comprises a sense strand and an antisense strand; and (ii) one or more nuclease-resistant nucleotides in an open-end region of the partially closed linear DNA product, wherein the open-end region is 5' of the antisense strand of the cassette. The partially closed linear DNA product may comprise (i) a cassette, wherein the cassette comprises a sense strand and an antisense strand; and (ii) one or more nuclease-resistant nucleotides in an open-end region of the partially closed linear DNA product, wherein the open-end region is 3' of the sense strand of the cassette. The partially closed linear DNA product may comprise (i) a cassette, wherein the cassette comprises a sense strand and an antisense strand; and (ii) one or more nuclease-resistant nucleotides in an open-end region of the partially closed linear DNA product, wherein the open-end region is 3' of the antisense strand of the cassette.

The partially closed linear DNA product may comprise (i) a cassette, wherein the cassette comprises a sense strand and an antisense strand; (ii) one or more nuclease-resistant nucleotides in an open-end region of the partially closed linear DNA product, wherein the open-end region is 5' of the sense strand of the cassette; and (iii) one or more nuclease-resistant nucleotides in an open-end region of the partially closed linear DNA product, wherein the open-end region is 3' of the antisense strand of the cassette.

The partially closed linear DNA product may comprise (i) a cassette, wherein the cassette comprises a sense strand and an antisense strand; (ii) one or more nuclease-resistant nucleotides in an open-end region of the partially closed linear DNA, wherein the open-end region is 3' of the sense strand of the cassette; and (iii) one or more nuclease-resistant nucleotides in an open-end region of the partially closed linear DNA product, wherein the open-end region is 5' of the antisense strand of the cassette.

The closed linear DNA product, linear DNA product or partially closed linear DNA product may comprise a cassette, optionally a single cassette. The linear region of a double-stranded DNA molecule (of a closed linear DNA product, linear DNA product or partially closed linear DNA product) may comprise a cassette, optionally a single cassette. Accordingly, the cassette (or single cassette) is located between the first and second adaptor molecules.

The term "single cassette" as used herein is intended to encompass a molecules that do not comprise or consist of a plurality of cassettes. That is to say that the closed linear DNA product, linear DNA product, or partially closed linear DNA product, comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

Described herein is a linear DNA product (e.g. a closed linear DNA product or a partially closed linear DNA product) obtainable by any of the methods described herein.

### 8. Uses and applications

Described herein is a use of a linear DNA product (e.g. a closed linear DNA product) as described herein in the production of viral or non-viral delivery system.

Described herein is a use of a linear DNA product (e.g. a closed linear DNA product) in the production of viral or non-viral delivery system, wherein the linear DNA product (e.g. the closed linear DNA product) is produced by performing the method described herein.

Described herein is a viral or non-viral delivery system comprising a linear DNA product (e.g. a closed linear DNA product) as described herein. Described herein is a viral or non-viral delivery system comprising a linear DNA product (e.g. a closed linear DNA product), wherein the linear DNA product (e.g. the closed linear DNA product) is produced by performing the method described herein.

### Viral vectors

Methods of producing viral vectors, such as AAV vectors, are known in the art. The most widely used method involves the co-transfection of HEK293 by three bacterial plasmids. The first plasmid encodes the *Rep* and Cap element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli*; 3) the incorporation of the plasmid backbone into the viral capsid (e.g. AAV capsid) might be challenging (e.g. issues with antibiotic resistance markers). Methods of producing viral vectors may be using other cell lines. For example, a cell line suitable for production of viral vectors may be a Vero cell, or any other stable cell line. Together these represent the main bottleneck in viral vector production (e.g. AAV production). Thus, the methods described herein provide a linear DNA product (e.g. a closed linear DNA product) suitable for use in production of viral vectors. The linear DNA product (e.g. the closed linear DNA product) overcomes the above issues with plasmid vectors.

Described herein is a method of producing a viral vector, the method comprising introducing the linear DNA product (e.g. a closed linear DNA product) produced by the methods described herein into a cell under conditions such that the viral vector is produced. The linear DNA product (e.g. the closed linear DNA product) may encode at least one element required for the production of the viral vector. For example, the linear DNA product (e.g. the closed linear DNA product) may encode Rep and/or Cap elements. The linear DNA product (e.g. the closed linear DNA product) may encode the helper plasmid elements. The linear DNA product (e.g. the closed linear DNA product) may encode Rep, Cap and helper plasmid elements. The linear DNA product (e.g. the closed linear DNA product) may encode a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

Described herein is a method of delivering the viral vector (e.g. the closed linear DNA product) to a cell, comprising contacting the viral vector produced by the methods described herein with the cell. The cell may be an animal cell, preferably mammal cell, such as human cell.

Described herein is a cell obtainable by the methods described herein.

### Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the linear DNA product (e.g. a closed linear DNA product) produced by the methods described herein is suitable for use in production of non-viral vectors. The linear DNA product (e.g. the closed linear DNA product) produced by the methods of the invention overcomes the issues of using plasmid vectors in non-viral vector preparation. For example, the linear DNA product (e.g. the closed linear DNA product), unlike plasmid DNA, does not comprise a bacterial backbone, allowing more transgene copies per mg of DNA. In addition, the linear DNA product (e.g. the closed linear DNA product) does not comprise antibiotic resistant genes and bacterial contaminants. Moreover, the linear DNA product (e.g. the closed linear DNA product) provides a prolonged transgene expression (due to the presence of exonuclease-resistant nucleotides), and a more cost-effective process of non-viral vector production.

Described herein is a method of delivering the non-viral vector to a cell, comprising contacting the non-viral vector comprising the linear DNA product (e.g. a closed linear DNA product) with the cell. The cell may be an animal cell, preferably mammal cell, such as human cell.

Described herein is a cell obtainable by the methods described herein.

### General therapeutic and diagnostic uses

The linear DNA product (e.g. a closed linear DNA product) produced by the methods described herein is particularly suitable for use in therapy. Described herein is a linear DNA product (e.g. a closed linear DNA product) as described herein for use in therapy. Described herein is a linear DNA product (e.g. a closed linear DNA product) obtainable by the method described herein for use in therapy. The linear DNA product (e.g. the closed linear DNA product) may encode a sequence of a therapeutic protein, a part of a vaccine, or an element of a genetic engineering mechanism, which is used to treat a disease or infection in a subject.

The linear DNA product (e.g. the closed linear DNA product) may encode a sequence of a therapeutic protein, a part of a vaccine, or an element of a genetic engineering mechanism, which is used to treat a disease or infection in a subject.

Described herein is the linear DNA product produced by the methods described herein for use in treating a disease.

The linear DNA product (e.g. the closed linear DNA product) described herein may be used to treat any disease or disorder. For example, the linear DNA product (e.g. the closed linear DNA product) may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product (e.g. the closed linear DNA product) is used to treat a genetic disorder. More preferably still, the linear DNA product (e.g. the closed linear DNA product) is used to treat a monogenic disorder. For example, the linear DNA product (e.g. the closed linear DNA product), may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

A subject treated with the linear DNA product (e.g. a closed linear DNA product) may receive the linear DNA product (e.g. a closed linear DNA product) in the form of any of the pharmaceutical compositions described herein.

A subject treated with the linear DNA product (e.g. a closed linear DNA product) may receive the linear DNA product (e.g. the closed linear DNA product) in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the linear DNA product into the subject's body as described in more detail above (see "Methods for producing a pharmaceutical composition"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the linear DNA product that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

Described herein is the use of the linear DNA product (e.g. a closed linear DNA product) as described herein in a method of diagnosing a disease and/or a disorder Described herein is the use of the linear DNA product (e.g. the closed linear DNA product) obtainable by the methods described herein in a method of diagnosing a disease and/or a disorder.

Described herein is the use of the linear DNA product (e.g. the closed linear DNA product) in the "in vitro" diagnosis of a disease. Described herein is the use of the linear DNA product (e.g. the closed linear DNA product) obtainable by the methods described herein in the "in vitro" diagnosis of a disease.

Described herein is the linear DNA product (e.g. the closed linear DNA product) for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to diagnose a genetic disorder. More preferably still, the linear DNA product is used to diagnose a monogenic disorder. For example, the linear DNA product may be used to diagnose sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the linear DNA product (e.g. closed linear DNA product).

To facilitate detection and/or quantification of the linear DNA product, the linear DNA product (e.g. the closed linear DNA product) may be attached or bound to a functional portion. The functional portion may be any functional portion described herein. For example the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the linear DNA product. For example, the signal may be measured by flow cytometry and/or fluorescence-activated cell sorting of a linear DNA product attached to a fluorescent probe.

The linear DNA product (e.g. the closed linear DNA product) may be detected by being bound to a capture moiety, for example in a lateral flow assay. In this example, the functional portion is a protein, for example, an antibody specific for the capture moiety. The capture of the antibody attached to the linear DNA product may produce a visual signal (e.g. a band of a different colour).

Described herein is a method that combines diagnosis of a disease with a treatment of the disease.

### Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the linear DNA product (e.g. the closed linear DNA product) may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

Described herein is the linear DNA product (e.g. the closed linear DNA product) described herein for use in cell therapy. Described herein is the linear DNA product (e.g. the closed linear DNA product) obtainable by the methods described herein for use in cell therapy.

Described herein is the linear DNA product (e.g. the closed linear DNA product) described herein for use in cell therapy. Described herein is the linear DNA product (e.g. the closed linear DNA product) obtainable by the methods described herein for use in cell therapy.

Preferably, cell therapy is ex-vivo cell therapy. The cell may be an animal cell, preferably mammal cell, such as human cell.

Described herein is a cell obtainable by any methods described herein. For example, the cell may be suitable for use in cell therapy.

### Vaccines

The linear DNA products (e.g. the closed linear DNA products) produced by the methods described herein are particularly suitable for use in vaccine production. A vaccine may comprise a linear DNA product (e.g. a closed linear DNA product) described herein. A vaccine may comprise a linear DNA product (e.g. a closed linear DNA product) obtainable by the methods described herein. Alternatively, the linear DNA product (e.g. the closed linear DNA product) may be used to produce a vaccine, preferably an mRNA-based vaccine. For example, vaccines such as the BioNTech and Moderna mRNA vaccines against COVID-19.

Described herein is the use of the linear DNA product (e.g. the closed linear DNA product) described herein in the production of a vaccine. Described herein is the use of the linear DNA product (e.g. the closed linear DNA product) obtainable by the methods described herein in the production of a vaccine.

The linear DNA product (e.g. the closed linear DNA product) may encode an antigen, which may cause an immune response in a subject. The subject may be human. Preferably, the antigen is encoded on the cassette.

### CAR-T cells

Described herein is the use of a linear DNA product (e.g. the closed linear DNA product) described herein in the production of a CAR-T cell. Described herein is the use of the linear DNA product (e.g. the closed linear DNA product) obtainable by the methods described herein in the production of a CAR-T cell.

Described herein is a method for producing a genetically engineered CAR-T cell comprising: (a) introducing the linear DNA product (e.g. a closed linear DNA product) described herein into a T cell; and (b) expressing a gene of interest encoding by the linear DNA product (e.g. the closed linear DNA product). Preferably, the gene of interest is a tumour-specific CAR.

Described herein is a method for producing a genetically engineered CAR-T cell comprising: (a) introducing the linear DNA product (e.g. a closed linear DNA product) obtainable by the methods described herein into a T cell; and (b) expressing a gene of interest encoding by the linear DNA product (e.g. the closed linear DNA product). Preferably, the gene of interest is a tumour-specific CAR.

The method may further comprise, before step (a), a step of removing mononuclear cells from a patient. Preferably, the step of removing is performed using leukapheresis. Preferably the mononuclear cells are T cells. The method may further comprise, after step (b), a step of returning the genetically engineered cells to the patient.

Described herein is an engineered T cell obtainable by any methods described herein. The engineered T cell may be suitable for use in CAR T-cell therapy.

### CRISPR delivery

The products produced by the methods described herein are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The linear DNA product (e.g. the closed linear DNA product) may comprise a gene sequence encoding any component of the CRIPSR machinery. The linear DNA product (e.g. the closed linear DNA product) may encode all components of the CRIPSR machinery.

The linear DNA product (e.g. the closed linear DNA product) may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1, or MAD7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The linear DNA product (e.g. the closed linear DNA product) encoding the repair template may be delivered to a cell by a nanoparticle, a non-viral vector or a viral vector, or without the aid of any carrier. The linear DNA product (e.g. the closed linear DNA product) encoding the repair template may be delivered to a cell by electroporation. The linear DNA product (e.g. the closed linear DNA product) encoding the repair template may be delivered to a cell by hydrodynamic needle.

The linear DNA product (e.g. the closed linear DNA product) may comprise (or further comprise) a gene sequence encoding a nuclease protein of a CRISPR system (e.g. Cas9, Cpf1, or MAD7), and/or a guide RNA. The linear DNA product (e.g. the closed linear DNA product) may comprise (or further comprise) a gene sequence encoding a nuclease protein of a CRISPR system (e.g. Cas9, Cpf1, or MAD7). The linear DNA product (e.g. the closed linear DNA product) may comprise (or further comprise) a gene sequence encoding a guide RNA. The linear DNA product (e.g. the closed linear DNA product) may comprise (or further comprise) a gene sequence encoding a nuclease protein of a CRISPR system (e.g. Cas9, Cpf1, or MAD7) and a guide RNA. The linear DNA product (e.g. the closed linear DNA product) may comprise (or further comprise) a gene sequence encoding a genomic target to be modified (e.g. a spacer). The linear DNA product (e.g. the closed linear DNA product) may be ligated to a vector. The vector may comprise a sequence of some of the components of the CRIPSR system. The vector may comprise a sequence of guide RNA or a sequence of a part of the guide RNA. If the vector comprises a sequence of a part of the guide RNA, the linear DNA product (e.g. the closed linear DNA product) may comprise the missing sequence part of the guide RNA, so that upon ligation, the ligated vector comprises a full sequence of a guide RNA. The nuclease of the CRISPR system and guide RNA may be encoded on a single vector or on two different vectors. The linear DNA product (e.g. the closed linear DNA product) may encode the nuclease of the CRISPR system and guide RNA. One linear DNA product (e.g. one closed linear DNA product) may encode the nuclease of the CRISPR system, and the other linear DNA product (e.g. the other closed linear DNA product) may encode guide RNA.

The linear DNA product (e.g. the closed linear DNA product) may be for use in CRISPR-Cas mediated repair by recombination, homology-directed repair, or non-homologous end joining.

If the nuclease of the CRISPR system and the guide RNA are encoded by a different linear DNA product (e.g. a different closed linear DNA product), they may be part of a different or the same delivery mechanism. For example, the linear DNA product (e.g. the closed linear DNA product) encoding the nuclease of the CRISPR system may be delivered to a cell by a first nanoparticle, non-viral vector or viral vector, whereas the linear DNA product (e.g. the closed linear DNA product) encoding the guide RNA may be delivered to a cell by a second nanoparticle, a non-viral vector, or viral vector. For example, the linear DNA product (e.g. the closed linear DNA product) encoding the nuclease of the CRISPR system and the linear DNA product (e.g. the closed linear DNA product) encoding the guide RNA may be delivered to a cell by the same nanoparticle, non-viral vector or viral vector.

If the nuclease of the CRISPR system and the guide RNA are encoded by the same linear DNA product (e.g. the closed linear DNA product) (or by a vector that comprises the linear DNA product (e.g. the closed linear DNA product)) they may be part of the same delivery mechanism. For example, the linear DNA product (e.g. the closed linear DNA product), or the vector, encoding the nuclease of the CRISPR system and the guide RNA may be delivered to a cell by a nanoparticle, a non-viral vector or a viral vector.

The linear DNA product (e.g. the closed linear DNA product) encoding the nuclease of the CRISPR system and the linear DNA product (e.g. the closed linear DNA product) encoding the guide RNA may be delivered to a cell by electroporation. The linear DNA product (e.g. the closed linear DNA product) encoding the nuclease of the CRISPR system and the linear DNA product (e.g. the closed linear DNA product) encoding the guide RNA may be delivered to a cell by hydrodynamic needle.

Described herein is the linear DNA product (e.g. the closed linear DNA product) described herein for use in CRISPR system delivery to a cell. Described herein is a method of delivering the CRISPR system to a cell, comprising contacting the linear DNA product (e.g. the closed linear DNA product) described herein with a cell. Described herein is the linear DNA product (e.g. the closed linear DNA product) obtainable by the methods described herein for use in CRISPR system delivery to a cell. Described herein is a method of delivering the CRISPR system to a cell, comprising contacting the linear DNA product (e.g. the closed linear DNA product) obtainable by the methods described herein with a cell.

The cell may be an animal cell, preferably mammal cell, such as human cell.

Described herein is a cell obtainable by the methods described herein. The cell is particularly suitable for use in cell therapy and/or in vivo therapy.

The linear DNA product produced by the methods described herein may be used in transcription, to generate RNA, preferably mRNA, in vitro or in vivo.

### 9. Kits

Described herein is a kit comprising components required to carry out the method described herein. The kit comprises at least:
(a) first and second adaptor molecules;
(b) an endonuclease; and
(c) a protelomerase.

The kit may additionally comprise a ligase, a DNA polymerase, at least one buffer and/or a nuclease.

The first adaptor molecule and/or the second adaptor molecule may comprise a sequence of SEQ ID NO: 13 or a portion thereof. The first adaptor molecule and/or the second adaptor molecule may comprise at least 13, at least 14, at least 15, at least 16, at least 17, at least 18, or at least 19 contiguous nucleotides of SEQ ID NO: 13. The first and second adaptor molecules may comprise an identical nucleic acid sequence. The first and second adaptor molecules may comprise a truncated protelomerase sequence. The first and second adaptor molecules may comprise a different nucleic acid sequence. The first adaptor molecule may comprise a truncated protelomerase sequence and the second adaptor molecule may comprise one or more protected nucleotides. The second adaptor molecule may comprise or consist of the sequence of SEQ ID NO:18 and/or SEQ ID NO:19. The first adaptor molecule may comprise a truncated protelomerase sequence and the second adaptor molecule may comprise a hairpin or stem loop region. The second adaptor molecule may comprise or consist of the sequence of SEQ ID NO:17.

The first adaptor molecule and/or the second adaptor molecule may comprise a truncated TelN protelomerase target sequence. The first and/or second adaptor molecules may comprise a sequence selected from SEQ ID NO:8, 9, 10, 11, 12 or 13. The first adaptor and/or the second adaptor molecule may consist of a pair of complementary sequences selected from SEQ ID NO:s 8 and 9, SEQ ID NO:s 10 and 11 or SEQ ID NO:s 12 or 13. The first adaptor molecule and/or the second adaptor molecule may comprise a sequence selected from SEQ ID NO:24, 25, 26, 27, 28 or 29. The first adaptor and/or the second adaptor molecule may consist of a pair of complementary sequences selected from SEQ ID NO:s 24 and 25, SEQ ID NO:s 26 and 27 or SEQ ID NO:s 28 or 29.

The first and second adaptor molecule may be provided in a kit together or separately.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3I,SapI, Aarl, Acc361, AclWI, Acul, Ajul, AloI, Alw261, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse11, Bse3DI, BseGI, BseMI, BseMII, BseNI, BseRI, BseXI, Bsgl, BsIFI, BsmAl, BsmFI, Bsml, Bso31I, BspCNI, BspMI, BspPI, BspQI, BspTNI, BsrDI, Bsrl, Bst6I, BstF51, BstMAI, BstV11, BstV2I, Bsul, BtgZI, BtsCl, Btsl-v2, BtsMutl, Bvel, Csel, CspCI, Eam11041, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, Plel, Ppsl, Psrl, Schl, SfaNI, Taqll, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The protelomerase may be TelN protelomerase from *E. coli* phage N15 TelN protelomerase along with the sequence defined herein. Alternatively, the protelomerase may be Klebsiella phage Phi K02 protelomerase, Yersinia phage PY54 protelomerase, Vibrio phage VP882 protelomerase, Borrelia burgdorferi protelomerase.

If a protelomerase other than TelN is used, the truncated protelomerase target sequence may be a truncated variant of a protelomerase target sequence specific for the protelomerase enzyme that is used.

The nuclease may be an exonuclease e.g. exonuclease I, exonuclease III and/or exonuclease VIII.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

### 10. Methods of producing linear DNA products with multiple adaptor molecules

The invention provides further methods of producing linear DNA products, such as closed linear DNA products, partially closed DNA products and linear DNA products comprising protected nucleotides.

The invention provides a method of producing a closed linear DNA product, wherein the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase target sequence is non-functional;
(b) appending a first terminal adaptor to the first end of the digested double-stranded DNA molecule, and sequentially appending m intermediate adaptor molecules to the second end of the digested double-stranded DNA molecule wherein m is an integer of at least one, and appending a second terminal adaptor molecule to the mth intermediate adaptor molecule, to generate a precursor double-stranded DNA molecule, wherein the first terminal adaptor molecule comprises a truncated protelomerase target sequence that forms a first functional protelomerase target sequence with the truncated protelomerase sequence at the first end of the digested double-stranded DNA molecule, and wherein the mth intermediate adaptor molecule comprises a truncated protelomerase sequence, wherein the truncated protelomerase target sequence is non-functional, and wherein the second terminal adaptor molecule comprises a truncated protelomerase target sequence that forms a second functional protelomerase target sequence with the truncated protelomerase target sequence of the mth intermediate adaptor molecule; and
(c) incubating the precursor double-stranded DNA molecule with a protelomerase to generate the closed linear DNA product, wherein the protelomerase closes the first end of the precursor double-stranded DNA molecule at the first functional protelomerase target sequence, and the protelomerase closes the second end of the precursor double-stranded DNA molecule at the second functional protelomerase target sequence.

The invention provides a method of producing a closed linear DNA product, wherein the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase target sequence is non-functional;
(b) appending a first terminal adaptor to the first end of the digested double-stranded DNA molecule, and sequentially appending m intermediate adaptor molecules to the second end of the digested double-stranded DNA molecule wherein m is an integer of at least one, and appending a second terminal adaptor molecule to the mth intermediate adaptor molecule, to generate a precursor double-stranded DNA molecule, wherein the first terminal adaptor molecule comprises a truncated protelomerase target sequence that forms a first functional protelomerase target sequence with the truncated protelomerase sequence at the first end of the digested double-stranded DNA molecule; and
(c) incubating the precursor double-stranded DNA molecule with a protelomerase to generate the closed linear DNA product, wherein the protelomerase closes the first end of the precursor double-stranded DNA molecule at the first functional protelomerase target sequence, and the second end is closed by the second terminal adaptor molecule.

The invention provides a method of producing a partially closed linear DNA product, wherein the method comprises the steps of:
a. digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end, wherein the truncated protelomerase target sequence is non-functional;
b. appending a first terminal adaptor to the first end of the digested double-stranded DNA molecule, and sequentially appending m intermediate adaptor molecules to the second end of the digested double-stranded DNA molecule wherein m is an integer of at least one, and appending a second terminal adaptor molecule to the mth intermediate adaptor molecule, to generate a precursor double-stranded DNA molecule, wherein the first terminal adaptor molecule comprises a truncated protelomerase target sequence that forms a first functional protelomerase target sequence with the truncated protelomerase sequence at the first end of the digested double-stranded DNA molecule, and the second terminal adaptor molecule comprises one or more nuclease-resistant nucleotides; and
c. incubating the precursor double-stranded DNA molecule with a protelomerase to generate the partially closed linear DNA product, wherein the protelomerase closes the first end of the precursor double-stranded DNA molecule at the first functional protelomerase target sequence,

The invention provides a method for producing a closed linear deoxyribonucleic acid (DNA) product, wherein the method comprises:
(a) contacting a double-stranded DNA molecule with an endonuclease, a ligase and first terminal adaptor molecule, a second terminal adaptor molecule and n + m intermediate adaptor molecules, to form a precursor linear DNA product, wherein n and m are an integer of at least 1, and wherein the nth and mth intermediate adaptor molecules each comprise a truncated protelomerase sequence, wherein the truncated protelomerase target sequence is non-functional, and wherein the first and second terminal adaptor molecule each comprise a truncated protelomerase target sequence that each forms a functional protelomerase target sequence with the truncated protelomerase target sequence of the respective nth and mth intermediate adaptor molecules;
(b) incubating the precursor linear DNA product with a protelomerase to generate the closed linear DNA product, wherein the closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and m intermediate adaptor molecules are sequentially appended to a second end of the linear double-stranded region, and wherein the protelomerase closes the first end of the precursor closed linear DNA product at the first functional protelomerase target sequence and closes the second end of the precursor closed linear DNA product at the second functional protelomerase target sequence.

The invention provides a method for producing a closed linear deoxyribonucleic acid (DNA) product, wherein the method comprises:
(a) contacting a double-stranded DNA molecule with an endonuclease, a ligase and first terminal adaptor molecule, a second terminal adaptor molecule and n + m intermediate adaptor molecules, to form a precursor closed linear DNA product, wherein n is an integer of at least 1, and m is 0 or an integer of at least 1, and wherein the nth intermediate adaptor molecule comprises a truncated protelomerase sequence, wherein the truncated protelomerase target sequence is non-functional, and wherein the first terminal adaptor molecule comprises a truncated protelomerase target sequence that forms a functional protelomerase target sequence with the truncated protelomerase target sequence of the nth intermediate adaptor molecule; and
(b) incubating the precursor partially closed linear DNA product with a protelomerase to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and the first terminal adaptor molecule is appended to the nth intermediate adaptor molecule, and m intermediate adaptor molecules are sequentially appended to a second end of the linear double-stranded region, and the second terminal adaptor molecule is appended to the mth intermediate adaptor molecule, or when m is 0, to the second end of the linear double stranded region, and wherein the protelomerase closes the first end of the precursor closed linear DNA product at the first functional protelomerase target sequence, and wherein the second end of the closed DNA product is closed by the second terminal adaptor molecule.

The invention provides a method for producing a closed linear deoxyribonucleic acid (DNA) product, wherein the method comprises:
(b) contacting a double-stranded DNA molecule with an endonuclease, a ligase and first terminal adaptor molecule, a second terminal adaptor molecule and n + m intermediate adaptor molecules, to form a precursor closed linear DNA product, wherein n is an integer of at least 1, and m is 0 or an integer of at least 1, and wherein the nth intermediate adaptor molecule comprises a truncated protelomerase sequence, wherein the truncated protelomerase target sequence is non-functional, and wherein the first terminal adaptor molecule comprises a truncated protelomerase target sequence that forms a functional protelomerase target sequence with the truncated protelomerase target sequence of the nth intermediate adaptor molecule; and
(b) incubating the precursor partially closed linear DNA product with a protelomerase to generate the partially closed linear DNA product, wherein the partially closed linear DNA product comprises a linear double-stranded region, wherein the linear double-stranded region comprises a linear portion of the double-stranded DNA molecule, and wherein n intermediate adaptor molecules are sequentially appended to a first end of the linear double stranded region and the first terminal adaptor molecule is appended to the nth intermediate adaptor molecule, and m intermediate adaptor molecules are sequentially appended to a second end of the linear double-stranded region, and the second terminal adaptor molecule is appended to the mth intermediate adaptor molecule, or when m is 0, to the second end of the linear double stranded region, and wherein the protelomerase closes the first end of the precursor closed linear DNA product at the first functional protelomerase target sequence, and wherein the second terminal adaptor molecule comprises one or more nuclease-resistant nucleotides.

### BRIEF DESCRIPTION OF THE DRAWINGS

**FIG. 1** illustrates the workflow to obtain closed linear DNA product by digestion and ligation of adaptor molecules and processing with protelomerase, starting from amplified DNA obtained through rolling circle amplification (RCA) of a circular DNA template generated through the action of Cre recombinase on substrates containing two LoxP sequences (SEQ ID NO: 3) flanking the DNA of interest in the same direction.
**FIG. 2A** illustrates the sequence of the full length TelN protelomerase target sequence, and three of the truncated sequences that were tested for ability to be processed by protelomerase. The red arrows indicate where the sequence is digested by an endonuclease (e.g, Bsal) for compatibility with adaptor molecules. For example the telR + 8bp sequence and cut site is compatible with the adaptors shown in FIG. 3A
**FIG. 3A** shows the sequence of the inactive site, in which the TelR region has been reduced to 8 bp only. FIG. 3 also shows the sequences of three different adaptor molecules formed by complementary oligonucleotides. The three DNA adaptors restore the complete TelN target site if hybridized or ligated to the reduced TelR region after endonuclease digestion. Adaptor hybridization and ligation are driven by sequence complementarity of the protruding ends generated by digestion with a type IIS restriction endonuclease (i.e. Bsal).
**FIG. 3B** shows the sequence of the inactive site, in which the TelL region has been reduced to 8 bp only. FIG. 3B also shows the sequences of three different adaptor molecules formed by complementary oligonucleotides. The three DNA adaptors restore the complete TelN target site if hybridized or ligated to the reduced TelL region after endonuclease digestion. Adaptor hybridization and ligation are driven by sequence complementarity of the protruding ends generated by digestion with a type IIS restriction endonuclease (i.e. Bsal).

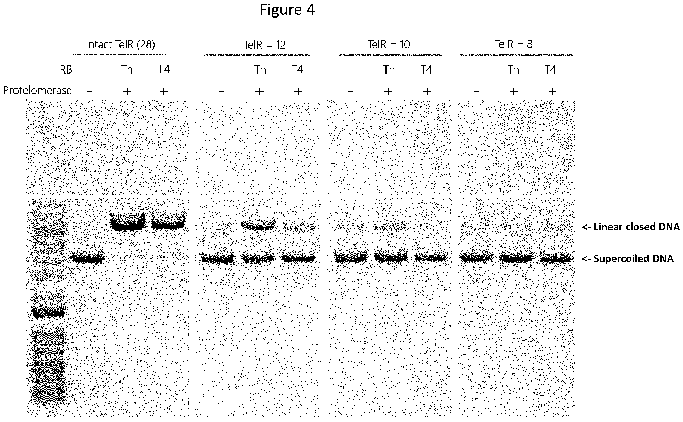

**FIG. 4** shows the enzymatic processing of different plasmid DNAs by TelN protelomerase. Two different reaction buffers are shown: ThermoPol^{®} reaction buffer ("Th") and T4 DNA ligase buffer ("T4"). Four different plasmids were tested (SEQ. ID 1, 2, 3 and 4). Positive control plasmid contained a single and complete TeIN site (SEQ. ID NO:1), formed by TelR (SEQ. ID NO:21) and TelL (SEQ. ID NO:22) sequences, each one having 28 bp. The TelR region of the other three plasmids was reduced to 12 (SEQ. ID NO:2), 10 (SEQ. ID NO: 3) or 8 bp (SEQ. ID NO: 4) of telR remaining, respectively. Native agarose gel electrophoresis (0,6%) showed the capacity to convert supercoiled plasmid DNA into linear closed DNA by TelN protelomerase depending on the length of the TelR sequence and the reaction buffer used.

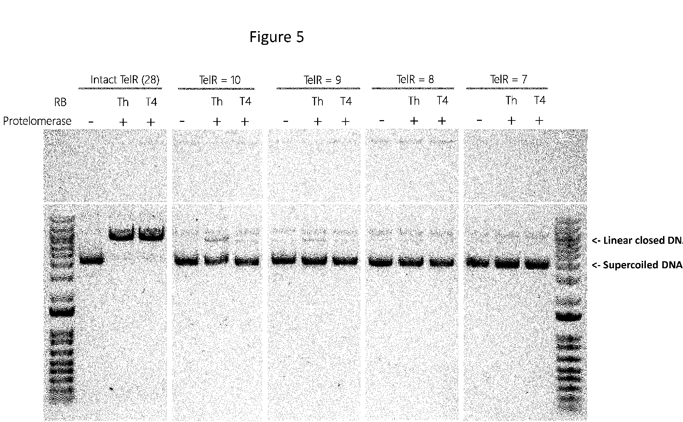

**FIG. 5** shows the enzymatic processing of different plasmid DNAs by TelN protelomerase. Two different reaction buffers are shown: ThermoPol^{®} reaction buffer ("Th") and T4 DNA ligase buffer ("T4"). Five different plasmids were tested (SEQ. ID 1, 3, 4, 5 and 6). Positive control plasmid contained a single and complete TelN site (SEQ. ID NO:1), formed by TelR (SEQ. ID NO:21) and TelL (SEQ. ID NO:22) sequences, each one having 28 bp. The TelR region of the other four plasmids had 10 (SEQ. ID NO:3), 9 (SEQ. ID NO:7), 8 (SEQ. ID NO:4) or 7 bp (SEQ. ID NO:5) respectively. Native agarose gel electrophoresis (0,6%) showed the capacity to convert supercoiled plasmid DNA into linear closed DNA by TelN protelomerase depending on the length of the TelR sequence and the reaction buffer used.

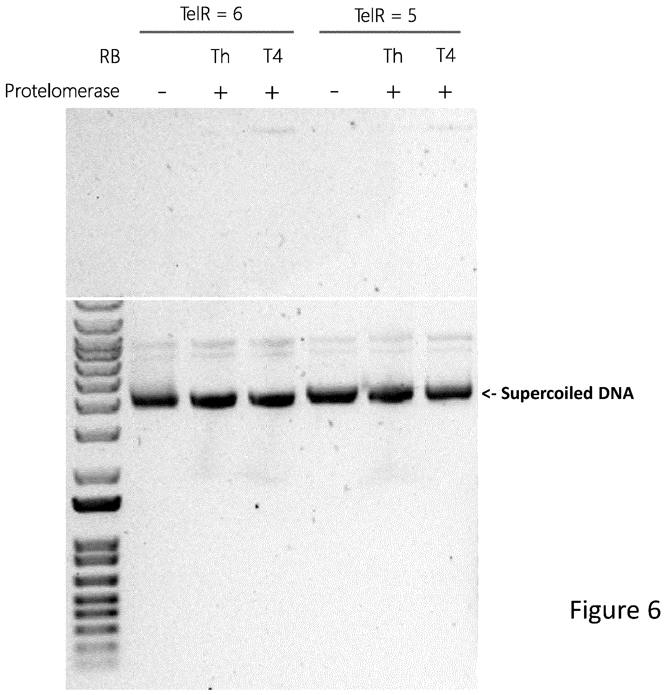

**FIG. 6** shows the enzymatic processing of different plasmid DNAs by TelN protelomerase. Two different reaction buffers are shown: ThermoPol^{®} reaction buffer ("Th") and T4 DNA ligase buffer ("T4"). Two plasmids were tested having TelR regions of 6 (SEQ. ID NO:6), and 5 bp (SEQ. ID NO:20) respectively. Native agarose gel electrophoresis (0,6%) showed the incapacity of TelN protelomerase to convert supercoiled plasmid DNA into linear closed DNA in both cases, independently of the reaction buffer used.

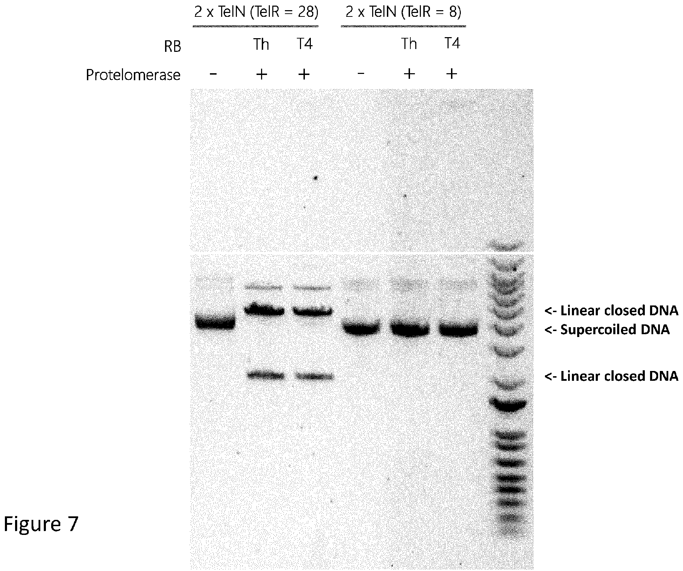

**FIG. 7** shows the enzymatic processing of different plasmid DNAs by TelN protelomerase. Two different reaction buffers are shown: ThermoPol^{®} reaction buffer ("Th") and T4 DNA ligase buffer ("T4"). Two plasmids were tested both having two TelN sites. Positive control plasmid contained two complete TelN sites (SEQ. ID NO:1), while the other plasmid had 8-bp TelR sequences (SEQ. ID NO:4). Native agarose gel electrophoresis (0,6%) showed the capacity to convert supercoiled plasmid DNA into linear closed DNA by TelN protelomerase depending on the length of the TelR sequences and the reaction buffer used.

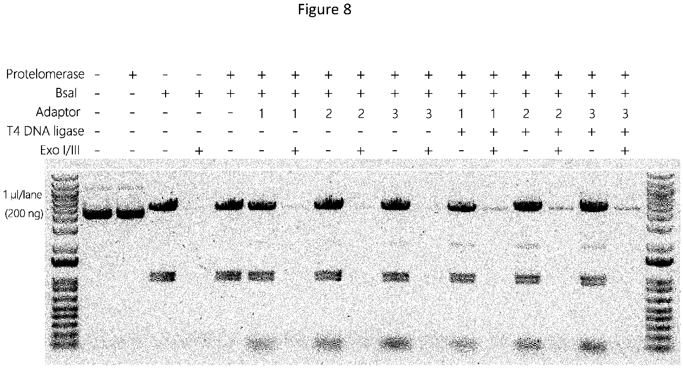

**FIG. 8** shows the agarose gel electrophoresis analysis of the plasmid DNA containing two inactive TelN sites (SEQ ID NO:4) processed with TelN protelomerase, Bsal endonuclease, T4 DNA ligase, different DNA adaptor molecules, exonucleases I and III, and combinations thereof.

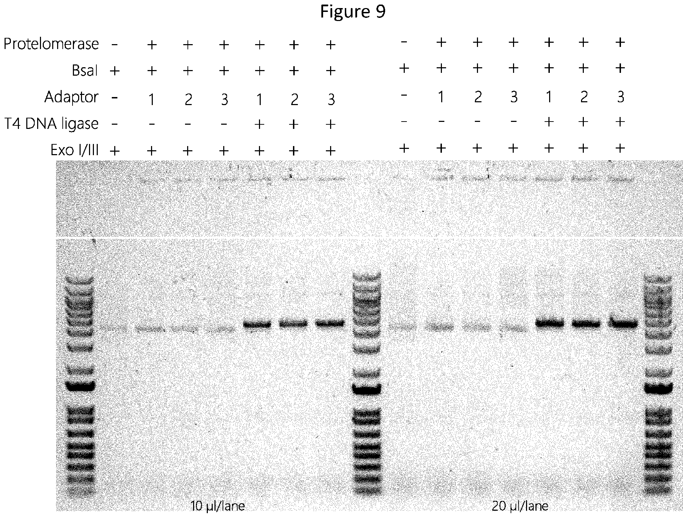

**FIG. 9** shows the agarose gel electrophoresis analysis of the samples treated with exonuclease I and III in FIG. 8 but loading 10 or 20 times more volume to increase sensitivity of detection and DNA size comparison.

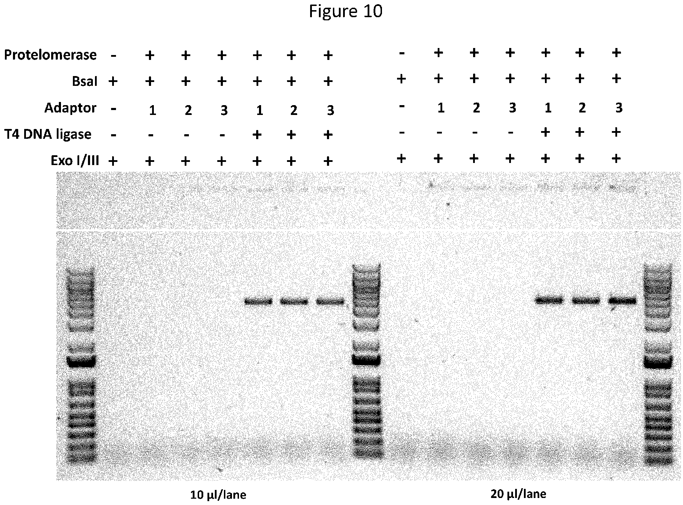

**FIG. 10** shows the agarose gel electrophoresis analysis of the samples shown in FIG 8 treated with exonuclease I, III and T5.

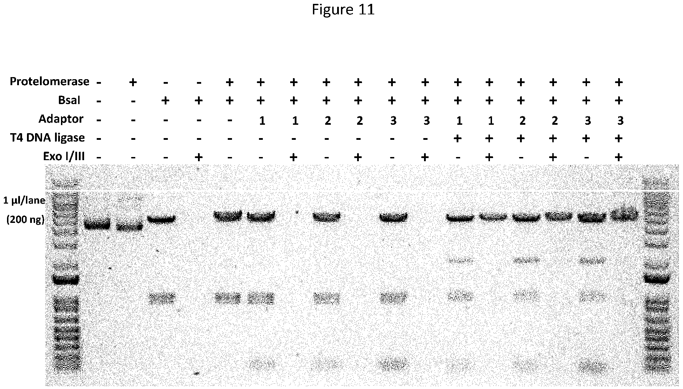

**FIG. 11** shows the agarose gel electrophoresis analysis of the plasmid DNA containing two inactive TelN sites (SEQ ID NO:4) processed with TelN protelomerase, Bsal endonuclease, T4 DNA ligase, DNA adaptors, exonucleases I and III, and combinations thereof using a longer incubation time (23 hours at 30°C) than in FIG. 8.

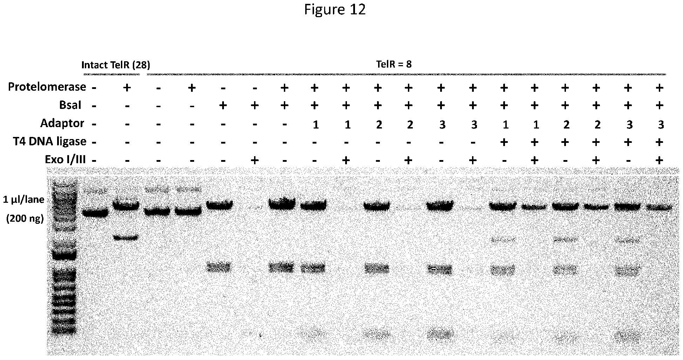

**FIG. 12** shows the agarose gel electrophoresis analysis of the plasmid DNA containing two inactive TelN sites (SEQ ID NO:4) sequentially processed with TelN protelomerase, Bsal endonuclease, T4 DNA ligase, DNA adaptors, exonucleases I and III, and combinations thereof.

The sequences discussed in the application are provided in the table below:

**Table 1. Sequences discussed in the application.**

| SEQ ID:NO | name | sequence |
|---|---|---|
| 1 | TelN full length | TATCAGCACACAATTGCCCATTATACGCGCGTATAATGGA CTATTGTGTGCTGATA |
| 2 | telR 12bp (telO+1) | CCCATTATACGCGCGTATAATGGACTATTGTGTGCTGATA |
| 3 | telR 10bp (telO-1) | CATTATACGCGCGTATAATGGACTATTGTGTGCTGATA |
| 7 | telR 9bp (telO-2) | ATTATACGCGCGTATAATGGACTATTGTGTGCTGATA |
| 4 | telR 8bp (telO-3) | TTATACGCGCGTATAATGGACTATTGTGTGCTGATA |
| 5 | telR 7bp (telO-4) | TATACGCGCGTATAATGGACTATTGTGTGCTGATA |
| 6 | telR 6bp (telO-5) | ATACGCGCGTATAATGGACTATTGTGTGCTGATA |
| 20 | telR 5bp (telO-6) | TACGCGCGTATAATGGACTATTGTGTGCTGATA |
| 8 | Adaptor 1 sense | TATCAGCACACAATTGCCCAT |
| 9 | Adaptor 1 antisense | ATAGTCGTGTGTTAACGGGTAATAT |
| 10 | Adaptor 2 sense | ATGTTAGTATCAGCACACAATTGCCCAT |
| 11 | Adaptor 2 antisense | TACAATCATAGTCGTGTGTTAACGGGTAATAT |
| 12 | Adaptor 3 sense | GGCCAACAAATGTTAGTATCAGCACACAATTGCCCAT |
| 13 | Adaptor 3 antisense | CCGGTTGTTTACAATCATAGTCGTGTGTTAACGGGTAATAT |
| 14 | Adaptor sequence | AGGGCTAACATTTGTTGGCC |
| 15 | Adaptor sequence | GGCCAACAAATGTTAG |
| 16 | loxP | ATAACTTCGTATAATGTATG CTATACGAAG TTAT |
| 17 | Adaptor sequence | AGGGCTAACATTTGTTGGCCACTCAGGCCA CAAATGTTAG |
| 18 | Adaptor sequence | GGCCAACAAATGTTAG |
| 19 | Adaptor sequence | AGGGCTAACATTTGTTGGCC |
| 21 | Complete telR | TATCAGCACACAATTGCCCATTATACGC |
| 22 | Complete telL | GCGTATAATGGACTATTGTGTGCTGATA |
| 23 | telL 8bp (telO-3) | TATCAGCACACAATTGCCCATTATACGCGCGTATAA |
| 24 | Adaptor 4 sense | TATAATGGACTATTGTGTGCTGATA |
| 25 | Adaptor 4 antisense | TACCTGATAACACACGACTAT |
| 26 | Adaptor 5 sense | TATAATGGACTATTGTGTGCTGATACTAACAT |
| 27 | Adaptor 5 antisense | TACCTGATAACACACGACTATGATTGTA |
| 28 | Adaptor 6 sense | TATAATGGACTATTGTGTGCTGATACTAACATTTGTTGGCC |
| 29 | Adaptor 6 antisense | TACCTGATAACACACGACTATGATTGTAAACAACCGG |
| 30 | telL 7bp | TATCAGCACACAATTGCCCATTATACGCGCGTATA |
| 31 | telL 6bp | TATCAGCACACAATTGCCCATTATACGCGCGTAT |
| 32 | telL 5bp | TATCAGCACACAATTGCCCATTATACGCGCGTA |

### EXAMPLES

### Example 1 Rolling circle amplification of Cre-derived circular DNA

Cre recombinase from the P1 bacteriophage is a Type I topoisomerase. The enzyme catalyzes the site-specific recombination of DNA between loxP sites (SEQ ID NO: 3). LoxP recognition site (34 bp) consists of two 13 bp inverted repeats which flank an 8 bp spacer region, which confers directionality. The products of Cre-mediated recombination are dependent upon the location and relative orientation of the loxP sites. Two DNA species containing single loxP sites were fused. DNA found between two loxP sites oriented in the same direction was excised as a circular loop of DNA, while DNA between opposing loxP sites was inverted with respect to external sequences. Cre recombinase requires no additional cofactors or accessory proteins for its function.

Cre reaction conditions: reaction volume 50 µl, DNA of interest purified by ethanol precipitation after restriction enzyme digestion (100 ng), Cre recombinase (NEB, 4 units), incubation time and temperature: 30 min at 37°C and 20 min at 80°C. Next, to remove remaining non-circular DNA molecules before the amplification step, *E. coli* exonuclease I (NEB, 20 units) and III (NEB, 100 units) were added and the reaction was incubated 30 min at 37°C and 20 min at 80°C.

Rolling circle amplification (RCA) is a faithful and isothermal DNA amplification method based on Phi29 DNA polymerase (Phi29DNApol). Phi29DNApol is the monomeric enzyme responsible for the replication of the linear double stranded DNA of bacteriophage phi29 from *Bacillus subtilis* (Blanco and Salas, 1984). It is an extremely processive polymerase (up to more than 70 kb per binding event) with a strong strand displacement capacity (Blanco *et al,* 1989). The enzyme displays 3'->5' proofreading exonuclease activity (Garmendia *et al,* 1992), resulting in an extremely high fidelity of synthesis (Esteban *et al,* 1993). These special features make this enzyme the perfect choice for isothermal DNA amplification.

RCA can be initiated by random synthetic primers (Dean *et al,* 2001) or a DNA primase like TthPrimPol (Picher *et al,* 2016) that synthesizes the primers for Phi29DNApol during the amplification reaction. Before the amplification, circularized DNA samples were first denatured by adding 1 volume of buffer D (400 mM KOH, 10 mM EDTA) and incubating 3 min at room temperature. Samples were then neutralized by adding 1 volume of buffer N (400 mM HCl, 600 mM Tris-HCl pH 7.5). Rolling circle amplification conditions: 10 ml reaction volume, 1 ml TruePrime WGA reaction buffer 10x (4basebio), 500 µl denatured DNA sample, 1 ml TthPrimPol (1 µM), 160 µl QualiPhi Phi29DNApol (12,5 µM), 2.5 units PPase (Thermo) and 1 ml dNTPs (10 mM). Incubation time and temperature: 20 hours at 30°C and 10 min at 65°C.

### Example 2 Digestion with TelN protelomerase

*Escherichia coli* phage N15 encodes a 630-residue protein of 72,2 kDa called protelomerase (TelN). TelN protelomerase is a component of the N15 replication system proposed to be involved in the generation of the linear prophage DNA (Deneke *et al,* 2000)*.*

Purified TelN can process circular and linear plasmid DNA containing its target site (SEQ. ID NO:1) to produce linear double-stranded DNA with covalently closed ends. The 56-bp target site consists of a central telO palindrome of 22 bp and two 14-bp flanking sequences comprising inverted repeats. telO is separated from these repeats by 3 bp on each side (FIG. 2; SEQ ID NO:1).

Plasmid DNAs were incubated with TelN protelomerase to check the capacity of the enzyme to process truncated target sites (SEQ. ID. NO:s 2-7 & 20) and generate linear double-stranded DNA with covalently closed ends. Reaction conditions: reaction volume 20 µl, 2 µl reaction buffer 10x (T4 DNA ligase or ThermoPol^{®}), 4 µg DNA and 800 ng TelN protelomerase. Incubation time and temperature: 30 minutes at 30°C and 5 minutes at 75°C.

### Example 3 Digestion with TelN protelomerase in the presence of adaptors restoring TelN complete target site

The identification of a completely inactive TelN target sequence (SEQ. ID NO: 4) formed by a complete TelL sequence (28 bp, SEQ. ID. NO: 22) and a TelR of only 8 bp, allowed us to test the capacity of TelN protelomerase to process target sites restored by adaptor hybridization or adaptor ligation. Shown in FIG. 2 & 3 are the sequences of the complete TelN target site and the sequence of the inactive site, in which the TelR region has been reduced to 8 bp only. Moreover, the sequences of three different adaptor molecules formed by complementary oligonucleotides are shown (SEQ ID NO: 8-13). The three DNA adaptors restore the complete TelN target site. As shown in FIG. 3, adaptor hybridization and ligation are driven by sequence complementarity of the protruding ends generated by digestion with a type IIS restriction endonuclease (i.e. Bsal).

Shown in FIG. 8 is the agarose gel electrophoresis analysis of the plasmid DNA containing two inactive TelN sites (lane 1) digested with TelN protelomerase (lane 2) or the restriction enzyme Bsal (lane 3), or both enzymes together (lane 5). As shown before (FIG. 4), TelN protelomerase was not able to convert this supercoiled plasmid DNA into linear closed DNA. Bsal endonuclease cut on both sides of the two inactive TelN sites of the plasmid, releasing the unit size of the cassette of interest (4,5 kb). Moreover, the enzyme also cut on the plasmid backbone, generating two fragments of approximately the same size (1 and 0,9 kb). Insufficient Bsal activity provoked another 1,9-kb band resulting from the incomplete digestion of the plasmid backbone. Bsal-digested DNA was degraded by exonucleases I and III (lane 4), since none of the fragments were covalently closed at both ends to prevent exonuclease degradation. The presence of the different adaptors, with (lanes 12-17) or without (lanes 6-11) T4 DNA ligase, enabled the appearance of a band resistant to exonuclease digestion, suggesting the conversion of linear DNA into linear closed DNA by TelN protelomerase. Reaction conditions: reaction volume 200 µl, 20 µl T4 DNA ligase reaction buffer 10x, 40 µg DNA, 8 µg TelN protelomerase, 30 units Bsal, 1,7 µg T4 DNA ligase, 1,4 µM adaptor DNA (10-fold molar excess). Incubation time and temperature: 30 minutes at 30°C and 5 minutes at 75°C. Exonuclease I and III digestion conditions: 107 ng exonuclease I and 188 ng exonuclease III. Incubation time and temperature: 120 minutes at 37°C.

However, as shown in FIG. 9, in which only exonuclease-treated samples were overloaded (10 and 20 times more DNA per lane), exonuclease resistant DNAs displayed different sizes depending on the presence or absence of T4 DNA ligase in the reaction. The ones generated in the presence of T4 DNA ligase were slightly larger.

To elucidate if those DNAs were DNAs with covalently closed ends, T5 exonuclease digestion was performed. T5 exonuclease from phage D15 degrades DNA in the 5' to 3' direction (opposite to exonuclease I and III). T5 exonuclease can initiate nucleotide removal from the 5' termini or at gaps and nicks of linear or circular dsDNA. T5 exonuclease digestion conditions: 10 units T5 exonuclease, 49 µl of exonuclease-resistant DNA from the previous step. Incubation time and temperature: 30 minutes at 37°C.

As shown in FIG. 10, only the DNAs generated in the presence of T4 DNA ligase are resistant to T5 exonuclease digestion, indicating that were the only ones having closed ends at both sides of the target molecule.

Shown in figure 11 is the agarose gel electrophoresis analysis of the plasmid DNA containing two inactive TelN sites processed followed the same conditions used in FIG. 8 but incubating the reactions for 23 hours at 30°C instead of 30 minutes. The three different adaptors in combination with T4 DNA ligase (lanes 12-17) enabled the protection of the target DNA fragment against exonuclease digestion, demonstrating the efficient conversion of linear DNA into linear closed DNA by TelN protelomerase due to the ligation of adaptors restoring TelR sites (SEQ ID NO:21) on both ends of the DNA of interest. Reaction conditions: reaction volume 200 µl, 20 µl T4 DNA ligase reaction buffer 10x, 40 µg DNA, 8 µg TelN protelomerase, 30 units Bsal, 1,7 µg T4 DNA ligase, 1,4 µM adaptor DNA (10-fold molar excess). Incubation time and temperature: 23 hours at 30°C and 5 minutes at 75°C. Exonuclease I and III digestion conditions: 107 ng exonuclease I and 188 ng exonuclease III. Incubation time and temperature: 120 minutes at 37°C.

Shown in figure 12 is the agarose gel electrophoresis analysis of the plasmid DNA containing two inactive TelN sites processed followed the same conditions used in FIG. 11 but incubating plasmid DNA first with Bsal and DNA adaptors in the presence or absence of T4 DNA ligase for 23 hours at 30°C. Next, TelN protelomerase was added and the mixture was incubated for another 30 minutes at 30°C. Finally, the mixture was heat inactivated for 5 minutes at 75°C. The three different adaptors in combination with T4 DNA ligase (lanes 14-19) enabled the protection of the target DNA fragment against exonuclease digestion, demonstrating the efficient conversion of linear DNA into linear closed DNA by TelN protelomerase due to the ligation of adaptors restoring TelN sites on both ends of the DNA of interest. Plasmid DNA containing two complete TelN sites was included (lanes 1 and 2) as positive control for TelN protelomerase. Reaction conditions: reaction volume 200 µl, 20 µl T4 DNA ligase reaction buffer 10x, 40 µg DNA, 8 µg TelN protelomerase, 30 units Bsal, 1,7 µg T4 DNA ligase, 1,4 µM adaptor DNA (10-fold molar excess). Exonuclease I and III digestion conditions: 107 ng exonuclease I and 188 ng exonuclease III. Incubation time and temperature: 120 minutes at 37°C.

## Claims

1. A method of producing a linear DNA product, wherein the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase target sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(c) incubating the precursor double-stranded DNA molecule with a protelomerase to generate the linear DNA product, wherein the protelomerase closes the first end of the precursor double-stranded DNA molecule at the first functional protelomerase target sequences.

2. A method of producing a closed linear DNA product, wherein the method comprises the steps of:
(a) digesting the double-stranded DNA molecule with an endonuclease that cleaves the endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase target sequences are non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
(c) incubating the precursor double-stranded DNA molecule with a protelomerase to generate the closed linear DNA product, wherein the protelomerase closes the first and second ends of the precursor double-stranded DNA molecule at the first and second functional protelomerase target sequences.

3. A method of producing a closed linear DNA product, wherein the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, a truncated protelomerase sequence at a first end and a truncated protelomerase sequence at a second end, wherein the truncated protelomerase target sequences are non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule and wherein the second adaptor molecule comprises a truncated protelomerase target sequence that forms a second functional protelomerase target sequence with the truncated protelomerase sequence at the second end of the digested double-stranded DNA molecule; and
(c) incubating the precursor double-stranded DNA molecule with a protelomerase to generate the closed linear DNA product, wherein the protelomerase closes the first and second ends of the precursor double-stranded DNA molecule at the first and second functional protelomerase target sequences.

4. The method of any one of claims 1 to 3, wherein the first and/or the second adaptor molecules are nucleic acid adaptor molecules.

5. The method of claims 1 to 4, wherein the first adaptor molecule and/or the second adaptor molecule comprises a double stranded region with an overhang.

6. The method of claims 1 to 5, wherein the first adaptor molecule hybridizes to the first end of the digested double-stranded DNA molecule and the second adaptor hybridizes to the second end of the digested double-stranded DNA molecule.

7. The method of any one of claims claim 1 to 6, wherein steps (a)-(c) are performed in a single contiguous aqueous volume.

8. The method of anyone of claims 1 to 6, wherein steps (a)-(c) are performed sequentially in separate reactions.

9. The method of anyone of claims 1 to 8, wherein the first adaptor is ligated to the first end of the linear double-stranded region and the second adaptor is ligated to the second end of the linear double-stranded region.

10. The method of claims 1 to 9, wherein the endonuclease is a type II endonuclease, a blunt end endonuclease or a type IIS endonuclease.

11. The method of claim 10, wherein the endonuclease is a Type IIS restriction endonuclease, optionally wherein the endonuclease is Bbsl, Bsal, BsmBI, BspOI, BtgZI, Esp3I,SapI, Aarl, Acc361, AclWI, Acul, Ajul, AloI, Alw261, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse11, Bse3DI, BseGI, BseMI, BseMII, BseNI, BseRI, BseXI, Bsgl, BsIFI, BsmAl, BsmFI, Bsml, Bso31I, BspCNI, BspMI, BspPI, BspQI, BspTNI, BsrDI, Bsrl, Bst6I, BstF51, BstMAI, BstV11, BstV21, Bsul, BtgZI, BtsCl, Btsl-v2, BtsMutl, Bvel, Csel, CspCI, Eam11041, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCI, PciSI, Pctl, Plel, Ppsl, Psrl, Schl, SfaNI, Taqll, TspDTI and/or TspGWI restriction endonuclease.

12. A method of producing a partially closed linear DNA product, wherein the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, and wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule, wherein the truncated protelomerase target sequence of the first adaptor is non-functional and wherein the second adaptor molecule is a nucleic acid molecule that comprises one or more nuclease-resistant nucleotides; and
(c) incubating the precursor double-stranded DNA molecule with a protelomerase to generate the partially closed linear DNA product, wherein the protelomerase closes the first end of the precursor double-stranded DNA molecule at the first functional protelomerase target sequence.

13. The method of claim 12 wherein the one or more nuclease-resistant nucleotides are one or more phosphorothioated nucleotides.

14. A method of producing a closed linear DNA product, wherein the method comprises the steps of:
(a) digesting a double-stranded DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, and wherein the truncated protelomerase sequence is non-functional;
(b) appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and
(c) incubating the precursor double-stranded DNA molecule with a protelomerase to generate the closed linear DNA product, wherein the protelomerase closes the first end of the precursor double-stranded DNA molecule at the first functional protelomerase target sequence, and the second end is closed by the second adaptor molecule.

15. The method of claim 14, wherein the second adaptor molecule comprises a hairpin.

## Patentansprüche

1. Verfahren zum Herstellen eines linearen DNA-Produkts, wobei das Verfahren die Schritte umfasst:
(a) Verdauen eines doppelsträngigen DNA-Moleküls mit einer Endonuklease, welche eine Endonuklease-Zielsequenz spaltet, um ein verdautes doppelsträngiges DNA-Molekül zu erzeugen, wobei das verdaute doppelsträngige DNA-Molekül einen linearen doppelsträngigen Bereich und eine trunkierte Protelomerase-Sequenz an einem ersten Ende umfasst, wobei die trunkierte Protelomerase-Zielsequenz nicht funktionell ist;
(b) Anfügen eines ersten Adaptermoleküls an das erste Ende des verdauten doppelsträngigen DNA-Moleküls und Anfügen eines zweiten Adaptermoleküls an das zweite Ende des verdauten doppelsträngigen DNA-Moleküls, um ein doppelsträngiges DNA-Vorläufermolekül zu erzeugen, wobei das erste Adaptermolekül eine trunkierte Protelomerase-Zielsequenz umfasst, welche eine erste funktionelle Protelomerase-Zielsequenz mit der trunkierten Protelomerase-Sequenz am ersten Ende des verdauten doppelsträngigen DNA-Moleküls bildet; und
(c) Inkubieren des doppelsträngigen DNA-Vorläufermoleküls mit einer Protelomerase, um das lineare DNA-Produkt zu erzeugen, wobei die Protelomerase das erste Ende des doppelsträngigen DNA-Vorläufermoleküls an den ersten funktionellen Protelomerase-Zielsequenzen verschließt.

2. Verfahren zum Herstellen eines geschlossenen linearen DNA-Produkts, wobei das Verfahren die Schritte umfasst:
(a) Verdauen des doppelsträngigen DNA-Moleküls mit einer Endonuklease, welche die Endonuklease-Zielsequenz spaltet, um ein verdautes doppelsträngiges DNA-Molekül zu erzeugen, wobei das verdaute doppelsträngige DNA-Molekül einen linearen doppelsträngigen Bereich, eine trunkierte Protelomerase-Sequenz an einem ersten Ende und eine trunkierte Protelomerase-Sequenz an einem zweiten Ende umfasst, wobei die trunkierten Protelomerase-Zielsequenzen nicht funktionell sind;
(b) Anfügen eines ersten Adaptermoleküls an das erste Ende des verdauten doppelsträngigen DNA-Moleküls und Anfügen eines zweiten Adaptermoleküls an das zweite Ende des verdauten doppelsträngigen DNA-Moleküls, um ein doppelsträngiges DNA-Vorläufermolekül zu erzeugen, wobei das erste Adaptermolekül eine trunkierte Protelomerase-Zielsequenz umfasst, welche eine erste funktionelle Protelomerase-Zielsequenz mit der trunkierten Protelomerase-Sequenz am ersten Ende des verdauten doppelsträngigen DNA-Moleküls bildet, und wobei das zweite Adaptermolekül eine trunkierte Protelomerase-Zielsequenz umfasst, welche eine zweite funktionelle Protelomerase-Zielsequenz mit der trunkierten Protelomerase-Sequenz am zweiten Ende des verdauten doppelsträngigen DNA-Moleküls bildet; und
(c) Inkubieren des doppelsträngigen DNA-Vorläufermoleküls mit einer Protelomerase, um das geschlossene lineare DNA-Produkt zu erzeugen, wobei die Protelomerase das erste und zweite Ende des doppelsträngigen DNA-Vorläufermoleküls an der ersten und zweiten funktionellen Protelomerase-Zielsequenz verschließt.

3. Verfahren zum Herstellen eines geschlossenen linearen DNA-Produkts, wobei das Verfahren die Schritte umfasst:
(a) Verdauen eines doppelsträngigen DNA-Moleküls mit einer Endonuklease, welche eine Endonuklease-Zielsequenz spaltet, um ein verdautes doppelsträngiges DNA-Molekül zu erzeugen, wobei das verdaute doppelsträngige DNA-Molekül einen linearen doppelsträngigen Bereich, eine trunkierte Protelomerase-Sequenz an einem ersten Ende und eine trunkierte Protelomerase-Sequenz an einem zweiten Ende umfasst, wobei die trunkierten Protelomerase-Zielsequenzen nicht funktionell sind;
(b) Anfügen eines ersten Adaptermoleküls an das erste Ende des verdauten doppelsträngigen DNA-Moleküls und Anfügen eines zweiten Adaptermoleküls an das zweite Ende des verdauten doppelsträngigen DNA-Moleküls, um ein doppelsträngiges DNA-Vorläufermolekül zu erzeugen, wobei das erste Adaptermolekül eine trunkierte Protelomerase-Zielsequenz umfasst, welche eine erste funktionelle Protelomerase-Zielsequenz mit der trunkierten Protelomerase-Sequenz am ersten Ende des verdauten doppelsträngigen DNA-Moleküls bildet, und wobei das zweite Adaptermolekül eine trunkierte Protelomerase-Zielsequenz umfasst, welche eine zweite funktionelle Protelomerase-Zielsequenz mit der trunkierten Protelomerase-Sequenz am zweiten Ende des verdauten doppelsträngigen DNA-Moleküls bildet ; und
(c) Inkubieren des doppelsträngigen DNA-Vorläufermoleküls mit einer Protelomerase, um das geschlossene lineare DNA-Produkt zu erzeugen, wobei die Protelomerase das erste und zweite Ende des doppelsträngigen DNA-Vorläufermoleküls an der ersten und zweiten funktionellen Protelomerase-Zielsequenz verschließt.

4. Verfahren nach einem der Ansprüche 1 bis 3, wobei das erste und/oder das zweite Adaptermolekül Nukleinsäure-Adaptermoleküle sind.

5. Verfahren nach den Ansprüchen 1 bis 4, wobei das erste Adaptermolekül und/oder das zweite Adaptermolekül einen doppelsträngigen Bereich mit einem Überhang umfasst.

6. Verfahren nach einem der Ansprüche 1 bis 5, wobei das erste Adaptermolekül an das erste Ende des verdauten doppelsträngigen DNA-Moleküls hybridisiert und das zweite Adaptermolekül an das zweite Ende des verdauten doppelsträngigen DNA-Moleküls hybridisiert.

7. Verfahren nach einem der Ansprüche 1 bis 6, wobei Schritte (a)-(c) in einem einzigen kontinuierlichen wässrigen Volumen durchgeführt werden.

8. Verfahren nach einem der Ansprüche 1 bis 6, wobei Schritte (a)-(c) nacheinander in getrennten Reaktionen durchgeführt werden.

9. Verfahren nach einem der Ansprüche 1 bis 8, wobei der erste Adapter an das erste Ende des linearen doppelsträngigen Bereichs ligiert wird und der zweite Adapter an das zweite Ende des linearen doppelsträngigen Bereichs ligiert wird.

10. Verfahren nach einem der Ansprüche 1 bis 9, wobei die Endonuklease eine Typ-II-Endonuklease, eine stumpf schneidende Endonuklease oder eine Typ-IIS-Endonuklease ist.

11. Verfahren nach Anspruch 10, wobei die Endonuklease eine Typ-IIS-Restriktionsendonuklease ist, gegebenenfalls wobei die Endonuklease Bbsl, Bsal, BsmBl, BspQI, BtgZI, Esp3I, Sapl, Aarl, Acc36I, AcIWI, Acul, Ajul, Alol, Alw26I, Alwl, Arsl, AsuHPI, Bael, Barl, Bbvl, Bccl, BceAI, Bcgl, BciVI, BcoDI, BfuAl, Bful, Bmrl, Bmsl, Bmul, Bpil, Bpml, BpuEl, BsaXI, Bse11, Bse3DI, BseGl, BseMI, BseMII, BseNI, BseRI, BseXI, Bsgl, BsIFI, BsmAl, BsmFl, Bsml, Bso31I, BspCNI, BspMI, BspPl, BspQl, BspTNI, BsrDI, Bsrl, Bst61, BstF5I, BstMAI, BstV1I, BstV21, Bsul, BtgZI, BtsCl, Btsl-v2, BtsMutl, Bvel, Csel, CspCI, Eam1104I, Earl, Ecil, Eco31I, Eco571, Esp3I, Faql, Faul, Fokl, Gsul, Hgal, Hphl, HpyAV, Lgul, Lmnl, Lsp11091, Lwel, Mboll, Mlyl, Mmel, Mnll, Mva1269I, NmeAIII, PaqCl, PciSI, Pctl, Plel, Ppsl, Psrl, Schl, SfaNI, Taqll, TspDTI und/oder TspGWI Restriktionsendonuklease ist.

12. Verfahren zum Herstellen eines teilweise geschlossenen linearen DNA-Produkts, wobei das Verfahren die Schritte umfasst:
(a) Verdauen eines doppelsträngigen DNA-Moleküls mit einer Endonuklease, welche eine Endonuklease-Zielsequenz spaltet, um ein verdautes doppelsträngiges DNA-Molekül zu erzeugen, wobei das verdaute doppelsträngige DNA-Molekül einen linearen doppelsträngigen Bereich und eine trunkierte Protelomerase-Sequenz an einem ersten Ende umfasst und wobei die trunkierte Protelomerase-Sequenz nicht funktionell ist;
(b) Anfügen eines ersten Adaptermoleküls an das erste Ende des verdauten doppelsträngigen DNA-Moleküls und Anfügen eines zweiten Adaptermoleküls an das zweite Ende des verdauten doppelsträngigen DNA-Moleküls, um ein doppelsträngiges DNA-Vorläufermolekül zu erzeugen, wobei das erste Adaptermolekül eine trunkierte Protelomerase-Zielsequenz umfasst, welche eine erste funktionelle Protelomerase-Zielsequenz mit der trunkierten Protelomerase-Sequenz am ersten Ende des verdauten doppelsträngigen DNA-Moleküls bildet, wobei die trunkierte Protelomerase-Zielsequenz des ersten Adapters nicht funktionell ist und wobei das zweite Adaptermolekül ein Nukleinsäuremolekül ist, welches ein oder mehrere Nuklease-resistente Nukleotide umfasst; und
(c) Inkubieren des doppelsträngigen DNA-Vorläufermoleküls mit einer Protelomerase, um das teilweise geschlossene lineare DNA-Produkt zu erzeugen, wobei die Protelomerase das erste Ende des doppelsträngigen DNA-Vorläufermoleküls an der ersten funktionellen Protelomerase-Zielsequenz verschließt.

13. Verfahren nach Anspruch 12, wobei die eine oder mehreren Nuklease-resistenten Nukleotide eine oder mehrere phosphorthioierte Nukleotide sind.

14. Verfahren zum Herstellen eines geschlossenen linearen DNA-Produkts, wobei das Verfahren die Schritte umfasst:
(a) Verdauen eines doppelsträngigen DNA-Moleküls mit einer Endonuklease, welche eine Endonuklease-Zielsequenz spaltet, um ein verdautes doppelsträngiges DNA-Molekül zu erzeugen, wobei das verdaute doppelsträngige DNA-Molekül einen linearen doppelsträngigen Bereich und eine trunkierte Protelomerase-Sequenz an einem ersten Ende umfasst und wobei die trunkierte Protelomerase-Sequenz nicht funktionell ist;
(b) Anfügen eines ersten Adaptermoleküls an das erste Ende des verdauten doppelsträngigen DNA-Moleküls und Anfügen eines zweiten Adaptermoleküls an das zweite Ende des verdauten doppelsträngigen DNA-Moleküls, um ein doppelsträngiges DNA-Vorläufermolekül zu erzeugen, wobei das erste Adaptermolekül eine trunkierte Protelomerase-Zielsequenz umfasst, welche eine erste funktionelle Protelomerase-Zielsequenz mit der trunkierten Protelomerase-Sequenz am ersten Ende des verdauten doppelsträngigen DNA-Moleküls bildet; und
(c) Inkubieren des doppelsträngigen DNA-Vorläufermoleküls mit einer Protelomerase, um das geschlossene lineare DNA-Produkt zu erzeugen, wobei die Protelomerase das erste Ende des doppelsträngigen DNA-Vorläufermoleküls an der ersten funktionellen Protelomerase-Zielsequenz verschließt und das zweite Ende durch das zweite Adaptermolekül verschlossen wird.

15. Verfahren nach Anspruch 14, wobei das zweite Adaptermolekül eine Haarnadel umfasst.

## Revendications

1. Procédé de production d'un produit d'ADN linéaire, où le procédé comprend les étapes consistant à :
(a) digérer une molécule d'ADN double brin avec une endonucléase qui clive une séquence cible d'endonucléase afin de générer une molécule d'ADN double brin digérée, dans lequel la molécule d'ADN double brin digérée comprend une région double brin linéaire, et une séquence de pro-télomérase tronquée à une première extrémité, dans lequel la séquence cible de pro-télomérase tronquée est non fonctionnelle ;
(b) ajouter une première molécule d'adaptateur à la première extrémité de la molécule d'ADN double brin digérée et ajouter une deuxième molécule d'adaptateur à la deuxième extrémité de la molécule d'ADN double brin digérée afin de générer une molécule d'ADN double brin précurseur, dans lequel la première molécule d'adaptateur comprend une séquence cible de pro-télomérase tronquée qui forme une première séquence cible de pro-télomérase fonctionnelle avec la séquence de pro-télomérase tronquée à la première extrémité de la molécule d'ADN double brin digérée ; et
(c) incuber la molécule d'ADN double brin précurseur avec une pro-télomérase afin de générer le produit d'ADN linéaire, dans lequel la pro-télomérase ferme la première extrémité de la molécule d'ADN double brin précurseur au niveau des premières séquences cibles de pro-télomérase fonctionnelles.

2. Procédé de production d'un produit d'ADN linéaire fermé, où le procédé comprend les étapes consistant à :
(a) digérer la molécule d'ADN double brin avec une endonucléase qui clive la séquence cible d'endonucléase afin de générer une molécule d'ADN double brin digérée, dans lequel la molécule d'ADN double brin digérée comprend une région double brin linéaire, une séquence de pro-télomérase tronquée à une première extrémité et une séquence de pro-télomérase tronquée à une deuxième extrémité, dans lequel les séquences cibles de pro-télomérase tronquée sont non fonctionnelles ;
(b) ajouter une première molécule d'adaptateur à la première extrémité de la molécule d'ADN double brin digérée et ajouter une deuxième molécule d'adaptateur à la deuxième extrémité de la molécule d'ADN double brin digérée afin de générer une molécule d'ADN double brin précurseur, dans lequel la première molécule d'adaptateur comprend une séquence cible de pro-télomérase tronquée qui forme une première séquence cible de pro-télomérase fonctionnelle avec la séquence de pro-télomérase tronquée à la première extrémité de la molécule d'ADN double brin digérée et dans lequel la deuxième molécule d'adaptateur comprend une séquence cible de pro-télomérase tronquée qui forme une deuxième séquence cible de pro-télomérase fonctionnelle avec la séquence de pro-télomérase tronquée à la deuxième extrémité de la molécule d'ADN double brin digérée ; et
(c) incuber la molécule d'ADN double brin précurseur avec une pro-télomérase afin de générer le produit d'ADN linéaire fermé, dans lequel la pro-télomérase ferme les première et deuxième extrémités de la molécule d'ADN double brin précurseur au niveau des première et deuxième séquences cibles de pro-télomérase fonctionnelles.

3. Procédé de production d'un produit d'ADN linéaire fermé, où le procédé comprend les étapes consistant à :
(a) digérer une molécule d'ADN double brin avec une endonucléase qui clive une séquence cible d'endonucléase afin de générer une molécule d'ADN double brin digérée, dans lequel la molécule d'ADN double brin digérée comprend une région double brin linéaire, une séquence de pro-télomérase tronquée à une première extrémité et une séquence de pro-télomérase tronquée à une deuxième extrémité, dans lequel les séquences cibles de pro-télomérase tronquée sont non fonctionnelles ;
(b) ajouter une première molécule d'adaptateur à la première extrémité de la molécule d'ADN double brin digérée et ajouter une deuxième molécule d'adaptateur à la deuxième extrémité de la molécule d'ADN double brin digérée afin de générer une molécule d'ADN double brin précurseur, dans lequel la première molécule d'adaptateur comprend une séquence cible de pro-télomérase tronquée qui forme une première séquence cible de pro-télomérase fonctionnelle avec la séquence de pro-télomérase tronquée à la première extrémité de la molécule d'ADN double brin digérée et dans lequel la deuxième molécule d'adaptateur comprend une séquence cible de pro-télomérase tronquée qui forme une deuxième séquence cible de pro-télomérase fonctionnelle avec la séquence de pro-télomérase tronquée à la deuxième extrémité de la molécule d'ADN double brin digérée ; et
(c) incuber la molécule d'ADN double brin précurseur avec une pro-télomérase afin de générer le produit d'ADN linéaire fermé, dans lequel la pro-télomérase ferme les première et deuxième extrémités de la molécule d'ADN double brin précurseur au niveau des première et deuxième séquences cibles de pro-télomérase fonctionnelles.

4. Procédé selon l'une quelconque des revendications 1 à 3, dans lequel la première et/ou la deuxième molécule d'adaptateur sont des molécules d'adaptateur d'acide nucléique.

5. Procédé selon les revendications 1 à 4, dans lequel la première molécule d'adaptateur et/ou la deuxième molécule d'adaptateur comprend une région double brin avec un porte-à-faux.

6. Procédé selon les revendications 1 à 5, dans lequel la première molécule d'adaptateur s'hybride à la première extrémité de la molécule d'ADN double brin digérée et le deuxième adaptateur s'hybride à la deuxième extrémité de la molécule d'ADN double brin digérée.

7. Procédé selon l'une quelconque des revendications 1 à 6, dans lequel les étapes (a)-(c) sont réalisées dans un seul volume aqueux contigu.

8. Procédé selon l'une quelconque des revendications 1 à 6, dans lequel les étapes (a)-(c) sont réalisées séquentiellement dans des réactions distinctes.

9. Procédé selon l'une quelconque des revendications 1 à 8, dans lequel le premier adaptateur est ligaturé à la première extrémité de la région double brin linéaire et le deuxième adaptateur est ligaturé à la deuxième extrémité de la région double brin linéaire.

10. Procédé selon les revendications 1 à 9, dans lequel l'endonucléase est une endonucléase de type II, une endonucléase créant des extrémités franches ou une endonucléase de type IIS.

11. Procédé selon la revendication 10, dans lequel l'endonucléase est une endonucléase de restriction de type IIS, facultativement dans lequel l'endonucléase est une endonucléase de restriction BbsI, BsaI, BsmBI, BspQI, BtgZI, Esp3I, SapI, AarI, Acc36I, AclWI, AcuI, AjuI, AloI, Alw26I, AlwI, ArsI, AsuHPI, BaeI, BarI, BbvI, BccI, BceAI, BcgI, BclVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BslFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnII, Mva1269I, NmeAIII, PaqCI, PciSI, PctI, PleI, PpsI, PsrI, SchI, SfaNI, TaqII, TspDTI, et/ou TspGWI.

12. Procédé de production d'un produit d'ADN linéaire partiellement fermé, où le procédé comprend les étapes consistant à :
(a) digérer une molécule d'ADN double brin avec une endonucléase qui clive une séquence cible d'endonucléase afin de générer une molécule d'ADN double brin digérée, dans lequel la molécule d'ADN double brin digérée comprend une région double brin linéaire, et une séquence de pro-télomérase tronquée à une première extrémité, et dans lequel la séquence de pro-télomérase tronquée est non fonctionnelle ;
(b) ajouter une première molécule d'adaptateur à la première extrémité de la molécule d'ADN double brin digérée et ajouter une deuxième molécule d'adaptateur à la deuxième extrémité de la molécule d'ADN double brin digérée afin de générer une molécule d'ADN double brin précurseur, dans lequel la première molécule d'adaptateur comprend une séquence cible de pro-télomérase tronquée qui forme une première séquence cible de pro-télomérase fonctionnelle avec la séquence de pro-télomérase tronquée à la première extrémité de la molécule d'ADN double brin digérée, dans lequel la séquence cible de pro-télomérase tronquée du premier adaptateur est non fonctionnelle et dans lequel la deuxième molécule d'adaptateur est une molécule d'acide nucléique qui comprend un ou plusieurs nucléotides résistants aux nucléases ; et
(c) incuber la molécule d'ADN double brin précurseur avec une pro-télomérase afin de générer le produit d'ADN linéaire partiellement fermé, dans lequel la pro-télomérase ferme la première extrémité de la molécule d'ADN double brin précurseur au niveau de la première séquence cible de pro-télomérase fonctionnelle.

13. Procédé selon la revendication 12, dans lequel les un ou plusieurs nucléotides résistants aux nucléases sont un ou plusieurs nucléotides à phosphorothioate.

14. Procédé de production d'un produit d'ADN linéaire fermé, où le procédé comprend les étapes consistant à :
(a) digérer une molécule d'ADN double brin avec une endonucléase qui clive une séquence cible d'endonucléase afin de générer une molécule d'ADN double brin digérée, dans lequel la molécule d'ADN double brin digérée comprend une région double brin linéaire, et une séquence de pro-télomérase tronquée à une première extrémité, et dans lequel la séquence de pro-télomérase tronquée est non fonctionnelle ;
(b) ajouter une première molécule d'adaptateur à la première extrémité de la molécule d'ADN double brin digérée et ajouter une deuxième molécule d'adaptateur à la deuxième extrémité de la molécule d'ADN double brin digérée afin de générer une molécule d'ADN double brin précurseur, dans lequel la première molécule d'adaptateur comprend une séquence cible de pro-télomérase tronquée qui forme une première séquence cible de pro-télomérase fonctionnelle avec la séquence de pro-télomérase tronquée à la première extrémité de la molécule d'ADN double brin digérée ; et
(c) incuber la molécule d'ADN double brin précurseur avec une pro-télomérase afin de générer le produit d'ADN linéaire fermé, dans lequel la pro-télomérase ferme la première extrémité de la molécule d'ADN double brin précurseur au niveau de la première séquence cible de pro-télomérase fonctionnelle, et la deuxième extrémité est fermée par la deuxième molécule d'adaptateur.

15. Procédé selon la revendication 14, dans lequel la deuxième molécule d'adaptateur comprend une épingle à cheveux.
